# EUROPEAN PATENT SPECIFICATION

(11) **EP 4 322 838 B1**
(45) Date of publication and mention of the grant of the patent: **11.11.2026**
(21) Application number: 22789124.9
(22) Date of filing: 13.04.2022
(51) Int. Cl.: A61B 5/02, A61B 5/00, A61B 5/01, A61B 5/0205, A61B 5/024

(54) **SYSTEMS AND METHODS FOR MULTIVARIATE STROKE DETECTION**
SYSTEME UND VERFAHREN ZUR MULTIVARIATEN SCHLAGANFALLDETEKTION
SYSTÈMES ET PROCÉDÉS DE DÉTECTION D'ATTAQUE MULTIVARIABLE

(30) Priority: 14.04.2021 US 202163175019 P; 28.01.2022 US 202263304259 P
(43) Date of publication of application: 21.02.2024
(73) Proprietor: Kandu Health, Inc., Campbell, CA 95008 (US)
(72) Inventor: STRASSER, Michael, Campbell, California 95008 (US); CARROLL, Kirsten, Campbell, California 95008 (US); BACKS, Jochen, Campbell, California 95008 (US); KHRAICHE, Massoud, Campbell, California 95008 (US); PARK, Sangshik, Campbell, California 95008 (US); HOSSAINY, Syed, Campbell, California 95008 (US); KRONE, Ryan Taylor, Campbell, California 95008 (US)
(74) Representative: ABG Intellectual Property Law, S.L.
(86) International application number: PCT/US2022/071701
(87) International publication number: WO 2022/221848

(56) References cited:
- WO-A1-2016/164221
- WO-A1-2016/164221
- WO-A1-2017/165532
- WO-A1-2018/015308
- WO-A1-2020/006048
- WO-A1-2020/257351
- CN-A- 107 233 085
- CN-A- 110 726 907
- CN-U- 208 404 565
- IN-A- 201821 025 060
- IN-A- 201821 025 060
- JP-A- 2010 535 542
- US-A1- 2008 294 058
- US-A1- 2010 331 916
- US-A1- 2014 118 931
- US-A1- 2014 121 555
- US-A1- 2017 021 172
- US-A1- 2018 168 508
- US-A1- 2018 307 362
- US-A1- 2019 282 152

## Description

### CROSS-REFERENCE TO RELATED APPLICATIONS

This application claims the priority benefit of U.S. Provisional Patent Applications: 63/175,019, filed April 14, 2021; and 63/304,259, filed January 28, 2022.

### TECHNICAL FIELD

This disclosure relates generally to the field of disease detection and, more specifically, to stroke detection.

### BACKGROUND

A stroke results from the death of brain tissue due to disruptions of blood flow to the brain. An ischemic stroke happens when there is a blockage of blood flow to the brain, usually as the result of a blood clot. Hemorrhagic stroke happens when there is a rupture of a blood vessel in the brain, resulting in bleeding into the brain tissue and surrounding space.

There are many physiologic symptoms of stroke onset that vary depending on the location of the affected tissue. Early symptoms of an evolving stroke may be able to reduce or even resolve if the interruption of blood flow is resolved quickly, before the tissue has died. One category of symptoms is disrupted vision, including blurred, dimming often likened to a curtain falling) or even complete loss of vision. Stroke patients (often also experience eye deviation or difficult with eye tracking.

Just as a stroke can affect the part of the brain that is associated with sight, it can also affect the parts of the brain that have to do with speech, comprehension and communication. Patients suffering from a stroke may exhibit slurred speech or garbled speech that renders them incomprehensible.

Another common symptom of stroke is weakness on one side of the body. This can manifest or partial or total paralysis of the side of the face, one arm, one leg, or the entire side of one's body.

Ischemic stroke is the most common type of stroke and is often painless when experienced, but hemorrhagic strokes are very painful, often being described as sudden onset of "the worst headache of one's life". Often, many people's headaches are accompanied with a feeling of dizziness, nausea, and vomiting. Smell and taste can also be impacted during the onset of a stroke.

Anything that affects the brain, from trauma to stroke, has the potential for cognitive disablement. A feeling of confusion, or a constant second-guessing of ones' actions, can sometimes appear days before a stroke occurs.

Another common symptom of a stroke is the sudden onset of fatigue.

Stroke symptoms can vary in duration and occur with or without pain, which can make stroke detection difficult. Further, strokes can occur during sleep, making detection even more difficult. If a stroke does occur while the person is sleeping, it may not wake a person up right away. As a result, when patients wake up symptomatic, it is unclear whether the stroke just started or whether it has already been occurring during sleep.

If a stroke is detected and patients seek care quickly, there are many evidence-based interventions that can dramatically reduce the death and disability resultant from the disease. In severe ischemic strokes, every minute of delay to flow restoration is equated to the loss of a week of Disability Adjusted Life Years (DALYs). Despite these treatments being available, fewer than 20% of patients receive them. Even among patients that do receive intervention, outcomes are often suboptimal because of the delays to intervention. Stroke detection is difficult because stroke frequently doesn't hurt, mimics other health events, and is heterogeneous in its presentation. Improvements in detection of and care-seeking for stroke onset could dramatically reduce the death and disability associated with the disease.

Like stroke, COVID-19 is proving to have heterogeneous symptoms, many of which resemble those of neurologic disorders. Recent publications have shown early evidence of encephalopathies, inflammatory central nervous system (CNS) syndromes, ischemic strokes, and peripheral neurological disorders in patients being treated for COVID-19. (Zubair, JAMA Neurology, 2020) With most COVID-19 patients being managed remotely, and a significant percentage of inpatients requiring invasive ventilation, monitoring for the obvious symptoms of neurological disruption may be difficult. As such, improvements in remote monitoring and care for COVID-19 patients could dramatically reduce the death and disability associated with the disease.

Document WO 2016/164221 A1 relates to devices configured to detect a stroke and/or a condition indicative of at least one neurological disease and methods of using such devices. Document IN 201821025060 A describes an insole device designed for the feet to detect changes associated with the potential formation of diabetic foot ulcers. Document WO 2020/006048 A1 describes a method for treating tremor using a wrist-worn device that delivers two types of stimulation: electrical stimulation from a first electrode/actuator and vibratory stimulation from a second, spaced-apart actuator. Document US 2019/282152 A1 discloses a device for assessing a psychophysiological responsiveness of a subject

### SUMMARY

The invention is set out in appended claims. In some aspects, the techniques described herein relate to a system for detecting an anomalous biologic event in a person, the system including: a wearable device configured to monitor a plurality of skin surface sites of a person, the device including: an electrode configured to obtain bioelectrical data, the electrode being placed in contact with at least one site in the plurality of skin surface sites; an electronic stimulus source having a surface area to provide stimulus; a sensor configured to sense physiological data, the sensor being placed adjacent to at least one of the plurality of skin surface sites; and at least one processor communicatively coupled to the wearable device. The processor is configured to: cause the stimulus source to generate a stimulus; cause excitation of the electrode to trigger monitoring of respective contacted skin surface sites; cause operation of the sensor; receive bioelectrical data from respective contacted skin surface sites; receive physiological data from the sensor; continuously compute a difference in the received bioelectrical data over a predefined time period; compute a difference in the received physiological data at intervals of the predefined time period; and generate, based on the computation, an assessment including a likelihood of occurrence of the anomalous biologic event.

In some aspects, the sensor is enclosed within the surface area of the stimulus source. In some aspects, the sensor is communicatively coupled with the stimulus source. In some aspects, the processor is further configured to: detect a lack of stimulus from the stimulus source; cause excitation of the electrode or a second electrode based on the detected lack of stimulus; obtain additional bioelectrical data; and responsive to determining that the additional bioelectrical data indicates an additional likelihood of the occurrence of the anomalous biologic event, updating the assessment.

In some aspects, the electrode includes a plurality of electrodes. In some aspects, the wearable device is configured to be worn on a wrist of the person, and the plurality of electrodes includes: a first electrode configured to contact a first skin surface site at a top region of the wrist and to obtain a skin hydration signal; a second electrode configured to contact a second skin surface site at a bottom region of the wrist to obtain a first skin conductance signal; and a third electrode configured to contact a third skin surface site at the bottom region of the wrist adjacent to the second electrode, the third electrode configured to obtain a second skin conductance signal. In some aspects, the plurality of electrodes includes: a first electrode configured to contact a first skin surface site and to obtain an electrodermal activity signal; a second electrode configured to contact a second skin surface site to obtain a first skin conductance signal; and a third electrode configured to contact a third skin surface site adjacent to the second electrode, the third electrode configured to obtain a second skin conductance signal. In some aspects, the at least one processor is further configured to switch between using the electrode with the sensor or a second sensor.

In some aspects, the wearable device is configured to be worn on a wrist of the person, and the plurality of electrodes includes: a first electrode configured to contact a first skin surface site at a top region of the wrist to obtain a reference electromyography (EMG) signal; a second electrode configured to contact a second skin surface site at a bottom region of the wrist to obtain a first EMG signal from a first muscle; and a third electrode configured to contact a third skin surface site at the bottom region of the wrist adjacent to the second electrode, the third electrode configured to obtain a second EMG signal from a second muscle.

In some aspects, the plurality of electrodes is associated with an electrodermal sensor configured for the excitation and collection of the bioelectrical data from at least one of the plurality of skin surface sites, and the plurality of sensors includes at least a blood volume sensor and an infrared temperature sensor. In some aspects, the bioelectrical data includes electromyography signals, bioimpedance signals, bioimpedance spectroscopy data, and electrodermal analysis data. In some aspects, the bioelectrical data is obtained simultaneously from the plurality of electrodes and analyzed in combination to generate the assessment. In some aspects, the bioelectrical data is obtained in a predefined sequential order based on a schedule configured for exciting one or more of the plurality of electrodes, the schedule being generated according to a cycle associated with the stimulus source and the plurality of sensors.

In some aspects, the wearable device includes a main portion, a first band portion, and a second band portion, and the plurality of electrodes include: a first electrode arranged on a first side of the main portion; a second electrode arranged on a first side of the first band portion; and a third electrode arranged on the first side of the first band portion and parallel to the second electrode. In some aspects, the wearable device is configured to be worn on a wrist of the person; the stimulus source is a heat stimulus source configured to uniformly heat the surface area and emit heat toward a top region of the wrist; and the at least one processor suppresses a portion of the plurality of electrodes from exciting until the stimulus source ceases generating stimulus.

In some aspects, the stimulus source is a thermal device configured to uniformly heat the surface area; and responsive to detecting that the surface area is heated to an offset temperature, triggering the sensor to sense the physiological data. In some aspects, the defined time period is based on a cycling time of the electronic stimulus source.

In some examples not according to the invention, the techniques described herein relate to a system for detecting an anomalous biologic event in a person, the system including: a wearable device configured to monitor a plurality of skin surface sites of a person, the device including: an electrode configured to obtain bioelectrical data, the electrode being placed in contact with at least one site in the plurality of skin surface sites; an electronic stimulus source having a surface area to provide stimulus; at least two sensors configured to sense physiological data, the at least two sensors being placed adjacent to at least one of the plurality of skin surface sites; and at least one processor communicatively coupled to the wearable device and configured to: cause the stimulus source to generate a stimulus; select, based on the generated stimulus, which of the at least two sensors to operate; cause excitation of the electrode to trigger monitoring the at least one skin surface site; cause operation of the selected sensor to trigger monitoring of at least one of the plurality of skin surface sites; receive bioelectrical data from the at least one skin surface sites in contact with the electrode; receive physiological data from the at least one of the plurality of skin surface sites; continuously compute a difference in the bioelectrical data over a predefined time period; for the selected sensor, compute a difference in the physiological data at intervals of the predefined time period; and generate, based on the computations, an assessment for including a likelihood of detection of the anomalous biologic event.

In some examples not according to the invention, the predefined period of time is based on a cycling time of the electronic stimulus source. In some examples not according to the invention, the at least two sensors are communicatively coupled with the stimulus source. In some examples not according to the invention, the at least two sensors are each enclosed within the surface area of the stimulus source. In some examples not according to the invention, the bioelectrical data includes electromyography signals, bioimpedance signals, bioimpedance spectroscopy data, and electrodermal analysis data.

In some examples not according to the invention, the electrode includes a plurality of electrodes. In some examples not according to the invention, the wearable device is configured to be worn on a wrist of the person; and the plurality of electrodes include: a first electrode configured to contact a first skin surface site at a top region of the wrist to obtain a reference electromyography (EMG) signal; a second electrode configured to contact a second skin surface site at a bottom region of the wrist to obtain a first EMG signal from a first muscle; and a third electrode configured to contact a third skin surface site at the bottom region of the wrist adjacent to the second electrode, the third electrode configured to obtain a second EMG signal from a second muscle. In some examples not according to the invention, the selected sensor is further selected based on which measurement is configured to be obtained by the wearable device, the measurements selected from a bioimpedance measurement, a bioimpedance spectroscopy measurement, an electromyography measurement, and an electrodermal analysis measurement. In some examples not according to the invention, the at least one processor is further configured to switch between using the selected sensor and an unselected sensor in the at least two sensors based on the measurement configured to be obtained by the wearable device.

In some examples not according to the invention, the plurality of electrodes each include an electrodermal sensor configured for the excitation and collection of bioelectrical data; and the at least two sensors include a blood volume sensor and an infrared temperature sensor. In some examples not according to the invention, the bioelectrical data is obtained in a predefined sequential order based on a schedule configured for exciting one or more of the plurality of electrodes, the schedule being generated according to a cycle associated with the stimulus source and the at least two sensors. In some examples not according to the invention, the wearable device includes a main portion, a first band portion, and a second band portion, and the plurality of electrodes include: a first electrode arranged on a first side of the main portion; a second electrode arranged on a first side of the first band portion; and a third electrode arranged on the first side of the first band portion and parallel to the second electrode.

According to the invention, the wearable device is configured to be worn on a wrist of the person; the stimulus source is a heat stimulus source configured to uniformly heat the surface area and emit heat toward a top region of the wrist; and the at least one processor suppresses the electrode from exciting until the stimulus source ceases generating stimulus. In some examples not according to the invention, the techniques described herein relate to a system, wherein: the stimulus source is a thermal device configured to uniformly heat the surface area; and responsive to detecting that the surface area is heated to an offset temperature, the at least one processor is further configured to trigger the at least two sensors to sense the physiological data.

In some examples not according to the invention, the techniques described herein relate to a method for detecting an anomalous biologic event in a person, the method including: for both a first wearable device associated with a left side of a person and a second wearable device associated with a right side of the person, each device being communicably coupled together and having a plurality of electrodes, a first sensor, and a second sensor: monitoring a plurality of skin surface sites of the person using a plurality of electrodes of the respective wearable device; causing excitation of the plurality of electrodes; causing operation of the first sensor to obtain a plurality of first measurements using the plurality of electrodes; causing a reconfiguration of the plurality of electrodes to trigger operation of the second sensor to obtain a plurality of second measurements using the plurality of electrodes, the reconfiguration connecting the plurality of electrodes from the first sensor to the second sensor; vacillating between the first sensor and the second sensor over a predefined time period to obtain a plurality of additional measurements for each of the first sensor and the second sensor; determining a skin conductance response of the left side and the right side, the skin conductance response based on the plurality of first measurements, the plurality of second measurements, and the plurality of additional measurements; determining differences between the skin conductance response of the left side associated with the first wearable device and the right side associated with the second wearable device; and generate, based on the differences, an assessment including a likelihood of detection of the anomalous biologic event.

In some examples not according to the invention, the plurality of first measurements and a portion of the additional measurements include bioimpedance measurements of at least one of the plurality of skin surface sites and the plurality of second measurements and a portion of the additional measurements include electrodermal analysis of at least one of the plurality of skin surface sites.

In some aspects, the techniques described herein relate to a thermal device configured to uniformly heat a surface area, including: a plurality of thin film layers, wherein at least one layer of the plurality of layers is a heater trace layer having a serpentine-shaped trace extending within a plane of the at least one layer to substantially cover a surface of the at least one layer; and a first aperture defined by the plurality of thin film layers and surrounded by the serpentine-shaped trace. In some aspects, the heater trace layer has a film thickness of about 15 micrometers to about 28 micrometers. In some aspects, each of the plurality of layers includes a relief perimeter, the relief perimeter including no active components or traces. In some aspects, the serpentine-shaped trace has a width of about 0.1 millimeters to about 0.2 millimeters; and gaps between adjacent traces are about 0.1 millimeters to about 0.3 millimeters.

In some examples not according to the invention, the gaps between adjacent traces are substantially equal in width. In some examples not according to the invention, the heater trace layer further includes a second aperture, wherein the serpentine-shaped trace surrounds the second aperture. In some examples not according to the invention, the traces include at least about 90 percent of the surface area of the heater trace layer. In some examples not according to the invention, the plurality of layers include at least: at least one physical insulator layer; at least one adhesive layer; at least one heat spreader layer; and at last one heater trace layer. In some examples not according to the invention, the plurality of layers include at least: a first layer including a first physical insulator layer; a second layer including a first adhesive layer; a third layer including a heat spreader layer; a fourth layer including a second physical insulator layer; a fifth layer including a heater trace layer; a sixth including a second adhesive layer; a seventh layer including a third physical insulator layer; and a eighth layer including a third adhesive layer. In some examples not according to the invention, the plurality of layers is arranged to emit heat uniformly over the surface area when installed in a thermal device housing and coupled to a power source.

In some examples not according to the invention, the first aperture sized for a first sensor. In some examples not according to the invention, the first sensor is a blood volume sensor. In some examples not according to the invention, the second aperture sized for a second sensor. In some examples not according to the invention, the second sensor is a skin temperature sensor. In some examples not according to the invention, the thermal device includes a plurality of heating zones, each zone being configured to maintain a different temperature. In some examples not according to the invention, the thermal device includes a wearable device, wherein the thermal device is positioned on a body of the wearable device for contact with a skin surface of a user.

In some examples not according to the invention, the techniques described herein relate to a wearable device configured to be worn on a wrist of a user, the wearable device including: a first electrode configured to obtain bioelectrical data; a first sensor configured to obtain physiological data; a processor; a heat stimulus source configured to uniformly heat a surface area and emit heat toward a skin region of a user, wherein the heat stimulus source includes a plurality of thin film layers, and wherein at least one layer of the plurality of layers is a heater trace layer having a serpentine-shaped trace extending within a plane of the at least one layer to substantially cover a surface of the at least one layer; and a first aperture defined by the plurality of thin film layers and surrounded by the serpentine-shaped trace, wherein the first aperture encloses the first sensor.

In some aspects, the skin region is a top region of a wrist of the user. In some examples not according to the invention, a second aperture is defined by the plurality of thin film layers, wherein the second aperture encloses a second sensor. In some examples not according to the invention, the first electrode includes a plurality of electrodes configured to obtain bioelectrical data.

In some aspects, a switching circuit is communicatively coupled to the processor and configured to select an operational state for a first electrode, a second electrode, and a third electrode in the plurality of electrodes, wherein the selected operational state triggers use of the first sensor or the second sensor. In some aspects, the selected operational state of the first electrode causes the first sensor to obtain a positive biopotential measurement and a positive bioimpedance measurement; the selected operational state of the second electrode causes the first sensor to obtain a negative biopotential measurement and a negative bioimpedance measurement; and the selected operational state of the third electrode causes the first sensor to obtain an electromyographical measurement. In some aspects, the selected operational state of the first electrode causes the second sensor to obtain a galvanic skin response measurement; the selected operational state of the second electrode causes the first sensor to obtain a bioimpedance measurement or a biopotential measurement; and the selected operational state of the third electrode causes the first sensor to obtain a positive biopotential measurement and a negative bioimpedance measurement. In some aspects, the selected operational state of the first electrode is disabled; the selected operational state of the second electrode causes the second sensor to obtain a negative galvanic skin response measurement; and the selected operational state of the third electrode is disabled.

In some aspects, the heat stimulus source is caused to operate in response to detecting that the first electrode is in the disabled operational state. In some aspects, a switching circuit is communicatively coupled to the at least one processor and configured to switch between operating the first sensor or the second sensor by connecting to one or more of a plurality of electrical connections to enable or disable one or more of the plurality of electrodes. In some aspects, switching amongst a plurality of electrical connections is associated with: a first switching circuit configured to utilize the first sensor, a second switching circuit configured to utilize the first sensor and the second sensor, and a third switching circuit configured to utilize the second sensor. In some aspects, the first sensor is a blood volume sensor, and the second sensor is a skin temperature sensor.

In some examples not according to the invention, the techniques described herein relate to a wearable device configured to be worn on a wrist of a user, the wearable device including: a body; at least one band coupled to the body and configured to secure the body to the user's wrist; a first electrode coupled to a portion of the at least one band and configured to contact a bottom portion of the user's wrist when the wearable device is in use; a second electrode operably positioned by a portion of the body and configured to contact a top portion of the user's wrist when the wearable device is in use; and one or more hardware processors positioned within an interior of the body and in communication with the first and second electrodes, the one or more hardware processors configured to obtain bioelectrical data using the first and second electrodes.

In some examples not according to the invention, the at least one band includes a first band and a second band, each of the first and second bands including a first end that is connected to the body of the wearable device and a second end opposite the first end; and the wearable device further includes an electrode housing coupled to the first band, wherein the first electrode is operably positioned by the electrode housing to contact the bottom portion of the user's wrist when the wearable device is in use. In some examples not according to the invention, a third electrode is operably positioned by the electrode housing and spaced from the first electrode, wherein one or more hardware processors is in communication with the third electrode and is configured to obtain bioelectrical data using the first, second, and third electrodes. In some examples not according to the invention, the first and third electrodes are spaced from one another by a distance that is between approximately 5 mm and approximately 100 mm.

In some examples not according to the invention, one of the first and third electrodes is configured to be substantially aligned with the second electrode when the at least one band and body of the wearable device are secured to the user's wrist. In some examples not according to the invention, the wearable device further includes a buckle configured to allow the first and second bands to form a closed loop around the user's wrist; and the electrode housing is coupled to the second end of the first band and forms a unitary structure with the buckle. In some examples not according to the invention, the first band includes at least one hole and the electrode housing includes at least one pin extending through the at least one hole to couple the electrode housing to the second end of the first band. In some examples not according to the invention, the at least one hole includes a plurality of holes and wherein the at least one pin includes a plurality of pins. In some examples not according to the invention, the electrode housing includes an upper portion and a lower portion, the upper and lower portions configured to secure together over the first band and apply a clamping force to the first band to secure the electrode housing to the first band.

In some examples not according to the invention, the electrode housing is fixed to the first band. In some examples not according to the invention, the electrode housing is movably coupled to the first band. In some examples not according to the invention, the electrode housing is slidable along a length of the first band. In some examples not according to the invention, the electrode housing includes an upper portion and a lower portion, the upper and lower portions configured to be: secured to one another around the first band to inhibit movement of the electrode housing relative to the first band; and at least partially removed from one another to allow a position of the electrode housing relative to the first band to be changed. In some examples not according to the invention, the first band includes at least one hole and the electrode housing includes at least one pin configured to extend through the at least one hole when the upper and lower portions are secured to one another around the first band. In some examples not according to the invention, the upper portion of the electrode housing includes the at least one pin.

In some aspects, the electrode housing includes a width that is greater than a width of the first band. In some aspects, the first and second electrodes are configured to be substantially aligned with one another when the at least one band and body of the wearable device are secured to the user's wrist. In some aspects, the one or more hardware processors are configured to determine at least one of electromyography (EMG) data, bioimpedance (BioZ/BIA) data, bioimpedance spectroscopy (BIS) data, and electrodermal analysis (EDA) data using the first and second electrodes.

In some aspects, a heat source is operably positioned by the body of the wearable device to contact the top portion of the user's wrist when the wearable device is use, and the heat source is configured to emit heat toward skin on the top portion, the heat source including a first opening and a second opening, wherein the first and second openings are spaced from one another and spaced inward from a perimeter of the heat source; a temperature sensor operably positioned by the body of the wearable device within the first opening of the heat source, the temperature sensor configured for measuring skin temperature at the top portion of the user's wrist; and at least one emitter and at least one detector operably positioned proximate the second opening of the heat source, the at least one emitter configured to emit light toward the top portion of the user's wrist and the at least one detector configured to detect at least a portion of the emitted light after attenuation by tissue and output one or more signals responsive to the detected light.

In some aspects, a heat source is operably positioned by the body of the wearable device to contact the top portion of the user's wrist when the wearable device is use, and the heat source is configured to emit heat toward skin on the top portion, the heat source including a first opening, a second opening, and a third opening; a temperature sensor operably positioned by the body of the wearable device within the first opening of the heat source; at least one emitter positioned within the second opening; and at least one detector positioned within the third opening.

In some examples not according to the invention, the techniques described herein relate to a wrist wearable physiological monitoring device including: a body including a skin facing surface, the skin facing surface including a heating pad including a plurality of openings configured to provide access to one or more sensor components, wherein the skin facing surface further includes an electrode; and a band configured to secure the body to a wrist of a user, wherein in a secured orientation, the electrode is positioned closer to a hand of the user than the heating pad.

In some examples not according to the invention, the heating pad is positioned on a first raised platform on the skin facing surface. In some examples not according to the invention, the electrode is positioned on a second raised platform on the skin facing surface. In some examples not according to the invention, the second raised platform is separate from the first raised platform. In some examples not according to the invention, a length of the heating pad is larger than a width of the heating pad. In some examples not according to the invention, a width of the heating pad is maximized to fit a surface of a wrist of the user. In some examples not according to the invention, a top surface, opposite the skin facing surface, includes an indicator configured to indicate a proper orientation when the body is secured to the wrist of the user. In some examples not according to the invention, the top surface further includes an opening configured to provide access to an ambient temperature sensor. In some examples not according to the invention, the ambient temperature sensor is placed on the opposite side of the heating pad. In some examples not according to the invention, the opening for the ambient temperature sensor includes a spoke design configured to protect the ambient temperature sensor from direct exposure and allow air to circulate.

In some aspects, the body does not include a display screen. In some aspects, the body includes a window proximate a charging port and a light emitting diode configured to transmit light through the window, wherein the light emitting diode is configured to indicate a status of the device.

In some examples not according to the invention, an electrode housing is integrated with a buckle of the band. In some aspects, an electrode housing is slidable across the band. In some examples not according to the invention, the electrode housing is configured to be positioned on a midline of the wrist in a secured orientation. In some examples not according to the invention, the electrode housing is wider than the band and configured to house two electrodes, wherein a combined width of the two electrodes is greater than a width of the band.

### BRIEF DESCRIPTION OF THE DRAWINGS

The foregoing is a summary, and thus, necessarily limited in detail. The above-mentioned aspects, as well as other aspects, features, and advantages of the present technology are described below in connection with various implementations, with reference made to the accompanying drawings.
**FIG. 1A** illustrates one implementation of a multivariate system for stroke detection.
**FIG. 1B** illustrates another implementation of a multivariate system for stroke detection.
**FIG. 2** shows blood pressure pulse in various parts of the body.
**FIG. 3** illustrates one implementation of a wearable device for stroke detection.
**FIG. 4** illustrates another implementation of a wearable device for stroke detection.
**FIG. 5** shows that as a wearable device is moved so does the plane of action, causing the accelerometer to track the change of plane and accordingly adjust the movement in three dimensions.
**FIG. 6** shows measurement of azimuth, roll and pitch by an accelerometer.
**FIG. 7** shows one implementation of a data capture workflow involving movement data measurements (e.g., acceleration).
**FIG. 8** shows one implementation of a workflow for calculating tremor measurements from captured acceleration data.
**FIG. 9** shows a graphical representation of acceleration data analyzed using an application on a computing device.
**FIG. 10** shows a graphical representation of distance data analyzed using an application on a computing device.
**FIG. 11** shows a graphical representation of movement data analyzed using an application on a computing device.
**FIG. 12** illustrates one implementation of a system for detecting symmetrical limb movement.
**FIG. 13** illustrates one implementation of a system for detecting asymmetrical limb movement.
**FIG. 14** illustrates another implementation of a system for detecting asymmetrical limb movement.
**FIG. 15** illustrates another implementation of a system for detecting symmetrical limb movement.
**FIG. 16** illustrates another implementation of a system for detecting asymmetrical limb movement.
**FIG. 17** illustrates another implementation of a system for detecting asymmetrical limb movement.
**FIG. 18** illustrates another implementation of a system for detecting symmetrical limb movement.
**FIG. 19** illustrates another implementation of a system for detecting asymmetrical limb movement.
**FIG. 20** illustrates another implementation of a system for detecting symmetrical limb movement.
**FIG. 21** illustrates another implementation of a system for detecting asymmetrical limb movement.
**FIG. 22** illustrates another implementation of a system for detecting symmetrical limb movement.
**FIG. 23** illustrates another implementation of a system for detecting asymmetrical limb movement.
**FIG. 24** illustrates another implementation of a system for detecting symmetrical limb movement.
**FIG. 25** illustrates another implementation of a system for detecting asymmetrical limb movement.
**FIG. 26** shows one implementation of an application on a computing device for comparing two sets of data from two limbs.
**FIG. 27** shows a graphical representation of acceleration data from two wrists.
**FIG. 28** shows a graphical representation of distance data from two wrists.
**FIG. 29** shows a graphical representation of movement data from two wrists.
**FIG. 30** shows a graphical representation of movement data from two wrists, while using a zoom feature of an application on a computing device.
**FIG. 31** shows a graphical representation of distance data from two wrists.
**FIG. 32** shows a graphical representation of acceleration data from two wrists.
**FIG. 33** illustrates one implementation of an architecture of a data processing module.
**FIG. 34** illustrates one implementation of machine learning model used to model movement patterns of a person, for example while sleeping.
**FIG. 35** illustrates another implementation of machine learning model used to model movement patterns of a person.
**FIG. 36** illustrates another implementation of machine learning model used to model movement patterns of a person.
**FIG. 37** illustrates an implementation of a system for detecting stroke.
**FIG. 38** illustrates an implementation of a digital "FAST" test.
**FIG. 39** illustrates an implementation of a system for detecting stroke that is configured to stimulate a response symmetrically and measure an output of the response to determine whether the response is symmetrical or asymmetrical.
**FIG. 40** illustrates an implementation of a wearable system for detecting an anomalous biologic event.
**FIG. 41** illustrates another implementation of a wearable system for detecting an anomalous biologic event.
**FIG. 42** illustrates a support structure coupled to the heat source of one implementation of a wearable system for detecting an anomalous biologic event.
**FIG. 43** illustrates a cross-sectional view of a wearable system for detecting an anomalous biologic event.
**FIG. 44** illustrates one implementation of a tensionable band for coupling a wearable system to a skin surface.
**FIG. 45** illustrates a first and second wearable system for measuring response asymmetry across a right and left limb, respectively.
**FIG. 46A** illustrates in graph form a method of processing a signal received from a blood volume sensor.
**FIG. 46B** illustrates in graph form a method of monitoring a heating cycle and a corresponding vasodilation response over time.
**FIG. 47** illustrates in graph form a vasodilation response of a skin surface over time and in response to application of heat.
**FIG. 48** shows a method of detecting an anomalous biologic event by measuring a vasodilation response of a skin surface over time in response to application of heat.
**FIG. 49** illustrates an implementation of a thermal stimulator integratable into a wearable system.
**FIG. 50** illustrates another implementation of a thermal stimulator integrated into a wearable system.
**FIG. 51** illustrates an in-ear wearable system for measuring one or more biometrics.
**FIG. 52** illustrates a method of detecting an anomalous biologic event.
**FIG. 53** illustrates a method of measuring heart rate variability of a user.
**FIGS. 54-55** show graphs comprising electrocardiogram data for detecting an anomalous biologic event.
**FIG. 56** shows a graph comprising asymmetrical electrodermal activity data for detecting an anomalous biologic event.
**FIG. 57** shows a graph comprising various parameters of interest in electrodermal activity data.
**FIG. 58** shows a method for measuring heart rate variability of a user and various feature analyses.
**FIG. 59** shows a time domain analysis of heart rate variability data.
**FIG. 60** shows a geometrical analysis of heart rate variability data.
**FIG. 61** shows a frequency domain analysis of heart rate variability data.
**FIG. 62** shows a nonlinear analysis of heart rate variability data.
**FIG. 63** shows a method of measuring a skin conductance response.
**FIG. 64** shows a graph comprising asymmetrical skin conductance response over time.
**FIG. 65** shows a graph comprising amplitude of an asymmetrical skin conductance response over time.
**FIGS. 66A-66G** show implementations of a wearable device for detecting an anomalous biologic event featuring various arrangements of electrodes.
**FIGS. 67A-67I** show various views of an implementation of a wearable device for detecting an anomalous biological event.
**FIGS. 67J1-67K3** show various views of an implementation of a wearable device for detecting an anomalous biological event featuring an electrode housing on a band.
**FIGS. 67L1-67N2** show various views of an implementation of a wearable device for detecting an anomalous biological event featuring a device body.
**FIG. 67O** shows an implementation of a heat source for a wearable device for detecting an anomalous biological event.
**FIG. 67P** shows an implementation of an ambient temperature sensor and cover for a wearable device for detecting an anomalous biological event.
**FIGS. 67Q-67U** show various views of an implementation of a wearable device for detecting an anomalous biological event.
**FIG. 68** shows another implementation of a wearable device for detecting an anomalous biologic event with certain details circled for emphasis.
**FIGS. 69A-69C** show various views of an implementation of a wearable device for detecting an anomalous biologic event.
**FIGS. 69D-69K** show various views of another implementation of a wearable device for detecting an anomalous biologic event.
**FIGS. 70A-70D** show various views of another implementation of a wearable device for detecting an anomalous biologic event.
**FIGS. 71A-71F** show various views of another implementation of a wearable device for detecting an anomalous biologic event.
**FIGS. 72A-72G** show various views of another implementation of a wearable device for detecting an anomalous biologic event.
**FIGS. 73A-73E** show various views of another implementation of a wearable device for detecting an anomalous biologic event.
**FIGS. 74A-74G** show various views of another implementation of a wearable device for detecting an anomalous biologic event.
**FIGS. 75A-75D** show various views of another implementation of a wearable device for detecting an anomalous biologic event.
**FIGS. 76A-76H** show various views of another implementation of a wearable device for detecting an anomalous biologic event.
**FIGS. 77-81** show various views of various implementations of a wearable device for detecting an anomalous biologic event.
**FIG. 82** shows an example diagram of a heating assembly for installation in the wearable devices described herein.
**FIGS. 83A-83C** show various example layers of a heating assembly.
**FIG. 84** shows a diagram of an example thermal image 8400 captured during warming of a heating element.
**FIGS. 85A-D** show an example switching circuit for use with the electrodes, sensors, and heating elements described herein.
**FIGS. 86A-B** show exemplary electrode assemblies.

The illustrated implementations are merely examples and are not intended to limit the disclosure. The schematics are drawn to illustrate features and concepts and are not necessarily drawn to scale.

### DETAILED DESCRIPTION

The foregoing is a summary, and thus, necessarily limited in detail. The above-mentioned aspects, as well as other aspects not according to the invention, features, and advantages of the present technology will now be described in connection with various implementations. The inclusion of the following implementations is not intended to limit the disclosure to these implementations, but rather to enable any person skilled in the art to make and use the contemplated invention(s). Other implementations may be utilized, and modifications may be made without departing from the scope of the subject matter presented herein. Aspects of the disclosure, as described and illustrated herein, can be arranged, combined, modified, and designed in a variety of different formulations, all of which are explicitly contemplated and form part of this disclosure.

Described herein are systems, devices, and methods for multivariate detection of stroke or at minimum, a deviation from baseline. Multivariate may include using more than one, at least two, or a plurality of factors, markers, or other parameters to detect stroke. In some implementations, multivariate may include using one parameter measured at multiple locations or positions or at multiple times (e.g., random or fixed intervals, on demand, automatically, continuously, etc.). In various implementations, multivariate may include detecting a measured parameter symmetrically or asymmetrically. The measured parameter may include a functional parameter (e.g., gait, speech, facial changes, etc.); a biological parameter or marker (e.g., blood proteins, metabolites, etc.); a quantitative parameter (e.g., limb asymmetry, heart rate variability, etc.); a spatial (e.g., neck vs. chest; arm vs. leg; etc.) difference in one or multiple (e.g., 2, 3, 4, 5, 10, 15, 20, etc.) measured parameters; and/or a temporal difference in one or multiple measured parameters.

In some implementations, there may be an overlay of multivariate signals including two measurement data types, physiological (e.g., skin electric potential, Doppler flow signal anomaly, hyperhidrosis, cutaneous blood flow, brain perfusion, heartrate variability, etc.), and/or clinical manifestations or functional parameters (e.g., limb asymmetry, speech slur, facial droop, retinal abnormality, etc.). Clinical manifestations occur following stroke onset, but a faint signal from a clinical manifestation measurement combined with a physiological signal measurement may detect or predict stroke likelihood prior to stroke onset. Parameters that may be measured before, during, or after a stroke include quantitative parameters, functional parameters, and/or blood/fluid parameters. Any of the parameters shown/described herein may be measured asymmetrically, as described elsewhere herein. Exemplary, non-limiting examples of quantitative parameters include: volumetric impedance spectroscopy, EEG asymmetry, brain perfusion, skin/body temperature (e.g., cold paretic limb, up to 6°C colder or 16% colder than non-paretic limb), hyperhidrosis (e.g., greater than 40-60% increase on paretic limb), limb asymmetry, drift and pronation test, cutaneous blood flow, muscle tone, heartrate variability (e.g., decrease in spectral components by greater than 10X, lasting 3-7 days after stroke onset), facial surface electromyogram (EMG), cerebral blood flow (CBF), carotid artery stenosis, salivary cortisol, neuron specific enolase (NSE), salivary NSE, etc. Exemplary, non-limiting examples of functional parameters include: speech changes, speech comprehension, text comprehension, consciousness, coordination/directions, facial muscle weakness, arm weakness, body weakness (e.g., grip), leg weakness, foot weakness, unilateral weakness, difficulty walking, vertigo, sudden vision problems, limited visual field, altered gaze, thunderclap headache, nuchal rigidity (nape of neck), respiration, blood pressure (e.g., increase up to 60% in both systole (200 mHg) and diastole (140 mmHg)), etc. Exemplary, non-limiting examples of blood/fluid parameters include: CoaguCheck (Roche), HemoChron (ITC), iSTAT (Abbott), Cornell University, ReST (Valtari Bio Inc.), SMARTChip (sarissa Biomedical), etc.

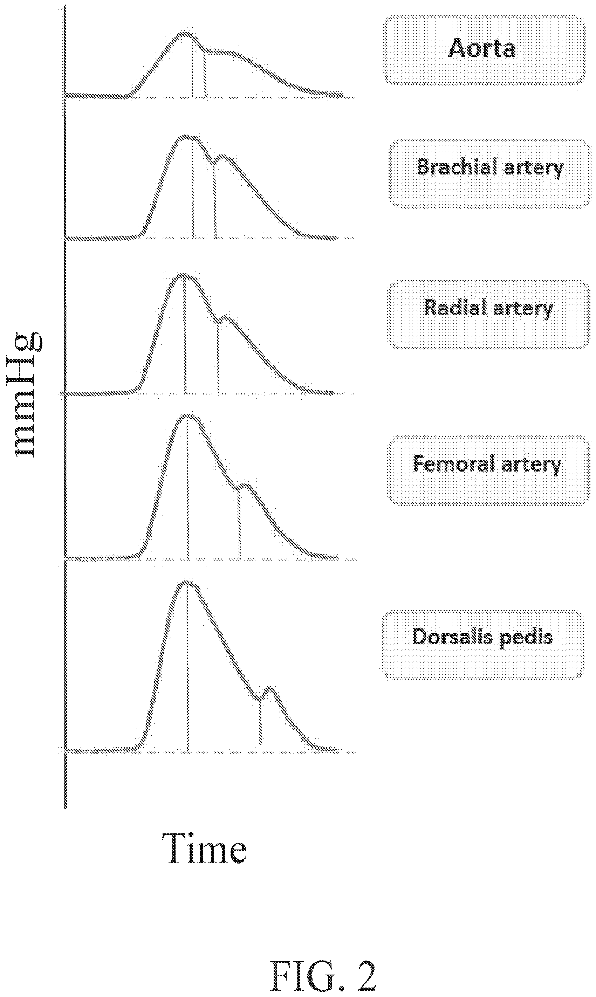

In some implementations, multiple measurement locations (e.g., radial, brachial, etc. vessels) may be used to measure a difference in signal or data pattern among those locations compared to nominal, healthy location measurements or compared to an individual baseline as an input into a data processing module. For example, an individual baseline may be recorded over time and, when an adverse event occurs, a change (e.g., absolute or relative value) from baseline is determined unilaterally or bilaterally. In some implementations, after the adverse event occurs, a new baseline may be established. Further for example, as shown in FIG. 2, blood pressure pulse varies depending on the location in the body, demonstrating that a slightly different signal is measured depending on location. For example, if only one location is measured, then changes over time are observed. If multiple locations are monitored and/or measured, then changes over time and changes relative to one another and/or a baseline can be used to identify a pattern or an asymmetric signal occurrence. In some implementations, an individualized baseline is further calculated based on a patient's health history (e.g., diabetes, heart-pacing, pre-existing stroke, etc.), demographics, lifestyle (e.g., smoker, active exerciser, drinks alcohol, etc.), etc.

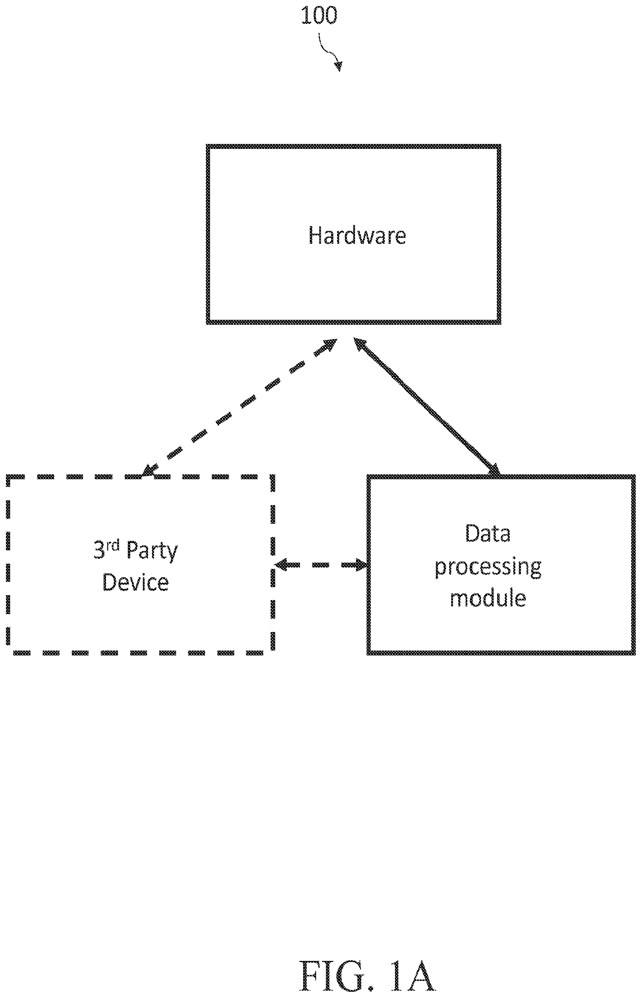

In some implementations, as shown in FIGS. 1A-1B, a system 100 for multivariate detection of stroke includes a hardware component (e.g., wearable device, sensor, computing device, remote sensing device, etc.) and a data processing module stored in the hardware or in communication with the hardware. The hardware component, for example one or more sensors, may be positioned on a user of the system, bilaterally on a user of the system, or throughout a location occupied by a user. Optionally (shown by dashed lines), a system for multivariate stroke detection may further include a third party device, for example a device including Amazon^{®} Alexa^{®} or an Amazon^{®} Echo^{®} device, as described in further detail elsewhere herein. For example, there may be bidirectional communication (e.g., via a wired connection or wireless communication) between the hardware component and the data processing module, the data processing module and the third party device, and/or the third party device and the hardware component.

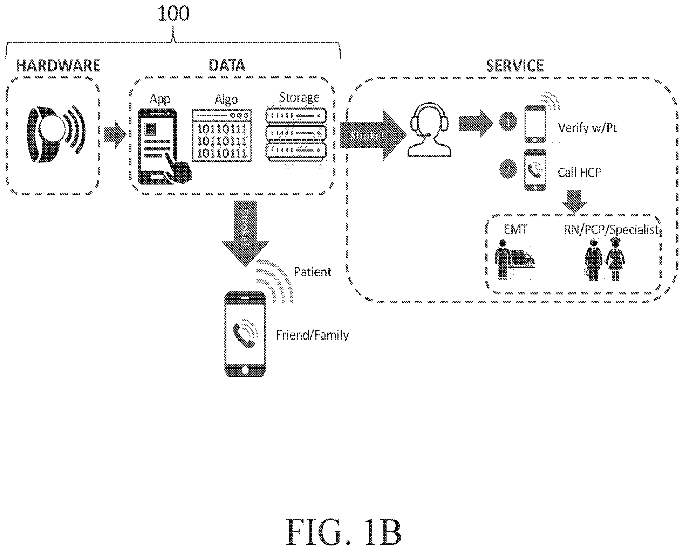

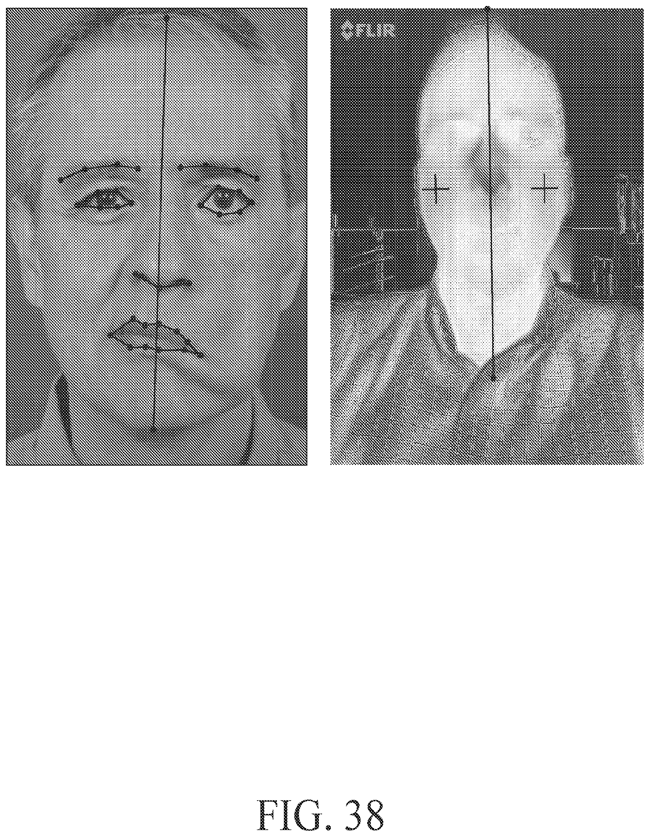

In one exemplary, non-limiting implementation of the system of FIGS. 1A and 1B, a digital FAST (i.e., facial drooping, arm weakness, speech difficulties, time for help) test may be performed by the system of FIGS. 1A-1B. For example, the hardware component may include one or more cameras positioned throughout a location occupied by a user and configured to detect changes (e.g., using computer vision techniques) in facial expressions (e.g., drooping) as a result of stroke, as shown in FIG. 38 (i.e., the "F" part of a FAST test). Further, one or more sensors or other hardware component (e.g., camera, microphone, etc.) may be positioned throughout the location occupied by user. The one or more sensors are communicatively coupled to the data processing module such that parameters sensed by the sensors may be transmitted to the data processing module for digitization, filtering, process, and/or analysis. In the case of a digital FAST test, asymmetrical arm weakness may be sensed by the one or more sensors. To discern speech difficulties, a third party device configured to receive and assess speech quality may be communicatively coupled to the data processing module and/or hardware component. As such, a user may be prompted to speak by the third party device and the user's response may be sensed by the hardware component (e.g., one or more microphones) so that a quality of speech of the user may be determined. One or more of these detected parameters may be analyzed and optionally sent to a caregiver, approved family and/or friends, healthcare provider, physician, and/or emergency services.

In some implementations, a system for multivariate stroke detection may further include an application downloaded and/or stored on a hardware component or downloaded and/or stored on a computing device (e.g., mobile computing device) communicatively coupled to the hardware component. The application may be configured to process sensor data, camera data, speech data, etc. and/or display data sensed or captured in real time, for example in a graphical representation, and/or allow zooming to view various features of the data.

In some implementations, data may be transmitted to and/or from the device for detecting stroke to a central hub, mobile computing device, server, or other storage and/or computing device. Data transmission may include wireless communication (e.g., a nearfield communications (NFC) protocol, a low energy Bluetooth^{®} protocol, other radiofrequency (RF) communication protocol, etc.) between sensor locations on the body and/or a central hub. In other implementations, data transmission may include wire communication between sensor locations on the body and/or a central hub. In some implementations, the central hub may be a monitor in a medical facility, home monitor, patients' mobile computing device, or other wireless device. Alternatively, one or more of the sensors on the body may act as the central hub. The hub device may wirelessly send signals to activate a medical care pathway and/or notify one or more individuals (e.g., family, friends, physician, EMS, etc.).

In some implementations, data transmission, following multivariate analysis, to the central hub may alert the patient, the next of kin, and/or a third party to identify possible false positives or negatives.

In some implementations, a device for stroke detection may be worn on an exterior or skin surface of the patient or implanted as hardware prior to and/or during stroke, including up to days before the event and during the event to provide continuous variable monitoring of various physiological parameters. The various implementations described herein may either be a wearable device or an implantable device.

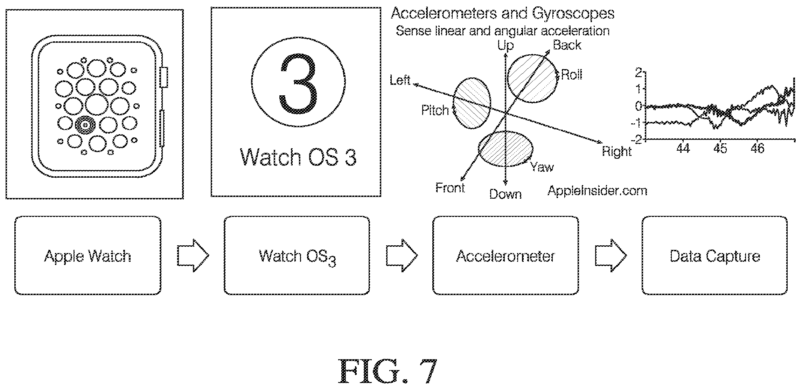

In some implementations, a device for detecting stroke may include a wearable device, for example a patch, headband or sweatband, ring, watch (e.g., to measure movement as shown in FIG. 7), adhesive strip, helmet, bracelet, anklet, sock (e.g., to measure heart rate, heart rate variability, temperature, gait, etc.), shoe insoles (e.g., to measure heart rate, heart rate variability, temperature, gait, etc.), clothing, belt, necklace, earring (e.g., over or in the ear to measure heart rate, heart rate variability, EEG asymmetry, etc.), hearing aid, earbuds, glasses or sunglasses or smart glasses (e.g., to measure EOG, EMG, sEMG, EEG, gaze, facial muscle movement or drooping, etc.), smart tattoo (e.g., to measure EEG, ECG, etc.), bra, bra clip, chest strap, contacts (e.g., to measure tear composition, etc.), mouthguard or bite splint (e.g., to measure saliva neuron specific enolase, cortisol, temperature, motion, etc.), hat or cap (e.g., to measure various signals using ultrasound), wearable speaker (e.g., to measure heart rate, heart rate variability, motion, etc.), or otherwise a sensor integrated into any wearable clothing, accessory, or device. For example, a patch (e.g., wearable on the neck) may be used to estimate cerebral blood flow using doppler ultrasound, blood oxygen content, or other blood feature as an indicator of blood going into the brain (carotid artery) or leaving the brain (jugular vein); a patch or strip (e.g., wearable on the head) may be used to detect EEG or sEMG. Further for example, a wearable device for detecting stroke may include one or more transdermal sensors that are configured to measure changes in one or more gasses transfused through the skin (e.g., nitric oxide (NO) could either be measured directly, or through measurement of particular bi-products); one or more biomarkers that are in the blood that are diffused into the subcutaneous region or into the epidermis and can be measured externally. In some implementations, such a wearable device may be configured to monitor indicators of high NO in the blood. For example, changes in vasodilation may indicate particular NO levels in the blood. In some implementations, a wearable device for detecting stroke may comprise a wristband or patch with a combination of micro-needles that are configured to measure the fluid sub-dermally or interstitial fluid (e.g., similar to continuous glucose monitors).

In some implementations, a wearable device for detecting stroke may comprise a wearable array of indicators (e.g., chromogenic indicators) configured to measure a chemical, analyte, protein, etc. in a bodily fluid of an individual (e.g., blood, interstitial fluid, etc.). For example, the array may comprise a membrane with a printed array thereon that when exposed to one or more analytes, a subset of the indicator spots responds by changing color or properties. The color response of the indicators may be optically read, for example using a camera on a computing device or other image sensor and compared to a baseline reading or a reference or standard. A color difference map may be generated by superimposing and/or subtracting the two images (baseline and experimental or experimental and reference/standard). As an exemplary, non-limiting analyte, an increase in nitric oxide may be detected in blood or interstitial fluid of an individual after a stroke event and/or modification of one or more proteins by nitric oxide may be detected in blood or interstitial fluid of an individual after a stroke event and/or one or more intermediates or byproducts of nitric oxide may be detected in blood or interstitial fluid of an individual after a stroke event. For example, nitric oxide has been shown to modify proteins via: 1) binding to metal centers; 2) nitrosylation of thiol and amine groups; 3) nitration of tyrosine, tryptophan, amine, carboxylic acid, and phenylalanine groups; and 4) oxidation of thiols (both cysteine and methionine residues) and tyrosine. Such methods may bypass the need to measure an asymmetrical change in one or more parameters, as described elsewhere herein.

In some implementations, a system for stroke detection may include one or more Doppler radar sensors, microphones, and cameras throughout a home to detect visual signs of stroke, equivalent to a "FAST" test using computer vision or similar techniques, as shown in FIG. 38. For example, a machine learning model may be trained on a training data set of images of stroke patients to identify asymmetrical facial features, such as facial drooping. As can be seen in FIG. 38, the system is able to identify drooping in a mouth, nose, and eye positioning of the patient. Facial capillary asymmetries via high frame-rate Eulerian video processing techniques may also be detected by the systems described herein. The system may further employ confirmation biometrics such as HR/HRV, respiratory rate (e.g., via Doppler radar), and/or bilateral temperature via infrared camera (i.e., FLIR)

In some implementations, a device for detecting stroke may include a device positionable in a room, office, home, vehicle, or other location; or in or on a bed or other furniture (e.g., bedside monitors; monitors within mattresses, bedding, etc.). For example, a smart speaker (e.g., to prompt a user to respond to a question to analyze speech quality), microphone, camera, and/or mirror may be positionable in a location to detect changes in a user's speech, activities, movement, gait, facial appearance, heart rate, and/or heart rate variability or changes from baseline. The device may comprise a data processing module to differentiate changes in the measured parameters as compared to that from healthy learned patient data or individualized baseline data.

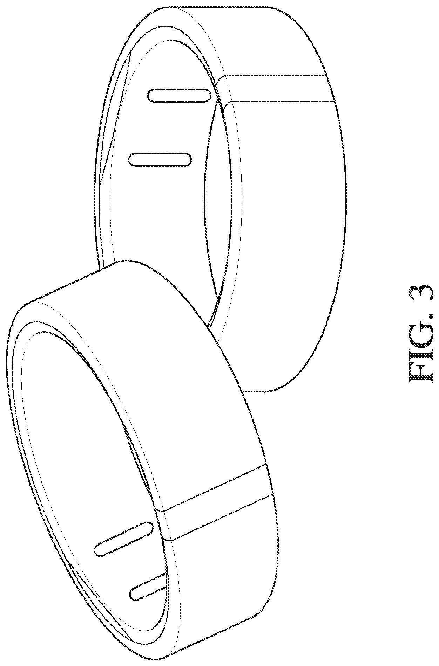

In some implementations, as shown in FIG. 3, the device may be a ring or a pair of rings to be worn one on each hand or each foot to measure temperature; volumetric impedance spectroscopy; hyperhidrosis; heart rate or heart rate variability through, for example, a PPG sensor to monitor rate of blood flow; and/or motion (e.g., by including an accelerometer and/or gyroscope therein) to measure, for example, limb asymmetry or changes in gait. Temperature measurement devices may include, but are not limited to, infrared sensors, thermometers, thermistors, or thermal flux transducer. Hyperhidrosis measurement devices may include, but are not limited to, detection of analytes including ions, metabolites, acids, hormones, and small proteins through potentiometry, chronoamperometry, cyclic voltammetry, square wave stripping voltammetry, or detection of changes in conductivity. Sensor measurement devices may include, but are not limited to, a photoplethysmographic (PPG) device, a skin conductance sensor measuring skin conductance/galvanic skin response (GSR) or electrodermal activity (EDA), or a skin temperature measurement device (e.g., contact devices and non-contact devices, like IR imaging camera).

In some implementations, the ring may incorporate a stretchable or expandable element or stretch sensor to allow the ring to expand or stretch when the finger, wrist, ankle, etc. swells. This element may include, but is not limited to, elastomer film polymers of various degree of bonding to allow for different pliable elements or measuring the reflectivity of polarized light. This element may comprise a plastic segment of the ring that can be loosened/tightened, or by building a slidable element that can be pulled apart. Non-limiting examples of a stretch sensor include, but are not limited to, a strain gauge or an electrical component configured to change inductance, resistance, or capacitance when stretched.

In some implementations, the device may be a strip that measures brain waves through electroencephalogram (EEG) and/or muscle contractions through surface electromyography (sEMG). The measurement of EEG may be compared to a baseline value to detect a change or asymmetry of the EEG. In some implementations, EMG measures facial muscle changes compared to a baseline measurement to identify muscle weakness and tone.

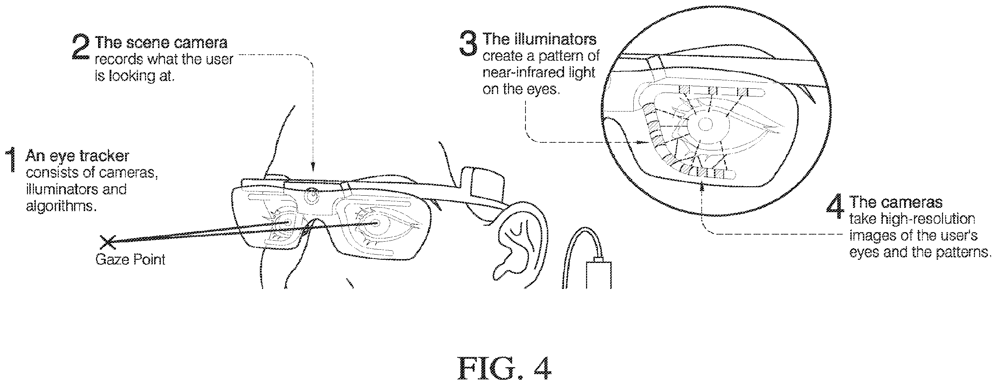

In some implementations, as shown in FIG. 4, the device may be a wearable eyeglass device that measures electrooculography (EOG), EMG, EEG, gaze, and facial muscle symmetry. The measurement of EOG identifies a change in the corneo-retinal standing potential between the front and back of the eye that may detect a change in gaze and size of visual field and may be compared to either the other eye or a previous baseline value. In other implementations, gaze or facial expression may be a network of sensors including, for example non-contact devices placed in an environment.

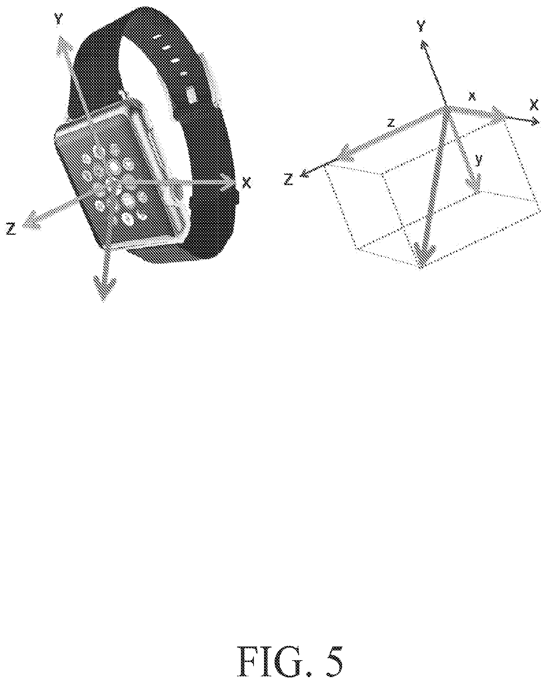

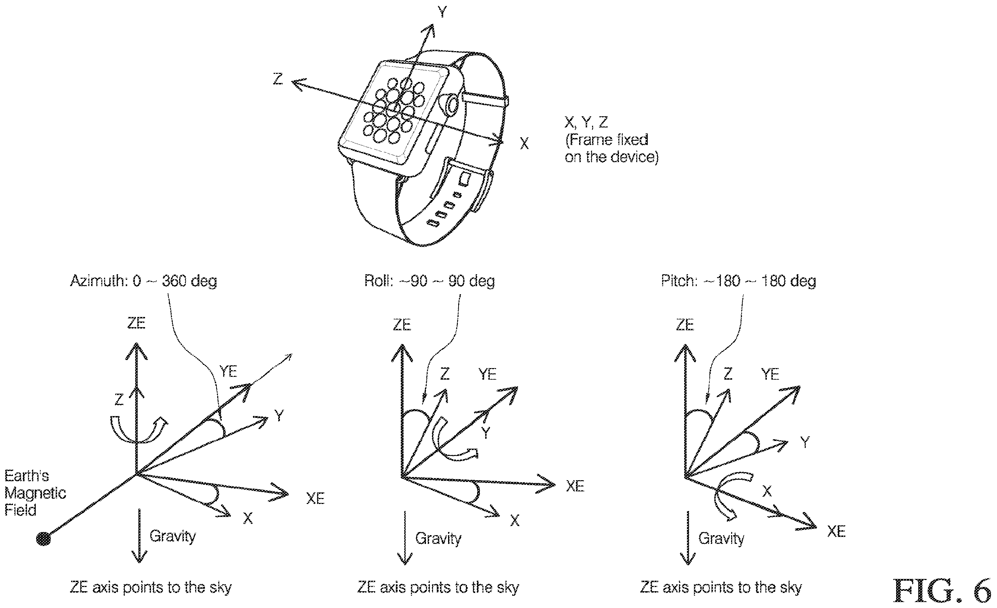

In some implementations, as shown in FIGS. 5-6, a device for stroke detection may include a wearable device for measuring changes in motion (e.g., in three axes), for example asymmetrical motion to detect tremors. In some implementations, a device for stroke detection may include a wearable device for measuring changes in motion (e.g., in three axes), for example asymmetrical changes in motion to detect tremors. Such device may include an accelerometer, gyroscope, inclinometer, compass, or other device for measuring acceleration, distance, and/or movement. For example, as shown in FIG. 5, as the wearable device is moved so does a plane of action. The accelerometer may track a change of plane and accordingly adjust the movement in three dimensions. Further, as shown in FIG. 6, an accelerometer may track azimuth, roll and pitch.

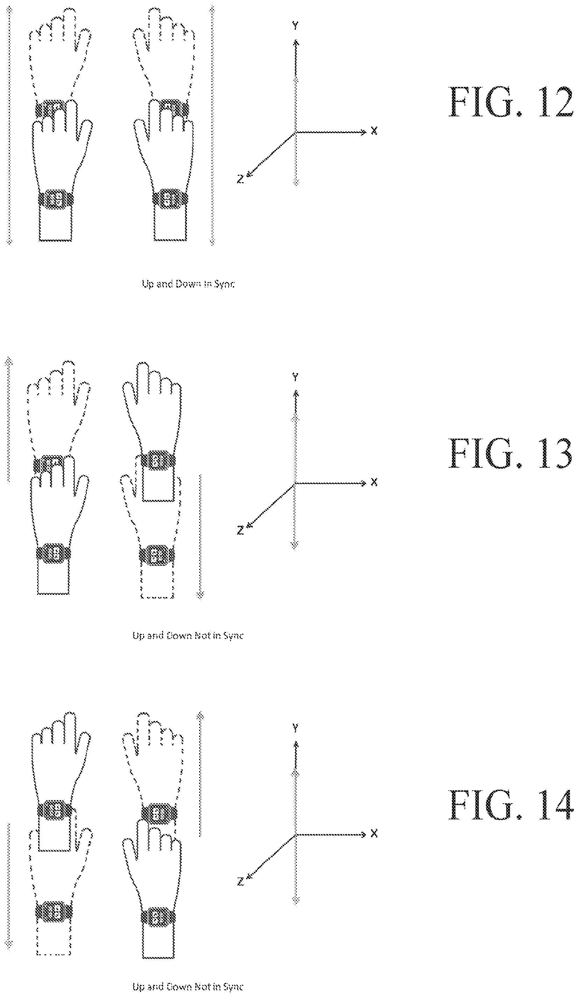

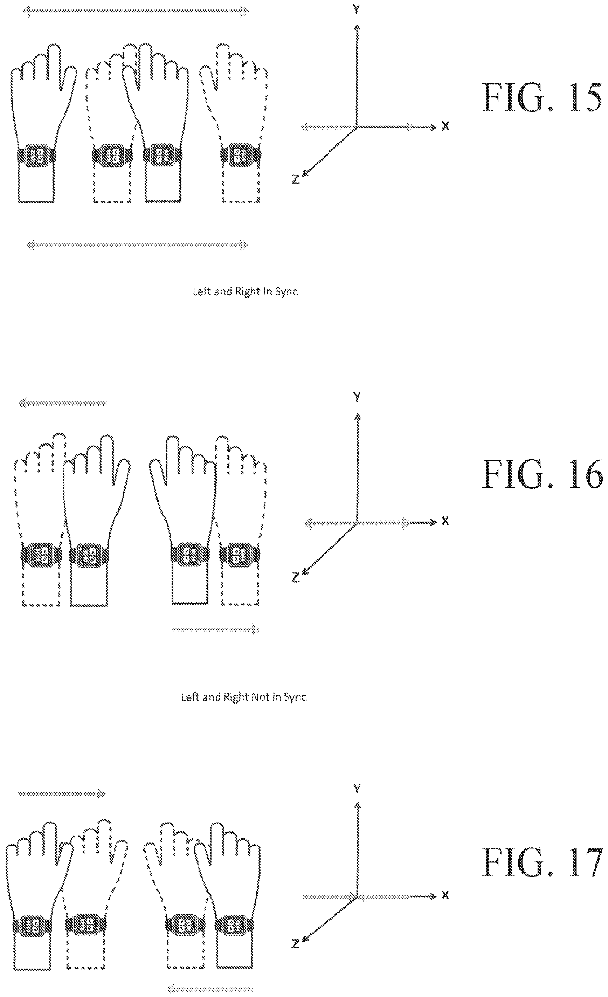

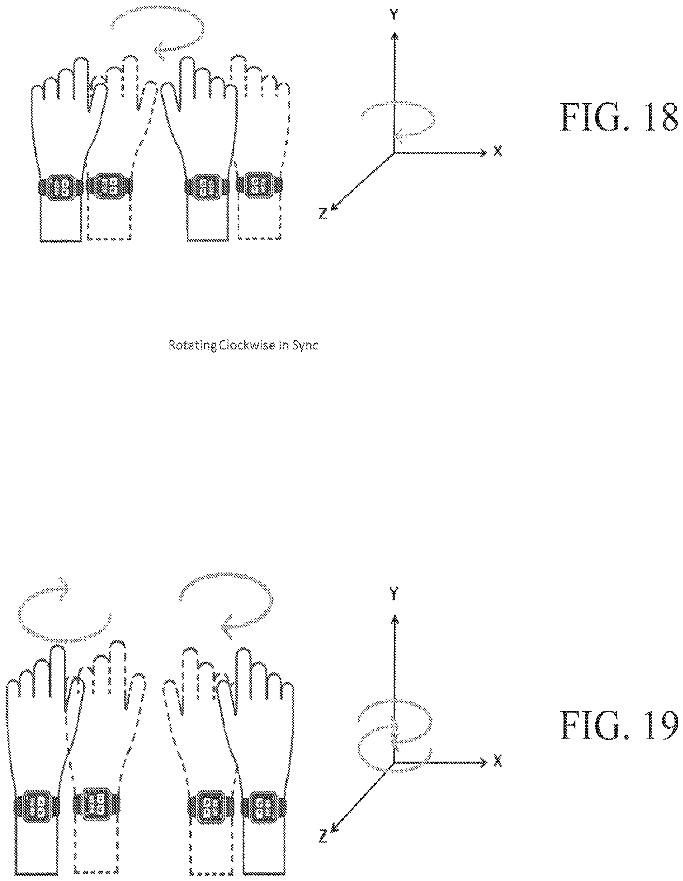

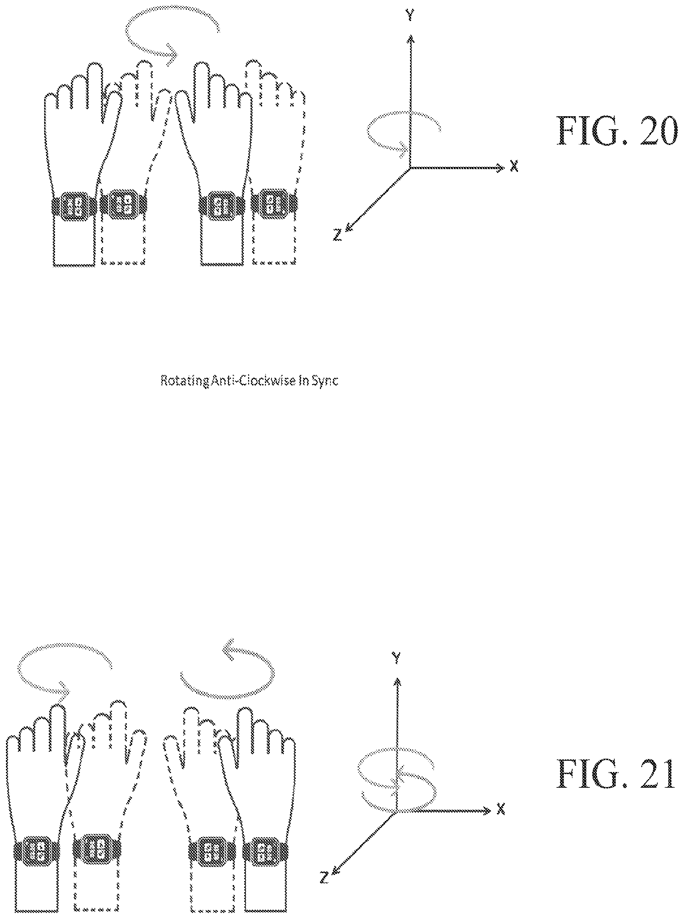

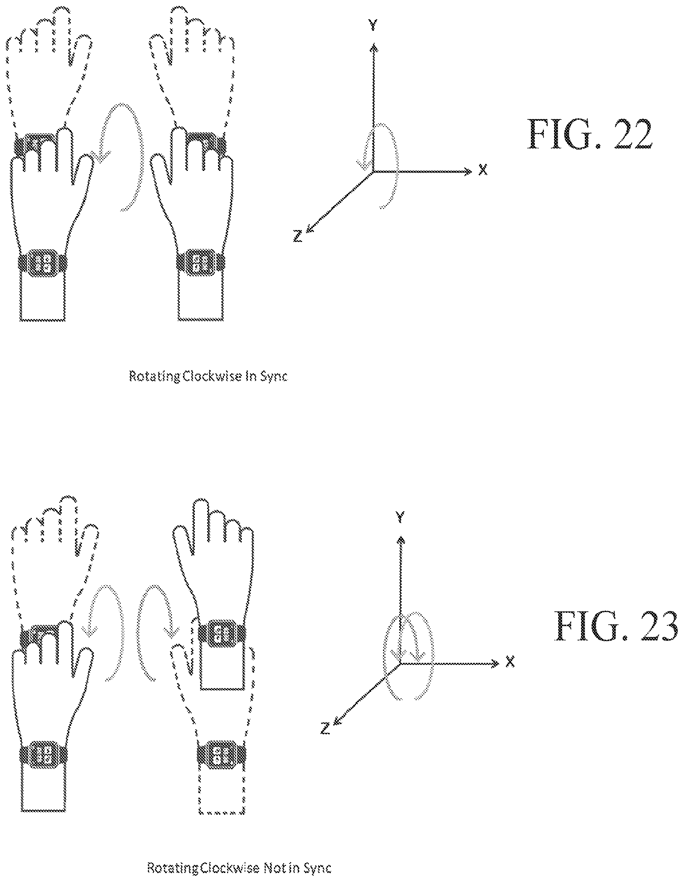

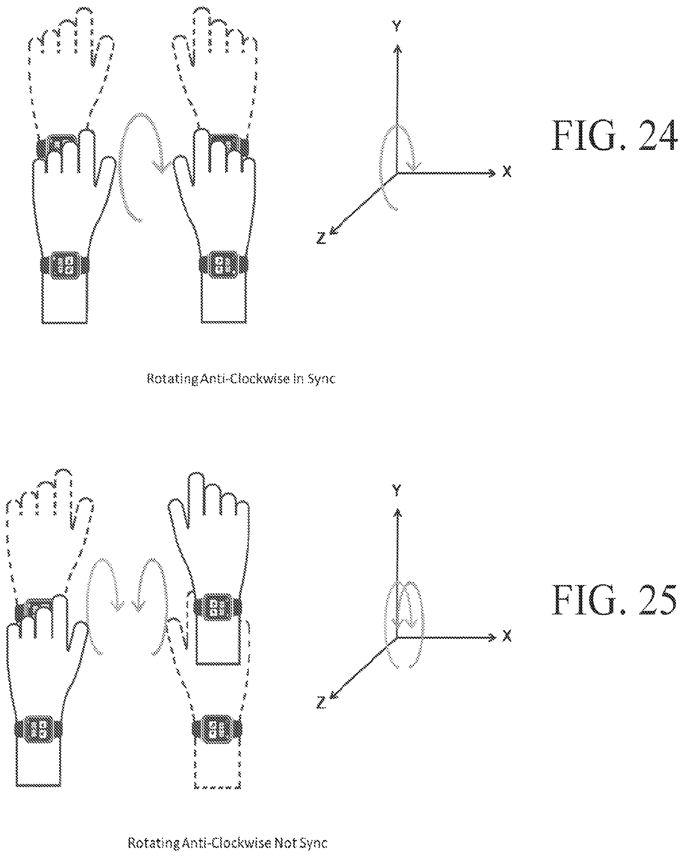

In some implementations, a device for detecting stroke may be configured to detect asymmetrical responses, outputs, or signals or deviation(s) from a baseline. For example, one or more devices (e.g., ring, watch, etc.) described herein may be used to measure symmetrical and asymmetrical limb movement. FIGS. 12-25 show various symmetrical and asymmetrical movements that may be measured by one or more implementations described herein. For example, FIGS. 12, 15, 18, 20, 22, and 24 show various implementations of symmetrical movements (e.g., up and down movement, left and right movement, rotational movement, etc.) between two limbs measurable by various devices described herein. FIGS. 13-14, 16-17, 19, 21, 23, and 25 show various implementations of asymmetrical movements (e.g., up and down movement, left and right movement, rotational movement, etc.) of limbs measurable by various devices described herein.

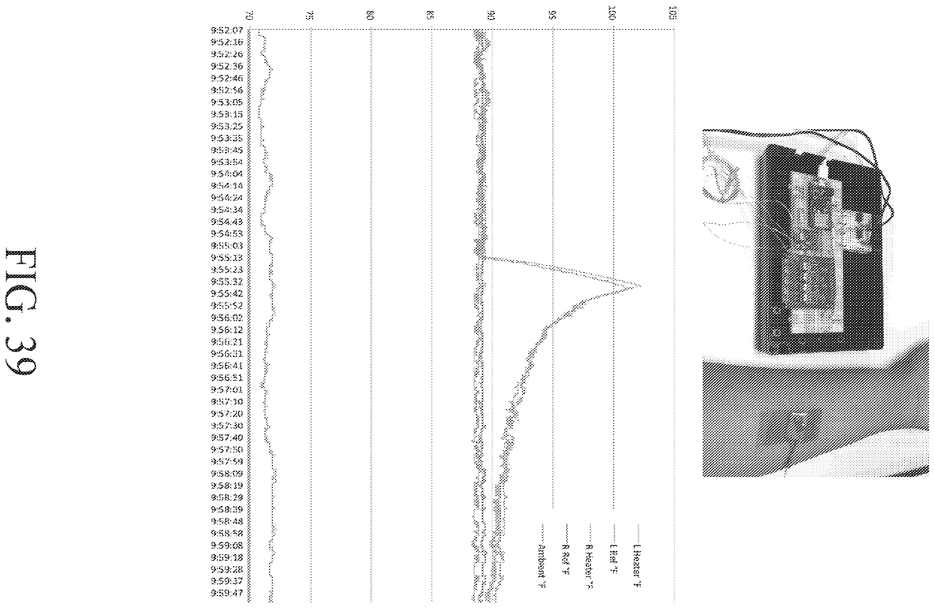

In some implementations, as shown in FIG. 39, a device or system for detecting stroke may be configured to stimulate a response and measure the response on each side (e.g., to detect asymmetrical responses) of the body of the user to determine whether the response or the difference in response between the two sides indicates a stroke event, or at least a deviation from baseline. For example, the stimulus may be applied in a stimulus cycle such that the baseline, during stimulation, and post stimulation responses are measured, or even change in (e.g., slope, decay, etc.) between different measurement periods. For example, a thermal (i.e., hot or cold) stimulus may be applied to a section of skin on a body of a user (shown in top panel) and the body's response to the thermal stimulus may be monitored over time (shown in bottom panel) to determine whether homeostasis is reached and/or a difference in response or return rate exists between the two sides of the body (in other words, determine whether an asymmetrical response exists). Further examples include stimulating the muscular or nervous system using electrical signals and monitoring the response over time and/or between sides using electromyogram (EMG), bioimpedance, or electroneurogram (ENG), respectively. These "stimulators/transmitters" and "receivers/detectors" could be in the same region or could be separated to measure across regions of the body.

As discussed above, if a stroke is detected and patients seek care quickly, it can dramatically reduce death and disability. Continuous monitoring for a stroke event may improve the response time. However, continuous monitoring of anomalous biologic events such as stroke events using existing monitors can be challenging. These monitors are cumbersome and may be difficult for users to wear over an extended period of time. In contrast, the inventors realized that wearable devices, such as watches with integrated sensors and electronics may improve continuous monitoring of stroke events. An impaired vasodilation response may be indicative of a stroke, heart failure, hypertension, diabetes, or other conditions.

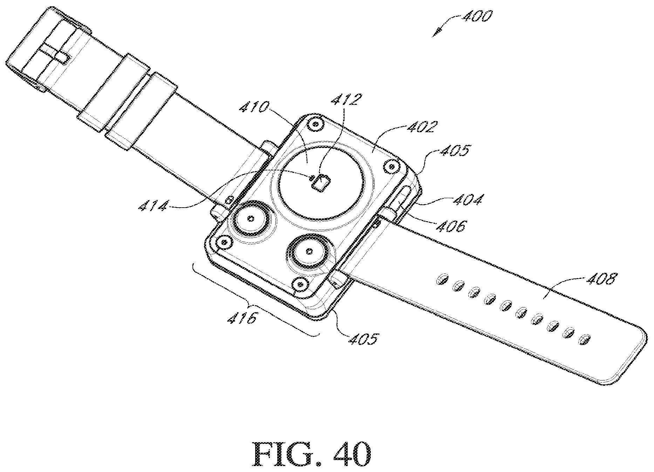

Applying heat stress to a portion of the skin may enable detection of vasodilation response. Accordingly, systems and methods described below enable detection of impaired vasodilation in a form factor that improves continuous anomalous cardiac event monitoring. In some implementations, as shown in FIG. 40, a system or device 400 for detecting an anomalous biologic event may function to heat a skin surface and measure a vasodilation response of the skin surface. The system or device 400 may further function to measure one or more additional parameters, biologic signals, etc. as will be described in greater detail elsewhere herein. In some implementations, the device 400 may use a measured bioimpedance (BioZ) to validate or invalidate a vasodilation measurement from another sensor on device 400.

In one example, a system or device 400 for detecting an anomalous biologic event may include a body 416 having a first surface 404 opposite a second surface 402 in contact with a skin surface of a person. The first surface 404 and second surface 402 may be coupled via one or more or a plurality of sidewalls 405. For example, one or more sidewalls 405 may extend from a perimeter of the first surface 404 and couple to a perimeter of the second surface 402. The first surface 404 and/or second surface 402 may include one or more sensors positioned thereon. For example, one or more sensors on the first surface 404 may measure an environment of the user wearing or using the wearable system, and one or more sensors on the second surface 402 may measure one or more properties, features, or characteristics of the skin surface of the user and thus the user itself. Alternatively, the first surface 404 may include one or more sensors or imagers or cameras for assessing a facial region of a user, for example, via a FAST test.

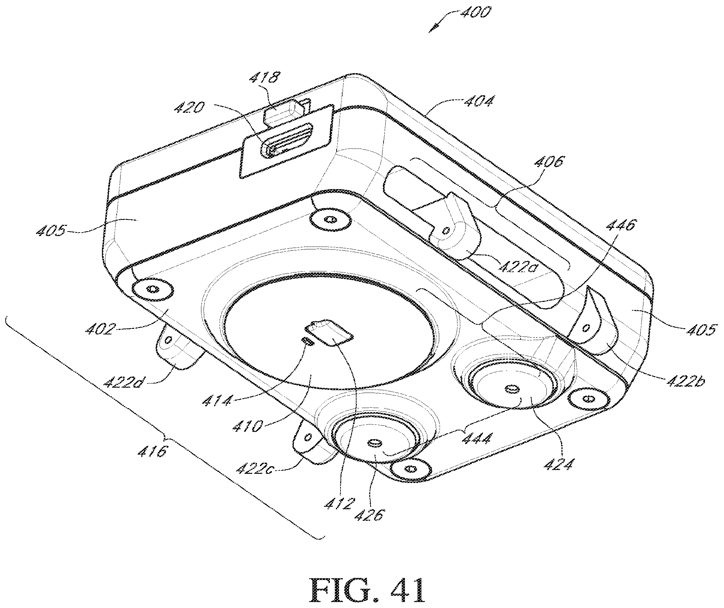

A wearable device 400 may be secured to a user, for example a limb of a user or a skin surface of a user, via a band 408, for example a tensionable band, which will be described in greater detail elsewhere herein. The band 408 may be adjustable such that the wearable device may be cinched or tensioned to promote greater contact and thus coupling between the wearable device and the skin surface or tension released to reduce contact or coupling between the wearable device and the skin surface. As shown in FIG. 41, a band 408 may be coupled to a body 416 of a wearable device via one or more connectors 422a, 422b, 422c, 422d. For example, a band 408 may couple to a body 416 of a wearable device via a connector 422 that includes one or more pin joints, a snap fit connection to the band 408, a slide and fit connection to the band 408, etc. When the tensionable band 408 is coupled to the body 416 via connectors 422, the tensionable band is centered with respect to one or more sensors positioned on the second surface, so that there is sufficient coupling between the sensors and the skin surface.

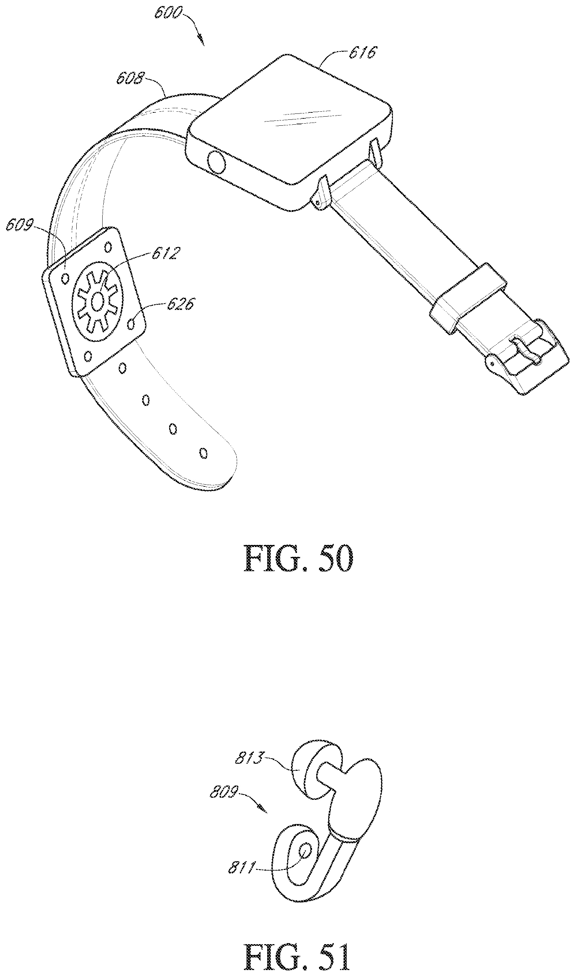

A wearable device 400 may include a heat source 410 in communication with the skin surface. The heat source 410 may be configured to heat the skin surface to a target temperature or a pre-determined temperature. The heat source 410 may be a heating element; thin film resistance flexible heater; polyimide heater; optical heater (e.g., a laser), etc. In other implementations, the heat source 410 an environmental heat source, for example a warm room, warm environment (e.g., under the covers, hot day, etc.). In such implementations, the stimulus may be a change in environmental temperature, for example, from a warm environment to a cool environment or a cool environment to a warm environment. In some implementations, a heat source 410 is positioned on a second surface 402 of the body 416, so that there is coupling or contact between the heat source 410 and a skin surface. Alternatively or additionally, a heat source 610 or one or more sensors 612, 626 may be positioned on a band 608 of the system 600, as shown in FIG. 50, such that the body 616 is separate from the sensor module 609 that includes the heat source 610 and the one or more sensors 612, 626. In some implementations, the heat source and/or one or more sensors may be distributed between the band, body, and sensor module depending on which sensors are incorporated into the system and their specific requirements or parameters.

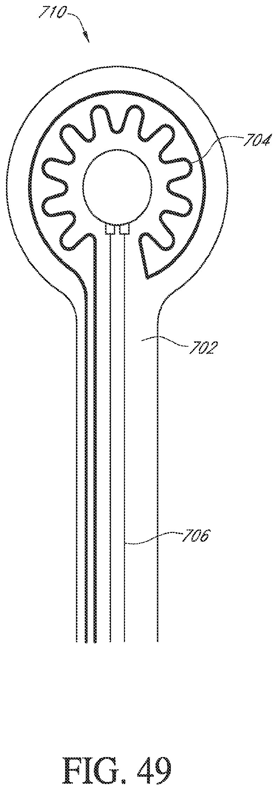

In some implementations, as shown in FIG. 49, a heat source 710 may comprise a thermal stimulator comprising a single printed layer of resistive ink on polyimide film 702. Heat traces 704 and traces to one or more sensors 706 (e.g., blood volume sensor, infrared sensor, temperature sensor, etc.) could also be likewise printed on the polyimide film 702, as shown in FIG. 49.

In a still further implementation, the sensor module 809 may be positionable in an in-ear device (e.g., ear lobe clip, ear bud, hearing aid, etc.), as shown in FIG. 51. The sensor module may be configured to measure one or more parameters, depending on which sensors 811, 813 are present, for example blood pressure, temperature, and/or oxygen saturation.

Further, the heat source 410 may be communicatively coupled to a hardware processor such that the hardware processor outputs a heating signal to the heat source 410 to activate the heat source to initiate a heating cycle. For example, a heating cycle may include receiving baseline temperature signals from a skin temperature sensor and an environmental temperature sensor (for example, ambient temperature sensor 6750 discussed below), determining the target temperature based on the baseline temperature signals, and determining whether the target temperature is below a maximum temperature value.

In some implementations, a target temperature may be equal to a baseline skin temperature as measured by the skin temperature sensor plus an offset, for example about 1 to about 20 degrees, about 1 to about 5 degrees, about 2 to about 10 degrees, about 2 to about 15 degrees, about 1 to about 10 degrees, about 5 to about 10 degrees, about 5 to about 15 degrees, about 8 to about 12 degrees, etc. In one implementation, the target temperature is equal to the baseline skin temperature as measured by the skin temperature sensor plus about 5 to about 15 degrees. In another implementation, the target temperature is equal to the baseline skin temperature as measured by the skin temperature sensor plus about 7 to about 13 degrees. In another implementation, the target temperature is equal to the baseline skin temperature as measured by the skin temperature sensor plus about 10 degrees. If the target temperature is greater than a maximum temperature value, the system pauses or delays until the baseline skin temperature drops below a minimum threshold or recalculates the target temperature so that it is less than the maximum temperature value. If the target temperature is less than a maximum temperature sensor, the system proceeds to activate the heat source to heat the skin surface to the target temperature.

In some implementation, the heat source cycles between the target temperature and a deactivated or off state or between the target temperature and a temperature that is lower than the target temperature but greater than the skin baseline temperature, for example to maintain the target temperature, hereinafter referred to as a dwell time.

In some implementation, a duration of a heating cycle and a target temperature are interconnected and based on user preference or user perception of heat on the skin surface or a vasodilation response of the user. For example, a higher target temperature may be used for a shorter time period or a lower target temperature may be used for a longer time period.

Further, the system or device 400 may be configured to receive one or more user inputs related to a perceived heat sensation on the skin surface and/or to a sensitivity of a vasodilation response of the user. For example, a user may input that the target temperature felt too hot or too cold, for example via a user input element (e.g., button), such that the system responds by reducing the target temperature but elongating an amount of time that the skin is heated. Additionally, or alternatively, based on user preference, preset configurations (e.g., during manufacturing), or as a result of sensed data (e.g., based on sensor data), the heat source may reach the target temperature via one of a plurality of ramping functions, for example slow ramping, larger step functions, etc. Alternatively, the heat source may reach the target temperature through a plurality of micro-stimulations. Further, for example, a target temperature may be individualized for the user based on the sensitivity of the vasodilation response of the user.

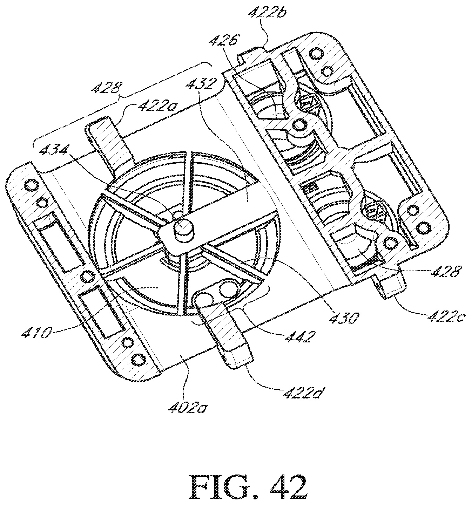

In some implementation, a device or system 400 for detecting an anomalous biologic event includes a support structure 428 coupled to the heat source 410 and configured to couple the heat source 410 to the second surface 402. For example, as shown in FIG. 42, the support structure 428 includes arm 432 that extends towards or to a center of the heat source 410 to support the heat source 410 and one or more spokes 430 that extend from the arm 432 to a perimeter of the heat source 410. The spokes 430 may be substantially equally spaced from adjacent spokes 430. The spokes 430 may also be circumferentially arranged about pin or joint 434. Spokes 430 of support structure 428 further define air flow apertures 442 to allow air to interact with the heat source 410 to cool the heat source 410. Spokes 430 further define air flow apertures 442 to at least partially expose the heat source to a cavity defined by the first surface 404 and second surface 402 as described elsewhere herein. Alternatively, or additionally, heat source 410 may be cooled by one or more vents, a blower for passing airflow over the heat source 410, coolant, or another mechanism known to one of skill in the art.

In some implementation, support structure 428 exerts pressure on the heat source 410 to increase contact or coupling between the heat source 410 and the skin surface. In one implementation, the tensionable band includes a strain gauge that determines the tensile stress the band is subjected to. The strain gauge output or signal could then be visualized or displayed to a user so the user knows if the band is tensioned to an appropriate level for the heat source and/or sensor(s). Alternatively, a spring constant (k) of the material may be used to calculate the force (F=kx), so depending on how much the material is stretched (put in tension), the band could indicate that force based on the displacement. As such, the support structure 428 may comprise a flexible material, for example a flexible plastic. In other implementation, the support structure 428 comprises a rigid material.

Further, as shown in FIGS. 40-41, a device or system 400 for detection of an anomalous biologic event further includes a skin temperature sensor 414 and a blood volume sensor 412. The blood volume sensor 412 can be integrated into a form factor such as the device or system 400 that improves continuous anomalous cardiac event monitoring. The blood volume sensor 412 can measure parameters that can provide vasodilation response. Furthermore, the skin temperature sensor 414 can also be integrated into the device or system 400. The skin temperature sensor 414 is positioned on the second surface 402 and configured to measure a temperature of the skin surface in contact with the heat source 410. The blood volume sensor 412 is positioned on the second surface 402 and configured to measure a blood volume of the skin surface. The blood volume sensor may be a photoplethysmography sensor or an impedance plethysmographic sensor. The blood volume sensor may employ light at 530 nm (green), 645 nm (red), 470 nm (blue) wavelength, or a combination thereof. Different wavelengths may be more appropriate for different applications, for example green (530 nm) light may be more accurate for heart rate measurements (e.g., heart rate variability, heart rate, etc.). In addition to, or alternatively, the blood volume sensor may be further configured to measure one or more of: heart rate, heart rate variability, or oxygen saturation.

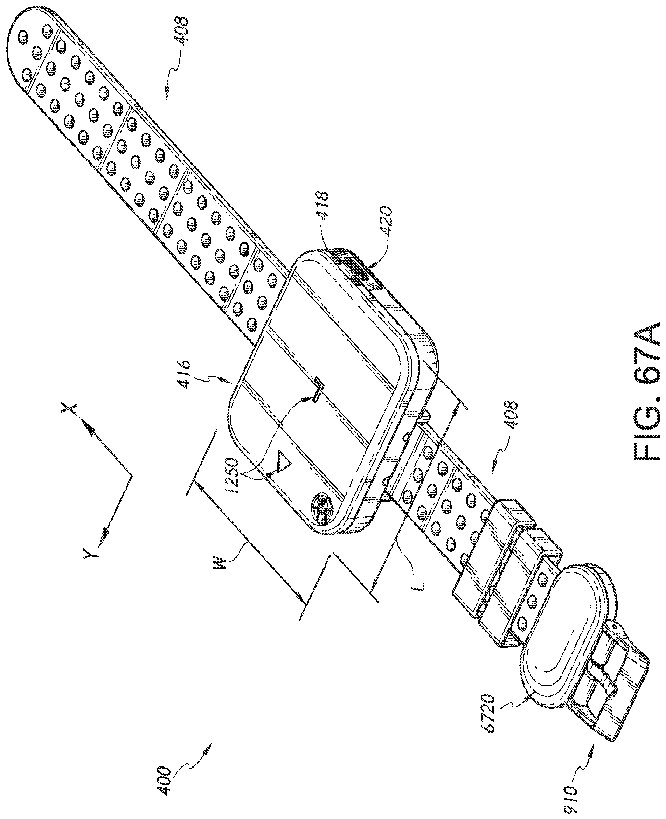

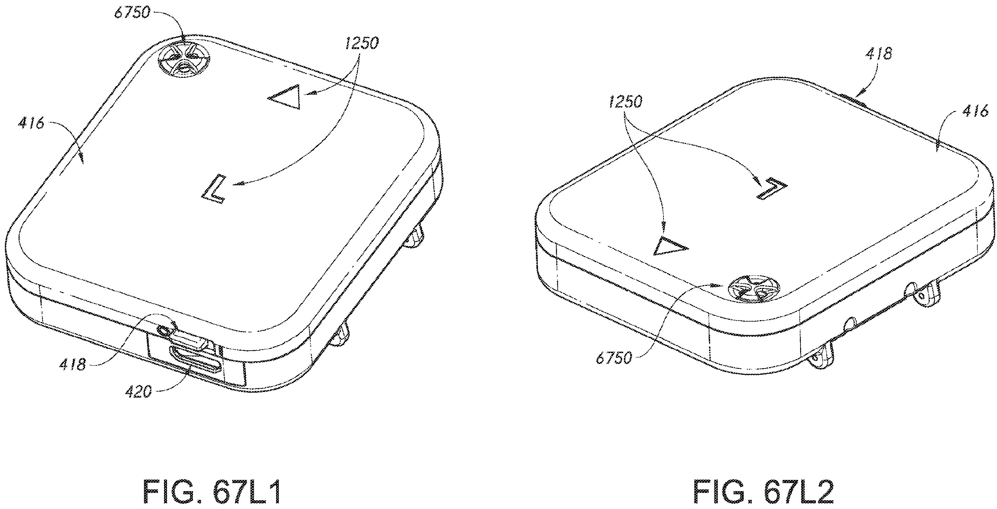

A system or device 400 for detection of an anomalous biologic event may include an environmental temperature sensor configured to measure a temperature of the environment around the wearable system 400. For example, an environmental temperature sensor, such as ambient temperature sensor 6750 shown in FIGS. 67A, 67L1, and 67P, may be positioned on the first surface 404 of the body 416 of the wearable system, opposite the second surface 402 that includes the heat source 410. Alternatively, the system or device 400 may be communicatively coupled to an environmental temperature sensor on or in a remote computing device. For example, the remote computing device may include a laptop, a cellular device, a workstation, a server, a desktop computer, a personal digital assistant, a second wearable system or device, a netbook, or the like.

The skin temperature sensor and/or environmental temperature sensor may include a thermocouple, a resistance temperature detector, a thermistor, or an infrared temperature sensor. The type of temperature sensor selected may depend on error rate, coupling to skin surface efficiency, among other features.

In some implementations, the heat source 410 is positioned concentrically about any one or more sensors. For example, a blood volume sensor 412 (shown in FIGS. 40-41), a skin temperature sensor 414 (shown in FIGS. 40-41), or additional sensors, for example an EDA sensor, a potentiometric ion-selective electrode sensor, a colorimetric sensor, or an enzymatic amperometric sensor may be positioned such that the stimulation source, e.g., heat source 410, is positioned about, encircles, encapsulates, or is concentrically disposed about the sensor. Although, a location or position of the blood volume sensor 412 and the skin temperature sensor 414 that enables coupling to a skin surface is envisioned. The blood volume sensor may be configured to measure one or both of a heart rate and a blood volume of the user.

A hardware processor (within the wearable system or communicatively coupled to the wearable system) communicatively coupled to the skin temperature sensor 414 and the environmental temperature sensor may be configured to perform a method comprising: receiving a first temperature signal using the skin temperature sensor and a second temperature signal using the environmental temperature sensor; and calculating a temperature differential between the skin temperature and the environment temperature. For example, if the temperature differential is below a set threshold, a difference between the target temperature and the maximum temperature value may be increased. In contrast, if the temperature differential is above a set threshold, a difference between the target temperature and the maximum temperature value may be reduced. The environmental temperature sensor may also be used in analysis of determining erroneous results, such as false positive indications of abnormalities. By comparing signals before and after stimulus and/or by comparing left versus right limb, externalities such ambient temperature response may be reduced in the analysis of abnormalities.

Further, the hardware processor may be coupled to the heat source 410 and the blood volume sensor 412. In some instances, the system 400 describe above can enable non-invasive monitoring of vasodilation and/or vasoconstriction. Human body regulates stable equilibrium through the process of homeostasis. For example, if a stimulus is applied to a body of patient, one or more homeostatic processes will attempt to counteract the effect of stimulus. For example, with respect to an induced thermal stimulus that increases or decreases temperature at a tissue site, the body will attempt to reverse the temperature change through blood flow (vasodilation or vasocontraction). Accordingly, the system 400 can induce and measure the vasodilatory response. As discussed above, stroke and other abnormalities can impair the vasodilatory response. Therefore, in some instances, it may be advantageous to monitor the change in the vasodilatory response to determine abnormalities, such as stroke. A blood volume sensor, such as optical sensors, can enable monitoring of the blood flow and correspondingly the vasodilatory response. In some instances, one or more temperature sensors (through a thermistor or optical radiation-based detectors) can also enable determination of the vasodilatory response by monitoring how quickly the temperature of the skin returns to equilibrium following the stimulus. In some examples, the vasodilatory response is correlated with a rate of change or slope in the measured parameter, such as blood volume parameters, temperature, and others discussed herein. In additional examples, the vasodilatory response can be correlated with a steepness of the rate of change. This can be calculated using a second derivative.

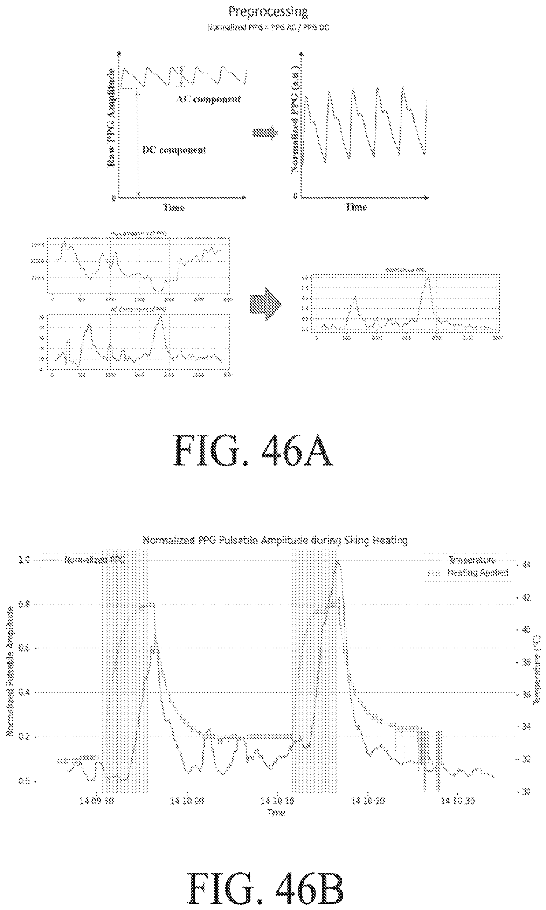

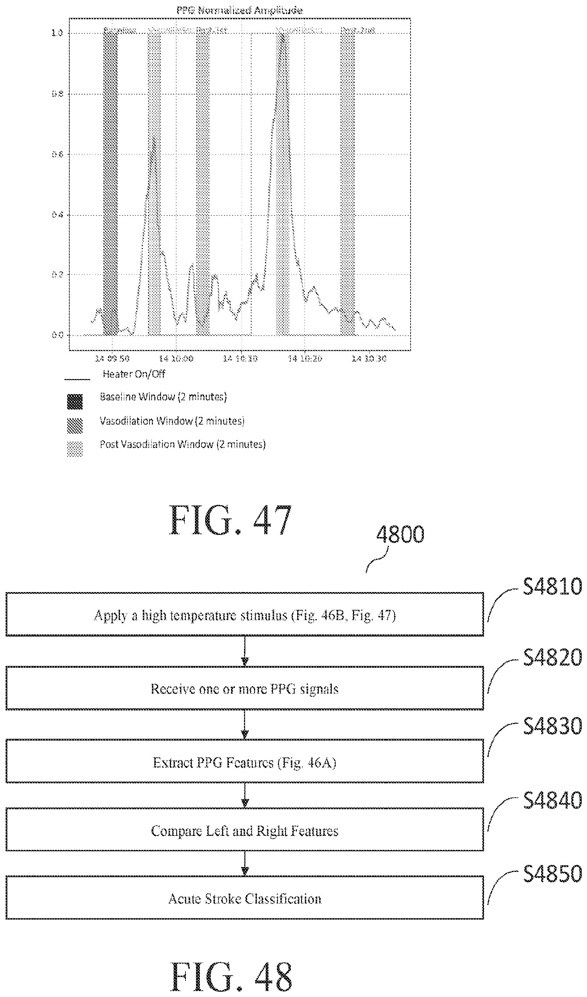

In some instances, it can be advantageous to use a combination of a heat source 410 and the blood volume sensor 412 to improve cardiac monitoring. The heat source 410 and the blood volume sensors 412 can be integrated into a form factor that a user can wear for continuous monitoring. The measurements can be repeated non-invasively without significant discomfort to the patients. Furthermore, as shown in Figs. 46A-B and 47, the response time between the application of heat and the change in blood volume is relatively small. This can enable a relatively fast determination of the anomalous biologic event. Therefore, it can be advantageous to integrate a heat source and a blood volume sensor in any wearable system disclosed herein to improve continuous cardiac monitoring. In some instances, a Peltier cooler can be used as a thermal source instead of or in addition to the heat source 410.

Furthermore, in some instances, the stimulus can be an electrical stimulus in addition to or instead of the thermal stimulus. For example, the system 400 may include a plurality of electrodes for applying current and/or voltage to a circuit associated with the plurality of electrodes in order to measure particular signals (e.g., electrical activity) from a tissue site. Electrical activity can include bioimpedance for detecting high or low muscle tone, which can occur with hemiplegia. The system 400 can include at least two electrodes. In some instances, the system 400 can include three electrodes. In some instances, the system 400 can include at least four electrodes. Furthermore, the system 400 can also include six or more electrodes. The electrodes can be integrated on the system 400 such that they are in contact with the skin tissue of the user.

As discussed above, an optical sensor, such as the blood volume sensor 412, can interrogate a target tissue to determine parameters that correlate with the vasodilatory response. Other sensors can also be used to extract parameters for determination of the vasodilatory response. For example, the system 400 can use minimally invasive and/or invasive sensors to determine hemodynamic parameters, such as cardiac output, to provide an indication of the vasodilation response. The system 400 can also include on or more electrical based sensors, such as bioimpedance sensors, EDA sensors, ECG sensors, EEG sensors, EMG sensors, and the like. Electrical sensors may enable measurement of hydration, skin conductance, and other electrical parameters that relate to hemodynamic function monitoring. Furthermore, the system 400 can include one or more ultrasound sensors to obtain hemodynamic parameters. Temperature sensors can also enable determination of the vasodilation response. Accordingly, the system 400 can include a combination of some or all of the sensors discussed above to extract one or more parameters that correlate with hemodynamic function or maintenance of homeostasis.

Patients are often monitored in neuro ICU after a stroke. This can be expensive as a human (e.g., a nurse) typically conducts periodic checks on the patient. Such human-based checks may also be subjective and/or inaccurately timed, thus missing conditions, symptoms, and/or events. Accordingly, the system 400 can enable improved monitoring without requiring the patient to be in the neuro ICU and/or without requiring a caregiver to conduct periodic checks. While the system 400 is described as a wearable system, in some examples, some or all of the components of the system 400 may be positioned in proximity to the user but not directly attached or worn by the user. For example, when a user needs to be monitored in a hospital environment, some or all of the components of the system 400 can be positioned in proximity to the user's hospital bed. For example, the thermal stimulus source can include a laser.

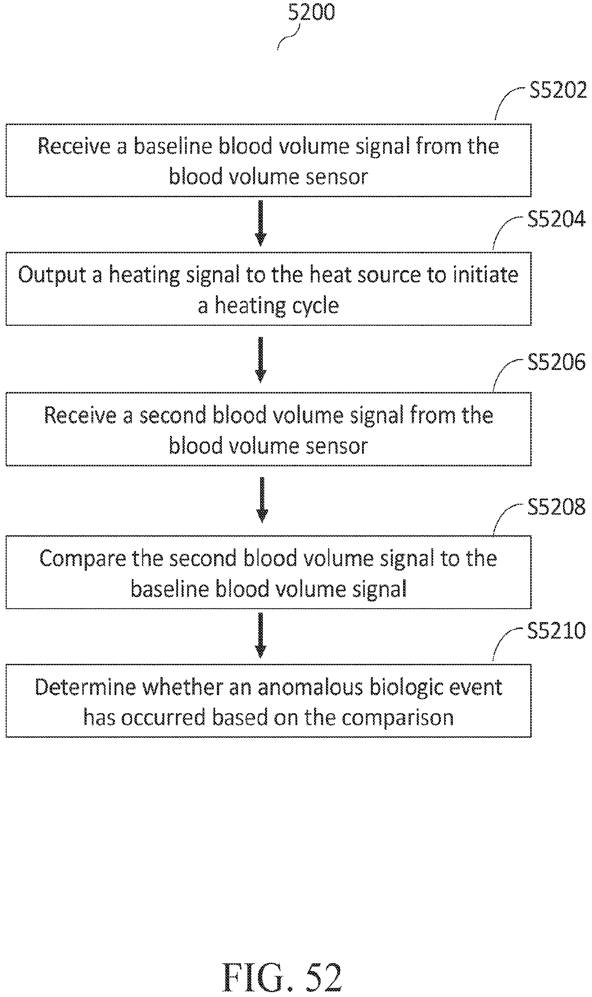

As such, the hardware processor may be configured to perform the method, as shown in FIG. 52, which includes: receiving a baseline blood volume signal from the blood volume sensor S5202, outputting a heating signal to the heat source to initiate a heating cycle S5204, receiving a second blood volume signal from the blood volume sensor S5206, comparing the second blood volume signal to the baseline blood volume signal S5208, and determining whether an anomalous biologic event has occurred based on the comparison S5210. The steps of the method may be repeated at least once, one or more times, a plurality of times, on a loop, according to physician, caregiver, or user preferences, or otherwise.

In some implementations, the second blood volume signal is a set of blood volume signals, such that the blood volume of the skin surface is measured repeatedly before, during, and/or after a heating cycle of the heat source. The blood volume of the skin surface may be measured at a pre-set interval, for example every about 10 ms to about 1 sec, about 1 sec to about 5 sec, about 5 sec to about 10 sec, etc. Alternatively, the blood volume of the skin surface is measured randomly or only upon detection of a change in temperature of the skin surface or upon detection of a change in vasodilation by the blood volume sensor. A measurement frequency may be individualized for a user, for example if a vasodilation response of a user in response to heat is very sensitive, a reduced frequency of blood volume measurements may be needed. In contrast, if a vasodilation response of a user in response to heat is less sensitive, an increased frequency of blood volume measurements may be needed.

In some implementations, the second blood volume signal is a plurality of blood volume signals, such that the blood volume of the skin surface is measured continuously before, during, and/or after a heating cycle of the heat source.

In some implementations, block S5206 includes receiving the second blood volume signal after the target temperature is reached, after a predetermined length of time has expired, after a dwell time (i.e., cycling heat source on and off during a heat cycle or cycling heat source between target temperature and lower temperature during a heat cycle) has expired, or after one or more heating cycles have concluded. A frequency of sampling and/or sampling relative to a heat cycle (before, during, or after the heat cycle) may be based on a user's biology, such that the sampling is individualized.

In some implementations, block S5208 includes calculating a baseline ratio of alternating current (AC) to direct current (DC) for the baseline blood volume signal and a second ratio of AC to DC for the second blood volume signal and comparing the baseline ratio to the second ratio, as shown in FIG. 46A. The methodology and rationale for the AC to DC ratio is described in Tusman et al. "Advanced uses of pulse oximetry for monitoring mechanically ventilated patients." AnesthAnalg 2017; 124: 62-71, which is herein incorporated by reference in its entirety. The top left panel of FIG. 46A shows raw PPG amplitude data and the respective DC and AC components of the signal. Taking the ratio of AC to DC of the raw signal yields the top right panel. During a two-heating cycle experiment, PPG data in the lower left panel was collected. The AC and DC components of the signal are represented in separate, stacked graphs. When the AC to DC ratio is calculated for this two-heating cycle experiment, a normalized PPG signal is achieved, which is shown in the lower right panel. The same PPG data is shown in FIG. 46B overlaid with heat cycle data. As shown, the temperature of the skin surface reaches the target temperature (i.e., about 42C) in each heat cycle, shown by the shaded portions of the graph. The perfusion index or normalized PPG signal similarly spikes during each heat cycle in response to the application of heat. FIG. 47 shows the same data as FIGS. 46A-46B with additional definition of baseline, vasodilation or stimulation, and post vasodilation or post-stimulation windows. The heat cycle was off for 5 min, on for 5 min, off for 15 min, on for 5 min, and off for 10 min. The time windows selected for comparison were: a baseline time window (e.g., minimum 2 minutes before "heat source first on"), a vasodilation or stimulation time window (e.g., maximum 2 minutes of "heat source on"), a first post stimulation or vasodilation time window (e.g., minimum 2 minutes after "heat source first on"), and a second post vasodilation (e.g., minimum 2 minutes after "heat source second on"). As shown in FIGS. 46A-47, application of heat elicits a vasodilation response that is reproducible over multiple cycles.

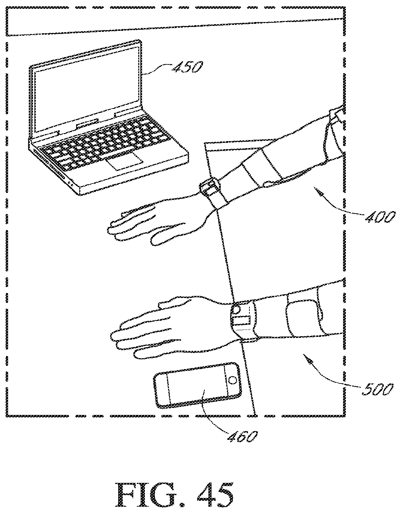

As discussed above, tracking a vasodilation response can be used in monitoring abnormalities, such as stroke. However, the vasodilation response in a user can be affected by several sources that are unrelated to the stroke or the abnormality that is being monitored. Accordingly, using the system 400 in only one tissue site may result in false positives. It was observed by the inventors that by monitoring multiple tissue sites, the monitoring results may more closely track the abnormalities and reduce erroneous results. Figure 45 illustrates a first system 400 and a second system 500 placed approximately symmetrically on the right and left limbs. Accordingly, if a stimulus is applied approximately in synchronization between the first system 400 and the second system 500, the degree of symmetry or asymmetry in the measurements responsive to the approximately simultaneous stimulation can be used in the determination of stroke and reduction of erroneous results. While the disclosure herein provides stroke as an example of abnormalities, the system 400 and the methods described herein can also be used to monitor other abnormalities. For instance, other abnormalities or physiological deviation can include menopause, diabetes, and peripheral blood circulation disorders that can affect peripheral blood circulation.

In some implementations, as shown in FIG. 48, a method 4800 of detecting an anomalous biologic event includes: applying a high temperature stimulus (e.g., shown in FIGS. 46B-47) S4810; receiving one or more signals indicative of a blood volume, blood flow, or blood perfusion in a tissue of the user in response to the high temperature stimulus S4820; extracting one or more features of the one or more signals S4830; comparing the one or more features for a right side and a left side of the user (e.g., right and left limbs, as shown in FIG. 45) S4840; and calculating an acute stroke classification score S4850. Furthermore, the method 4800 can optionally compare baseline measurements prior to the application of the stimulus and after the application of stimulus, as discussed in more detail with respect to Figure 52 for both left and right limbs. During multiple tissue site monitoring, such as the left and right limb monitoring as shown in Figure 45, the system 500 may include all the same components as the system 400 described above. In other cases, the system 500 may include less components than system 400. For example, both systems may not require a display. Additionally, one of the systems may include computational capabilities while the other one collects the data and transmits to the paired system for computation. Therefore, one of the systems 400 and 500 may not include a hardware processor. Accordingly, the system 400 and 500 may operate in a master-slave configuration. The systems 400 and 500 may be paired wirelessly via Bluetooth^{®} or other wireless protocol. In some instances, the systems 400 and 500 may be paired with an external computing system, such a patient monitor, a hub, or a smartphone.

In some implementations of block S4830, the one or more features include, but are not limited to, an amplitude or a systolic or diastolic wave, a waveform shape, a waveform complexity, a perfusion index (i.e., a relationship between the pulsatile (AC) and the non-pulsatile (DC) components of PPG signal), DC offset, a stiffness index (i.e., time between peaks of forward and backward waves along the vascular tree; h / ΔT, where h is a patient's height), a reflection index (i.e., a ratio between the heights of the backward and the forward waves; B / A x 100), a notch position (i.e., position of the dichrotic notch; e.g., with vasoconstriction, the position moves toward the left into the systolic wave), a peak to peak phase shift, slope onset of temperature signal and/or blood volume signal, slope decay of temperature signal and/or blood volume signal, midpoint of rising slop of temperature signal and/or blood volume signal, a vasodilation response as an indicator of a collateral state of the brain and/or heart, etc.

In any of the implementations described herein, a wearable system or device for detecting anomalous biologic events may include one or more electrodermal activity sensors positioned on the second surface 402 and/or a tensionable band 408 of the system. For example, as shown in FIG. 41, electrodermal sensors 424, 426 are positioned on the second surface 402 of the wearable system 400. Electrodermal sensors 424, 426 may be spaced apart from one another by distance 444, which equals about 5 mm to about 10 mm, about 10 mm to about 20 mm, about 20 mm to about 30 mm, about 30 mm to about 40 mm, about 40 mm to about 50 mm, about 50 mm to about 60 mm, about 60 mm to about 70 mm, about 70 mm to about 80 mm, about 80 mm to about 90 mm, about 90 mm to about 100 mm, measured from a center point of each sensor. Further, electrodermal sensors 424, 426 may be spaced apart from the heat source 410 by distance 446, which equals about 10 mm to about 20 mm, about 20 mm to about 30 mm, about 30 mm to about 40 mm, about 40 mm to about 50 mm, about 50 mm to about 60 mm, about 60 mm to about 70 mm, about 70 mm to about 80 mm, about 80 mm to about 90 mm, about 90 mm to about 100 mm, measured from a center point of the sensor 424, 426 and a center point of the heat source 410.

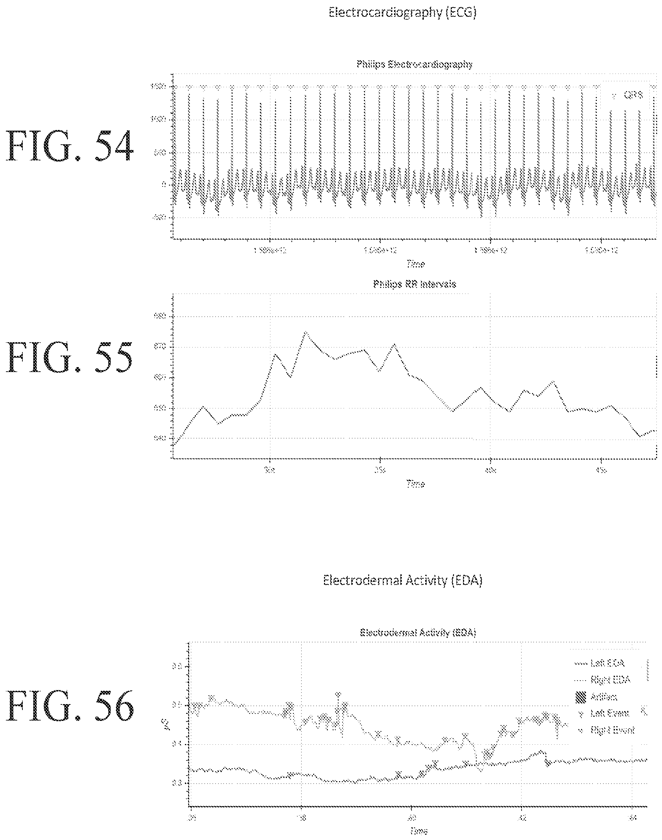

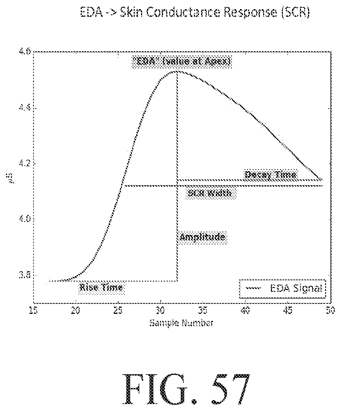

As shown in FIG. 56 as one example, electrodermal activity (EDA) of a skin surface of a user may be measured overtime. Left side and right side electrodermal activity may be measured over time and compared. FIG. 56 shows left and right side electrodermal activity including events (shown as triangles) potentially indicative of an anomalous biologic event. A signal collected by an electrodermal activity sensor may be processed to extract one or more features. For example, as shown in FIG. 57, one or more features may include a rise time (i.e., start of the SCR to the apex), an amplitude (i.e., EDA at apex minus an EDA at start of the SCR), a skin conductance response (SCR) width (i.e., between the 50% of the amplitude on the incline side and 50% of the amplitude on the decline side), a decay time (i.e., time from apex to 50% of the amplitude), an area under the curve (i.e., SCR width multiplied by amplitude), Maximum derivative of SCR, and/or an apex value.

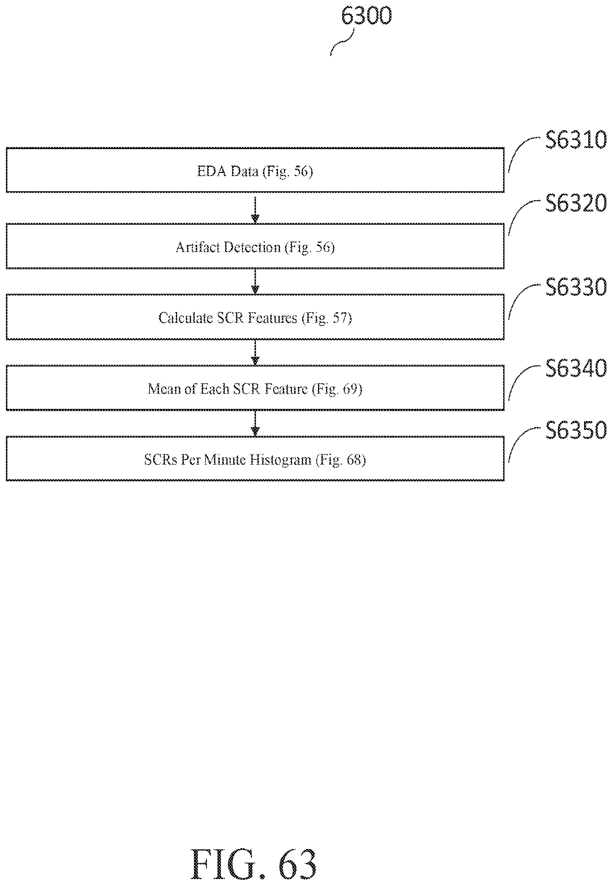

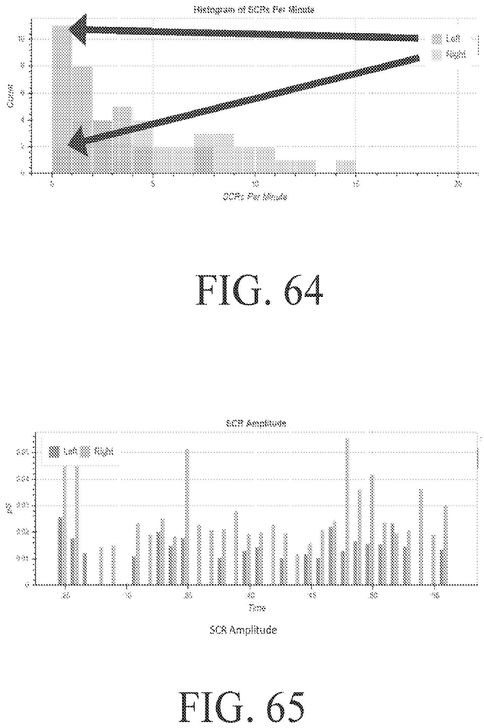

FIG. 63 shows a method 6300 of analyzing EDA data, and FIGS. 64-65 show representative EDA data. A method 6300 for analyzing EDA data includes: receiving signals from one or more EDA sensors (e.g., as shown in FIG. 56) S6310; detecting and/or removing one or more artifacts (e.g., as shown in FIG. 56) S6320; calculating or extracting one or more skin conductance response (SCR) features (e.g., as shown in FIG. 57) S6330; calculating a mean or average of one or more features S6340; and calculating an SCR for a period of time S6350. For example, SCR amplitude is shown graphically in FIG. 65 for one-minute intervals. As shown, for this individual, SCR amplitude varies over time and asymmetrically (i.e., comparing right vs. left response). Further, if the SCRs per minute are compared for left and right responses, as shown in FIG. 64, the SCR per minute varies over time and asymmetrically.

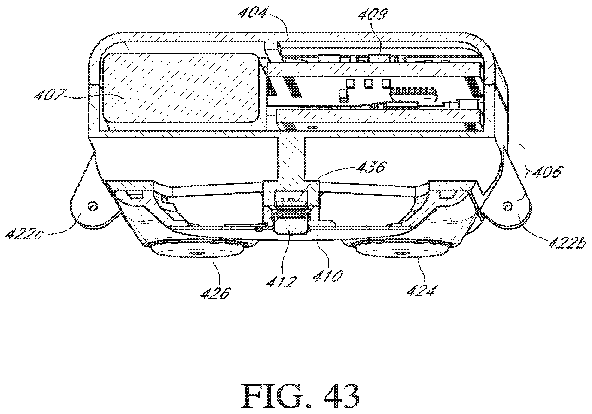

In any of the implementations described herein, a wearable system or device for detecting anomalous biologic events may include one or more motion sensors 436 configured to measure a motion of a body portion to which the wearable system 400 is coupled, as shown in FIG. 43. For example, the one or more motion sensors 436 may measure an acceleration in six or nine degrees of freedom. As described elsewhere herein, a wearable system or device for detecting stroke may, in combination with measuring a vasodilation response in response to application of heat, may measure asymmetrical movement or tremors of the right and left limbs. One or more motion sensors 436 may be positioned anywhere on the wearable device 400. For example, in one implementation, a motion sensor 436 is positioned in or on the first surface 404. In another implementation, a motion sensor 436 is positioned in or on the second surface 402. In another implementation, a motion sensor 436 is positioned in between the first surface 404 and second surface 402, for example, in a cavity 406. In another implementation, a motion sensor 436 is positioned on a sidewall 405 of the body 416 of the wearable device 400. In another implementation, a motion sensor 436 is positioned adjacent to a vasodilation sensor or blood volume sensor 412 or skin temperature sensor 414 of the system, for example concentrically surrounded by the heat source 410, as shown in FIG. 43.

The heat source 410 of the wearable device or system 400 may be cooled in between heating cycles to ensure a return to baseline or substantially baseline of the vasodilation response of the skin surface in between heating cycles. As such, the heat source 410 may be cooled by an airflow system (e.g., fan), a vacuum or vibrating mechanism configured to displace or pull or move environmental air across the heat source (e.g., solenoid and diaphragm, oscillating piezo element), etc. In one implementation, as shown in FIGS. 40-43, a wearable system or device for detecting an anomalous biologic event includes first surface 404 and second surface 402 that together define a cavity 406 therebetween to provide airflow between the first surface 404 and second surface 402. The cavity 406 defined by the first surface 404 and second surface 402 physically separates the heat source 410 from the hardware processor 409 positioned on or within the first surface 404. In some implementations, a battery 407 is physically separated from internal electronics, such as processor 409. In some implementations, the battery 407 may be separated from the heat source 410. In some implementations, the battery 407 may be separated from both the heat source 410 and the processor 409, and may optionally be accessible via a battery cover, for example to be recharged and/or replaced.

The hardware processor 409 can include microcontrollers, digital signal processors, application specific integrated circuit (ASIC), a field programmable gate array (FPGA) or other programmable logic device, discrete gate or transistor logic, discrete hardware components, or any combination thereof designed to perform the functions described herein. The cavity 406 functions to expose the heat source 410 to ambient or environmental or surrounding air to cool the heat source 410 to a temperature that approaches, substantially equals, or equals a temperature of the air in the environment or an ambient temperature. The cavity 406 may be an empty space, an interstitial space, a space that houses one or more components, etc. In some implementations, cavity 406 formed by the first surface 404 and second surface 402 is open to ambient air or environmental air such that the sidewalls 405 that couple together the first surface 404 and the second surface 402 are opposite one another so that the cavity 406 is open to the environmental air on opposing sides, as shown in FIGS. 40-41. Alternatively, the sidewalls 405 are connected to one another and adjacent to one another so that the cavity is open to the environmental air on adjacent or connected sides. In lieu of, or in addition to, a cavity, the device may include a thin insulative material to facilitate cooling of the heat source.

For example, in some implementations, the cavity 406 defined by the first surface 404 and second surface 402 has sufficient volume to facilitate cooling of the heat source 410 in between heating cycles. Alternatively, or additionally, the cavity 406 may further include an airflow system, vacuum or vibrating mechanism, or other airflow mechanism to promote airflow through the cavity 406 to reduce a temperature or cool the heat source 410.

In some implementations of a wearable system or device, the device includes a port 420 for electrically coupling the device to a power source, for example to charge a battery 407 in the device. Additionally, or alternatively, port 420 electrically couples the wearable device to an external or remote computing device (e.g., laptop, desktop, server, workstation, etc.) to download data from the device or upload system parameters or install updates to the wearable device. The wearable device may further include one or more user input elements 418 to power on and off the device; to input user specific reactions, features, or characteristics, to customize an interface or functionality of the user device, etc.

In some implementations, as shown in FIG. 45, a wearable system for detecting an anomalous biologic event includes a first system or device 400 positioned on a left limb of a user and a second system or device 500 positioned on a right limb of the user. The first and second devices 400, 500 may measure similar parameters or features so that the parameters or features are comparable over time and/or on an event-by-event basis to detect asymmetrical biologic responses and/or deviation from a baseline (e.g., individualized or population based). For example, a hardware processor as part of the system or communicatively coupled to the devices (e.g., laptop 450 or mobile computing device 460) may be configured to compare right side blood volume signals (e.g., in response to an application of heat) to left side blood volume signals (e.g., in response to application of heat) to determine whether the anomalous biologic event has occurred. The right and left side blood volume signals may be compared to a baseline right and left side blood volume signals, respectively, to account for any asymmetrical baseline differences that may exist between the left and right sides. Further, a method performed by the hardware processor may include synchronizing the signals received from the left limb and the right limb in time; and comparing the synchronized signals from the left limb and the right limb to determine whether the anomalous biologic event occurred.

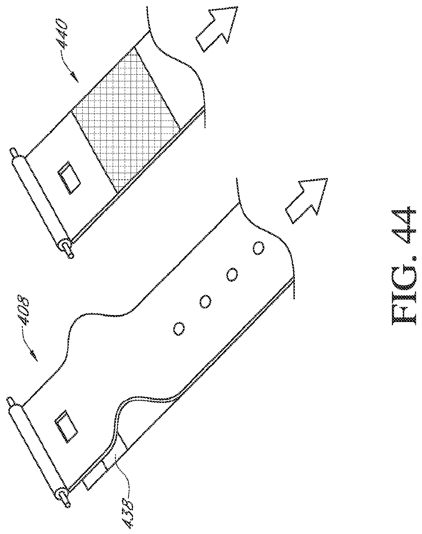

Turning now to FIG. 44, which shows a band 408, configured to couple a wearable system for detecting an anomalous event to a limb or body portion of a user. For example, the band 408 may be a tensionable band for coupling a detection system or device 400 to a limb or body portion of a user. The tensionable band is formed of or comprises a stretchable material (e.g., silicone, rubber, Lycra, Spandex, Elastane, neoprene, leather, fabric, etc.). Alternatively, a portion or section 440 of the band 408 may be stretchable, such that the stretchable portion or section 440 can be extended or retracted by applying varying amounts of tension to the band. Accordingly, the band may be adjustable so that the band 408 fits a variety of body portion shapes and sizes. For example, the band 408 may have an adjustable circumference. The band 408 may further include a visual indicator 438 to indicate when one or more of: the heating source 410, the skin temperature sensor 414, the blood volume sensor 412, or a combination thereof is sufficiently coupled to the skin surface to enable accurate sensor readings.

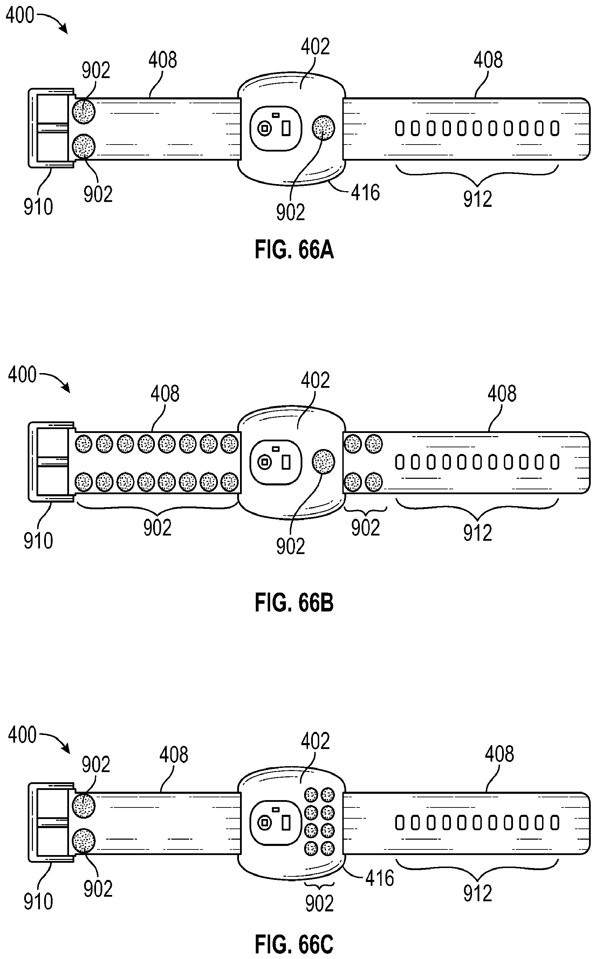

As shown in FIGS. 66A-66D device 400 for detecting an anomalous biological event can feature a plurality of electrodes 902a, 902b, and 902c in various locations on both the body 416 and the band 408 (e.g., a tensionable band). In some implementations, the electrodes can be the electrodermal sensors 424 and 426 of FIG. 41; in other implementations, they can be any electrode or electronic sensor suitable for the excitation and/or collection of various bioelectrical data including, but not limited to, electromyography (EMG), bioimpedance (BioZ/BIA), bioimpedance spectroscopy (BIS), and electrodermal analysis (EDA). In some implementations, the electrodes 902a-c can be adapted to receive a multiplex of bioelectrical data simultaneously (e.g., any combination of two or more of the above types) that can be parsed and analyzed by other portions of the system, as described herein. For example, in some implementations, each of the electrodes 902a-c can be adapted to simultaneously record EMG, BioZ, and EDA signals. In some implementations, simultaneous recording of EMG, BioZ, and EDA signals may be performed by switching between particular sensors associated with the plurality of electrodes 902a-902c. In some implementations, the electrodes 902a-c may be configured to record EMG, BioZ, and/or EDA signals in a sequential order, rather than simultaneous capture. Although three electrodes 902a, 902b, and 902c are depicted, any number of electrodes may be installed and configured for use with device 400.

In the implementations of FIGS. 66A-66D, as well as in other various implementations depicted throughout, the band 408 is depicted as a band featuring a buckle 910 on a first side and a series of receiving notches 912 on the second, opposing side. In these implementations, any electrode 902a-c positioned on the band 408 can be positioned such that the notches 912 are unobstructed and able to receive the buckle 910. In other implementations, alternative bands 408 can be employed that can allow for similar or alternative number and arrangements of electrodes 902a-c. Furthermore, in many implementations, electrodes 902a-c present in the band 408 are connected through the band 408 via traces (not shown - e.g., wires, circuitry or another connector) to other elements housed in the body 416 including, but not limited to, a power supply (for example, battery 407 shown in FIG. 43) and a processor (for example, processor 409 shown in FIG. 43). In some implementations, these traces from the electrodes 902a-c of the band 408 to elements of the body 416 can be formed by cold molding or insert molding.

In the implementation of FIG. 66A, one electrode 902a-c can be positioned on the second surface 402 of the body 416 of the device 400 while two electrodes 902a-c can be positioned on the band 408. When the device 400 is worn in the depiction of FIGS. 12-25, this positions one electrode 902a on a top side of a wrist and two electrodes 902b and 902c on a bottom side of a wrist. In implementations wherein the electrodes 902a-c are at least capable of receiving EMG signals, the one or more electrodes 902a-c on the top of the wrist can be a reference EMG electrode, and the one or more electrodes 902a-c on the bottom can be either monopolar electrodes (for the measurement of EMG from one muscle) or bipolar electrodes (for the measurement of EMG signals from two muscles). This arrangement of having at least one reference EMG electrode on top of the wrist and at least one monopolar or bipolar EMG electrode on the bottom of the wrist can be employed in any implementation described herein shown as having at least one electrode in each position.

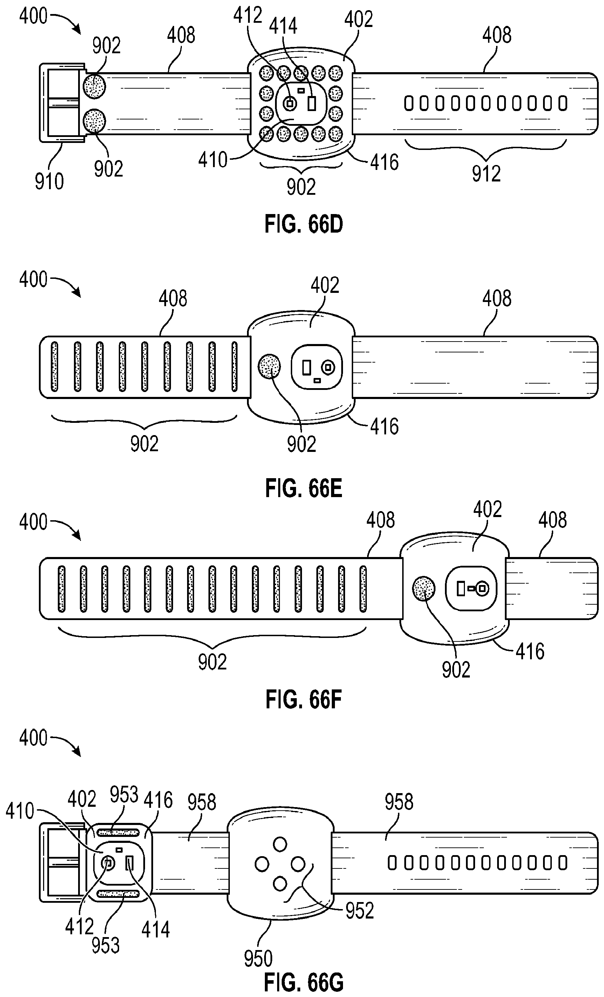

In the implementation of FIG. 66B, one electrode 902a-c can again be positioned on the second surface 402 of the body 416 while series of electrodes 902a-c can line the band 408 on either side of the body 416. While the implementation of FIG. 66B shows sixteen and four electrodes 902 (similar to 902a, 902b, and/or 902c) on a first and second side of the band 408, respectively, any number of electrodes can be present on each side in other implementations. When the device 400 is worn in the depiction of FIGS. 12-25, this positions one electrode 902 on a top side of a wrist and a series of electrodes 902 along the circumference of a wrist for as far as the band 408 can reach. In the implementation of FIG. 66C, an array of electrodes 902 can be clustered on the second surface 402 of the body 416 while two electrodes 902 can be positioned on the band 408. Although eight electrodes 902 are shown on the second surface 402 of the body 416 of the device 400, any number of electrodes 902 can be present in other implementations. When the device 400 is worn in the depiction of FIGS. 12-25, this positions the array of electrodes 902 on a top side of a wrist and two electrodes 902 on a bottom side of a wrist. In the implementation of FIG. 66D, an array of electrodes 902 can line the perimeter of the second surface 402 of the body 416 thereby surrounding the heat source 410, blood volume sensor 412, and skin temperature sensor 414 along with two electrodes 902 positioned on the band 408. Although fourteen electrodes 902 are depicted on the second surface 402 of the body 416 of the device 400, any number of electrodes 902 can be present in other implementations. When the device 400 is worn in the depiction of FIGS. 12-25, this positions the array of electrodes 902 on a top side of the wrist and the two electrodes 902 on a bottom side.

FIGS. 66E-66G depict the device 400 in alternative configurations. In the implementations of FIG. 66E and 66F, the band 408 lacks a buckle 910 (e.g., FIGS. 66A-D) and the corresponding receiving notches 912 that allow for alternative arrangements of electrodes 902. In some implementations, a Velcro^{®} or another hook-and-loop fastener (not shown) can be employed along at least a portion of the band 408 to avoid the use of a buckle 910.

In the implementation of FIG. 66E, the device 400 can feature one electrode 902 on the second surface 402 of the body 416 and a series of electrodes 902 along the length of a first side of the band 408. In some implementations, the electrodes 902 positioned on the band 408 can have an elongate or oval shape as depicted in FIG. 66E. In other implementations, a circular shape, such as those of FIG. 66B can be employed instead. Although nine electrodes 902 are depicted on the band 408 in FIG. 66E, any number of electrodes 902 can be employed in alternative implementations.

In implementations, employing a band 408 that needs no buckle, the band 408 can take on different proportions than those commonly used for traditional fabric or leather watch bands. As shown in FIG. 66F, certain implementations of the device 400 can make use of a lopsided arrangement of the band 408 in order to maximize a number of electrodes 902 that can be installed in the band 408. In this example, one electrode 902 is positioned on the second surface 402 of the body 416 and fifteen electrodes 902 are positioned on a first side of the band 408. In some implementations, the electrodes 902 positioned on the band 408 can have an elongate or oval shape as depicted in FIG. 66F. In other implementations, a circular shape, such as those of FIG. 66B can be employed instead. Although nine electrodes 902 are depicted on the band 408 in FIG. 66F, any number of electrodes 902 can be employed in alternative implementations. In some implementations, the side of the band 408 that lacks electrodes can be manufactured to be oversized (e.g., have a length greater than what is expected to be needed) and can be cut down to custom fit for a particular patient.

In some implementations, such as that of FIG. 66G, the device 400 can be adapted to be augmented by an independent or third-party device 950 (such as a smart watch) that itself features compatible electrodes 953. In these implementations, the device 400 can comprise a heat source 410, blood volume sensor(s) 412, 952, skin temperature sensor 414, and at least one electrode 902 on the second surface 402 of the body 416, and the device 400 can attach to a band 958 of the smart watch 950 (e.g., a watch band). In conjunction with the compatible electrodes 953 of the smart watch 950 coordinated with software (e.g., a mobile app), the device 400 in this implementation can function similarly as to other implementations described herein.

Device 400 may use electrodes 902a-c to perform other physiological monitoring beyond the measurements obtained directly by blood volume sensor 412 and skin temperature sensor 414. For example, one or more of the electrodes 902a-c may be used to obtain biomarkers to measure edema and/or hydration. For example, the electrodes 902a-c may be configured to measure local bioimpedance (BioZ) by sending frequencies anywhere from about 10 Hertz to about 10 Megahertz across a pole of one or more of the electrodes 902a-c. The frequencies may be sent intracellularly or transcellularly. In some implementations, the electrodes 902a-c may also be configured to obtain BioZ across a spectrum of frequencies. The device 400 may then process the measurements to perform a BioZ analysis across the spectrum of frequencies.

In some implementations, device 400 may be configured to analyze EDA to determine how particular symptoms relate to events such as stroke events. That is, symptoms such as cold, clammy skin (e.g., under an electrode) may occur quickly or be present upon initiating measurements for EDA. If the system 400 detects two or more of such symptoms, the device 400 may generate an alarm or an event to send to the patient via audio or visual information. In some implementations, such alarms or event data may be sent to a provider. In some implementations, the detection may trigger generation of an assessment that may include a determined likelihood of occurrence of a particular anomalous biologic event such as a stroke. In some implementations, the device 400 may be configured to analyze both EDA and BioZ concurrently.

In some implementations, the assessment may be the result of an analysis performed on any obtained measurements. For example, the device 400 may be configured to perform analysis on obtained measurements/recordings. For example, device 400 may be configured to assess a relevance of magnitude differences in BioZ and/or frequency differences, temperature differences, hydration differences, edema differences, etc. between known patient data and current measurement data.

In some implementations, the device 400 can be configured to obtain particular measurements on a scheduled basis. For example, BioZ may be measured infrequently when assessing for edema because edema may occur and persist for a long period of time (e.g., hour, days, etc.). By contrast, EDA may be measured frequently because an emotional trigger/response may be the event to capture. Emotional triggers/events may not persist and thus, measuring frequently may ensure that an emotional trigger/event is captured. The sensors described herein may be switched between to measure EDA frequently and then measuring BioZ infrequently. The device 400 may switch to trigger electrodes 902a-c, for example, to obtain particular measurements using, for example, skin temperature sensor 414 (or another sensor described herein) when assessing EDA. However, when assessing BioZ, the device 400 may switch to using electrodes 902a-c to use, for example, blood volume sensor 412 (or another sensor described herein).

In some implementations, one or more of the electrodes 902a-c may be configured to measure EMG. For example, device 400 may assess a trigger signal and deduce a lack of electrical movement by the user wearing the device 400. Such an assessment may determine that the wrist, finger, or hand, for example, should move responsive to the triggered signal, but instead did not move. If a signal indicating movement is not received, the device 400 may determine the likelihood that the user has experienced an anomalous biologic event (e.g., a paretic side).

In some implementations, one or more of the electrodes 902a-c may be configured to measure muscle tone and/or muscle atrophy. For example, by measuring EMG with one or more electrodes 902a-c, the device 400 may determine whether or not muscle atrophy has occurred.

In some implementations, when assessing EMG measurements, the device 400. may be configured to detect a state of an EMG signal at a particular tissue site. For example, the device 400 may be configured to determine that the state of an EMG signal indicates an absence or a presence of the EMG signal at a particular tissue site. In combination with the EMG signal state, the device 400 may be configured to compare captured accelerometer measurements associated with the timing of the capture of the state of the EMG signal in the tissue site. Accelerometer measurements can be likened to body or muscle movements or lack thereof. If an EMG signal is present during a movement, the device 400 may determine that an EMG signal amplitude indicates particular muscle movement or lack thereof. Although an accelerometer is described, one of skill in the art will appreciate that similar data can be acquired using a gyroscope, inertial measurement unit, tilt sensor, etc.

In some implementations, the device 400 may be configured to analyze a power spectrum of a captured EMG signal. For example, changes in the amplitude of specific frequencies of the EMG signal can lead to an assessment of muscle fiber recruitment magnitude and timing. Such information may be used to detect a paretic versus a contralateral limb.

In some implementations, by measuring EMG with one or more electrodes 902a-c, the water content (e.g., an indicator of muscle mass) of the tissue may be determined. If the water content of the tissue is determined, the device 400 may also determine dehydration and edema to determine hemiplegic issues or salt/electrolyte imbalance issues.

In some implementations, one or more of the electrodes 902a-c may be configured to measure EMG. For example, device 400 may assess a trigger signal and deduce a lack of electrical movement by the user wearing the device 400. Such an assessment may determine that the wrist, finger, or hand, for example, should move responsive to the triggered signal, but instead did not move. If a signal indicating movement is not received, the device 400 may determine the likelihood that the user has experienced an anomalous biologic event (e.g., a paretic side).

In some implementations, the device 400 may trigger indicators, information, messages, and/or warnings to be presented to the user of device 400 or to be presented to another device communicably coupled to device 400. For example, if a bad reading occurs from a sensor or electrode of device 400, a warning, a message, or other indicator may be generated and provided.

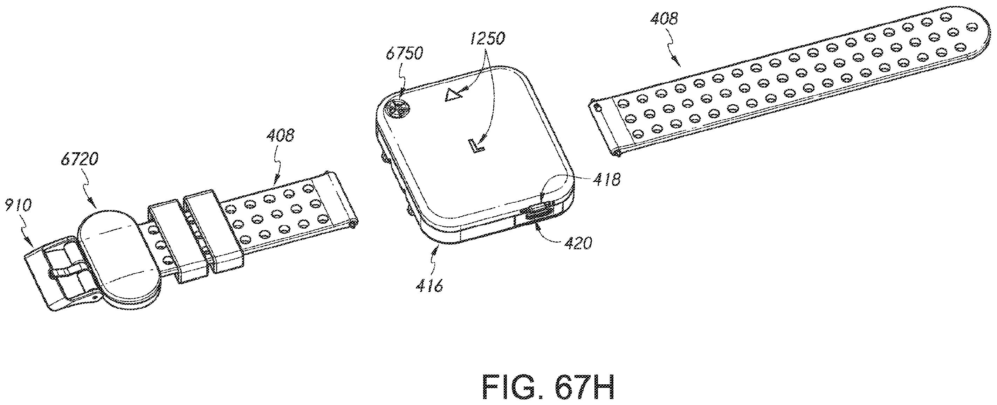

FIGS. 67A-67U show various implementations of a device 400. FIGS. 67A-67U depict various perspective views of one implementation of the device 400 comprising a body 416 featuring a heat source 410, blood volume sensor 412, skin temperature sensor 414, one electrode 902a, user input element(s) 418 and charging port 420. The body 416 may be arranged to contact the midline of the back (dorsal side) of the wrist when worn. In some implementations, the device 400 further comprises a band 408 with a buckle 910 an electrode housing 6720 that may carry additional electrodes 902, such as two electrodes 902b and 902c. For example, electrodes 902b and 902c are positioned such that they may contact a bottom region of a wrist. In some implementations, the electrodes 902b, 902c may be arranged to contact the midline of the bottom (palm side) of the wrist. FIGS. 67A-67W include surface and contour shading lines to illustrate aspects of the device 400; however, FIGS. 67H-67W include less surface/contour shading lines for clarity.

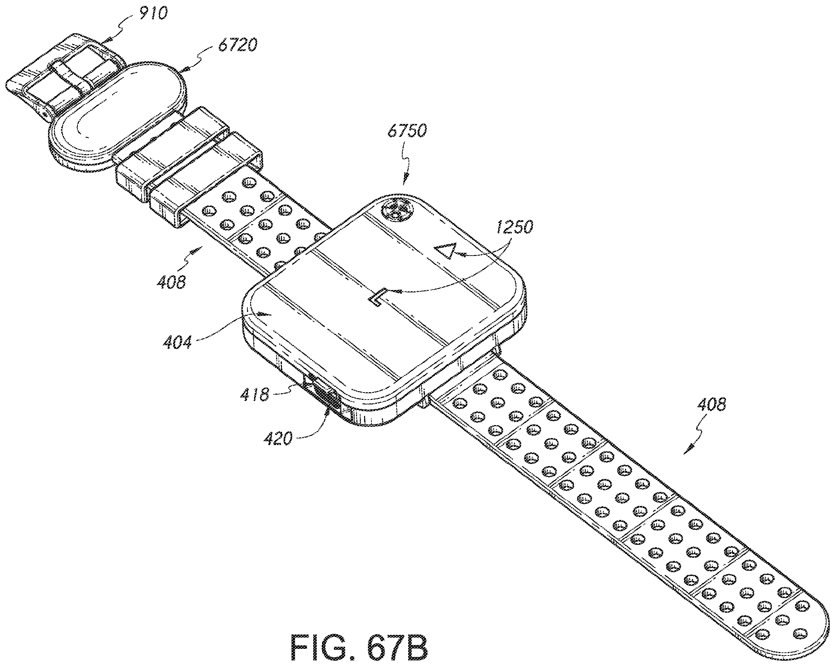

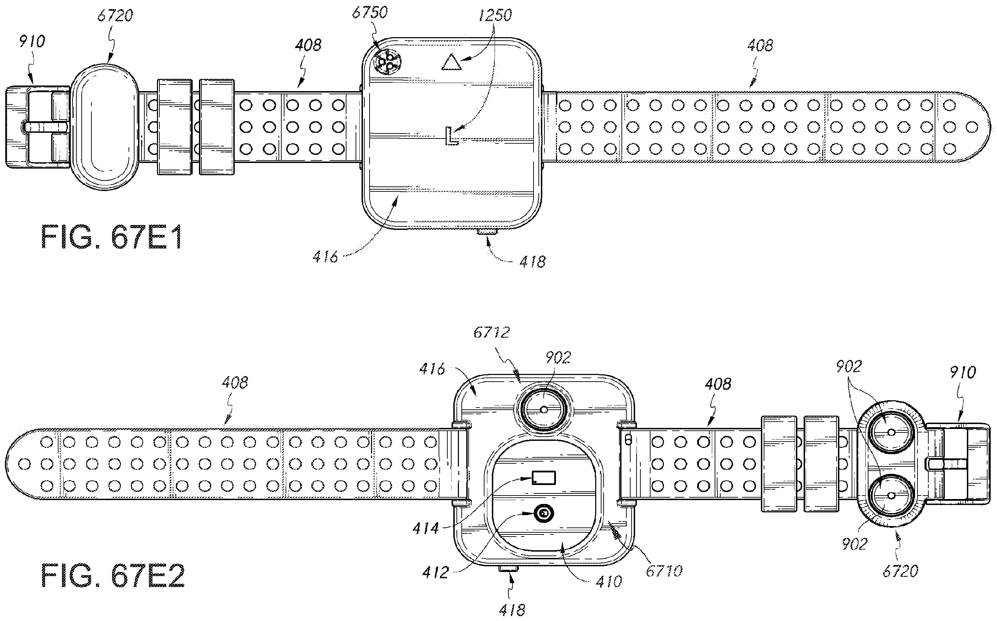

As shown in FIGS. 67A, 67B, 67E1, 67E2, 67H, 67L1, and 67L2, the body 416 of device 400 has a first surface 404 with one or more indicators 1250. The indicators 1250 may assist users with the proper fitting and/or placement of the device 400. For example, the first surface 404 of the body 416 can include an indicator 1250 (e.g., text reading "left" or "right," text reading "L" or "R", etc.) that indicates on which wrist the device 400 is to be worn. Alternatively or additionally, the first surface 404 of the body 416 may include one or more directional indicators 1250 (e.g., arrow, triangle, dot, or other marking) to mark a particular side(s) of the body 416 to help a user orient the device. For example, as illustrated in FIG. 67A, body 416 includes an indicator 1250 as an "L" to indicate the device 400 should be worn on a left wrist. Body 416 also includes an indicator 1250 as an arrow or triangle on one side to further indicate the device 400 should be oriented with the arrow 1250 located nearest the wrist (thereby also locating the user input element(s) 418 and charging port 420 toward the elbow). In some implementations, the indicators 1250 are arranged to indicate placement of the body 416 on a finger, arm, ankle, or other location. For example, indicators 1250 may include a similar "R" and arrow to indicate the body 416 should be worn on a right ankle with the arrow pointed downward. In some implementations, the indicators 1250 may help orient the body 416, heat source 410, blood volume sensor 412, skin temperature sensor 414, and electrode(s) 902 against the user. For example, the arrow indicator 1250 illustrated in FIG. 67A can enable a user to orient the body 416 on the wrist such that the (arrow) indicator 1250 is pointing towards the hand of the user or is closer to the hand of the user. When the body 416 is secured on or near the wrist of the user with the band 408, the indicator 1250 can help the user orient the body such that the y-axis (as illustrated) is approximately parallel to the forearm of the user. In some implementations, there is only one indicator 1250 and the devices can be reversibly worn between left and right arm. In some implementations, it may be advantageous for the optical sensor 412 to be further up on the forearm and away from the wrist.

As shown in FIGS. 67A, 67B, 67E1, 67E2, 67H, 67L1, and 67L2, the body 416 of device 400 may optionally include an ambient temperature sensor 6750 on the first surface 404. In some implementations, an ambient temperature sensor 6750 may be located on the first surface 404. In some implementations, an ambient temperature sensor 6750 may be located in the body 416, for example in cavity 406. The ambient temperature sensor 6750 may be exposed to the air via an aperture so that the ambient temperature sensor 1116 does not measure device temperature but rather environmental temperature. In some implementations, the ambient temperature sensor 6750 is located as far away as possible from the heat source 410, the skin of the wearer, and/or internal components that tend to generate heat, such as battery 407 and/or processor 409. In some implementations, an ambient temperature sensor 6750 may be located in the body 416 with a hole, vent, or other exposure to a surface of the body 416, for example the first surface 404. Additionally, in some implementations, it may be advantageous to position the ambient temperature sensor 6750 further away from the heat source 410 and closer to the edge or corner proximate to the arrow indicator 1250. As discussed herein, the heat source 410 can also be replaced by a cooling source in some implementations.

As illustrated in FGI. 67P, an ambient temperature sensor 6750 may be located in or under an opening 6756 in the body 416. The opening 6756 may include a cover or other protective structure over it to protect the ambient temperature sensor 6750 from direct exposure to dust or direct sunlight. For example, the opening 6756 may be covered by one or more spokes 6752 (for example, 2, 3, 4, 5, 6, 7, 8, 9, 10, or more spokes 6752) that may extend from a center to a perimeter of the opening 6756. Each spoke 6752 may be substantially equally spaced from adjacent spokes 6752. Spokes 6752 may be asymmetrically arranged, for example to provide increased protection and/or air access on one side of the ambient temperature sensor 6750. The spokes 6752 may also be circumferentially arranged symmetrically or asymmetrically about a center of the opening 6756. Spokes 6752 may further define air flow apertures 6754 to allow air to interact with the ambient temperature sensor 6750. Alternatively or additionally, ambient temperature sensor 6750 may be cooled by one or more vents, a blower for passing airflow, coolant, or another suitable mechanism. In some implementations, the body 416 includes multiple ambient temperature sensors 6750. In some implementations, the ambient temperature sensor 6750 includes a thermistor 6758, which may be placed in or under the opening 6756. In some implementations, a thermistor 6758 may be located on a circuit board inside the body 416 and may measure the ambient temperature via an opening 6756 and air flow apertures 6754.

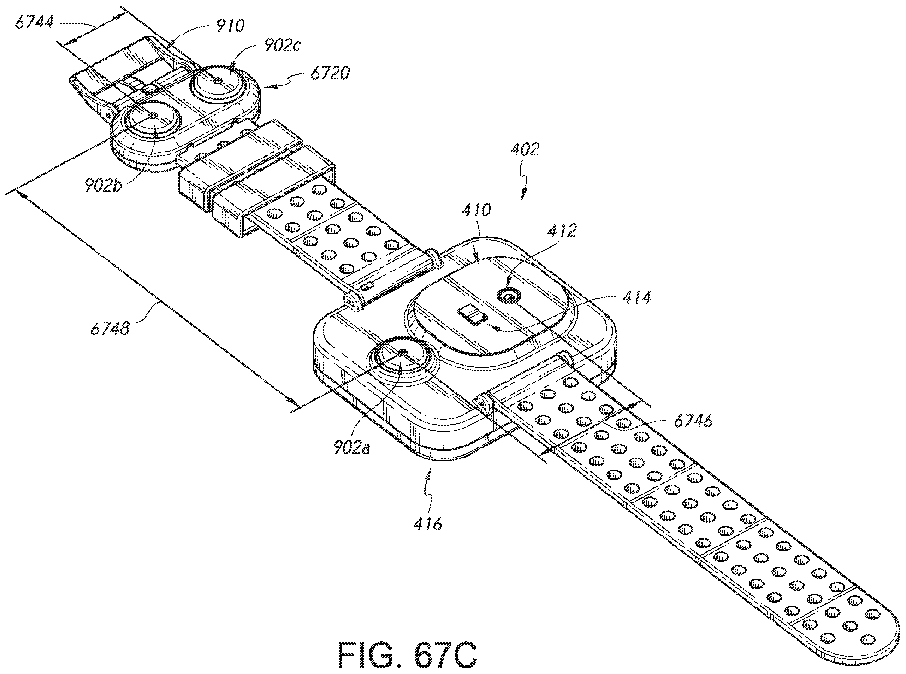

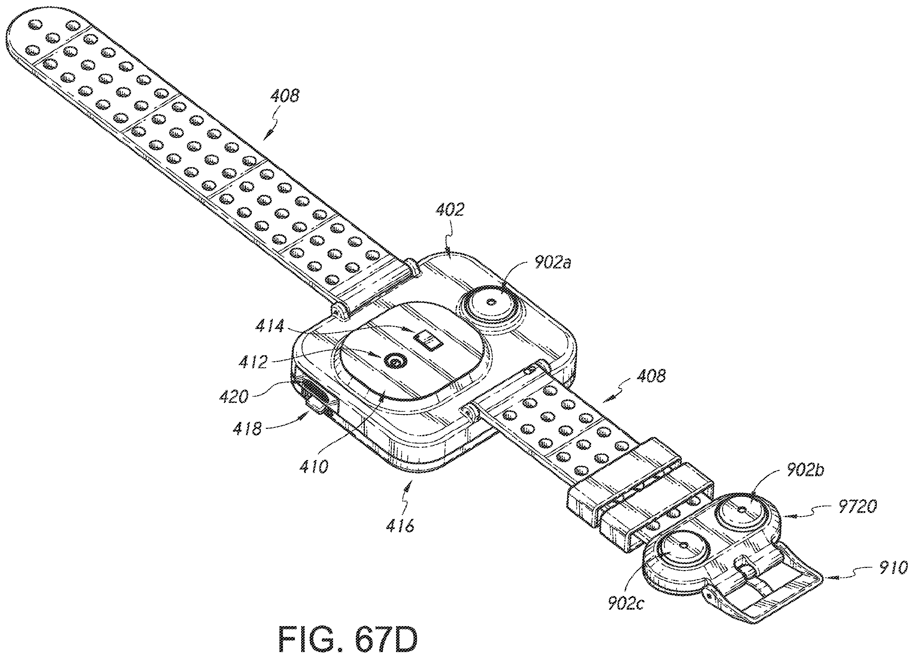

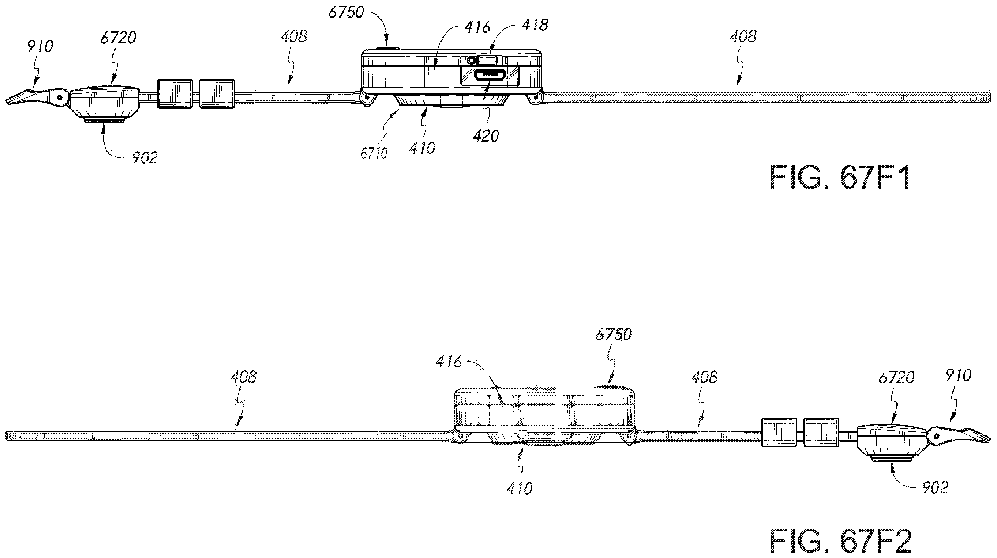

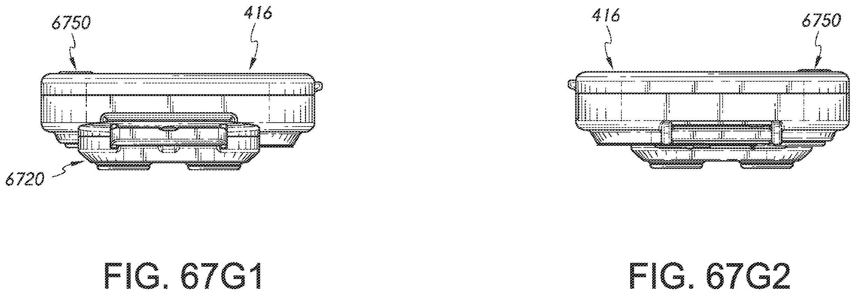

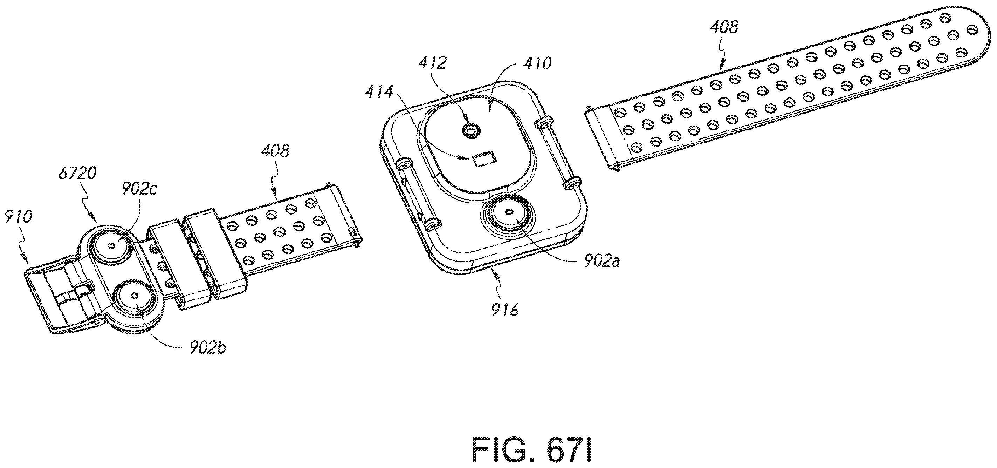

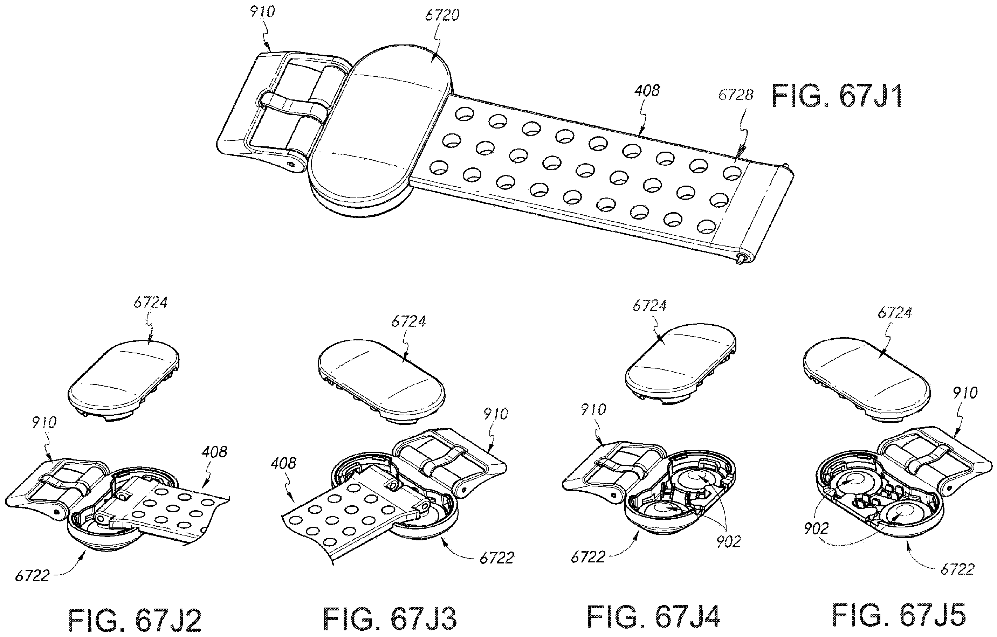

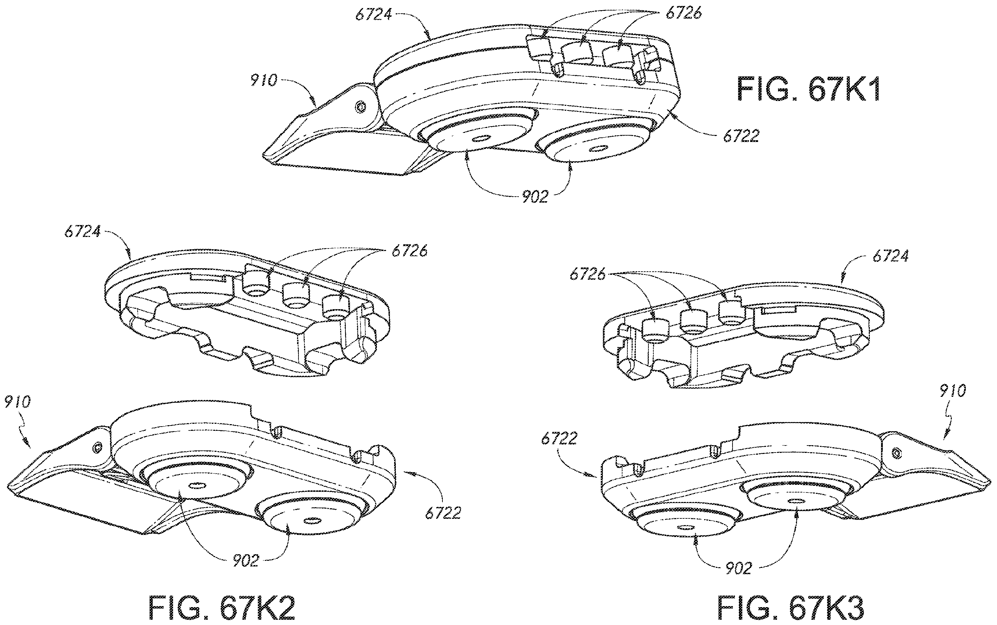

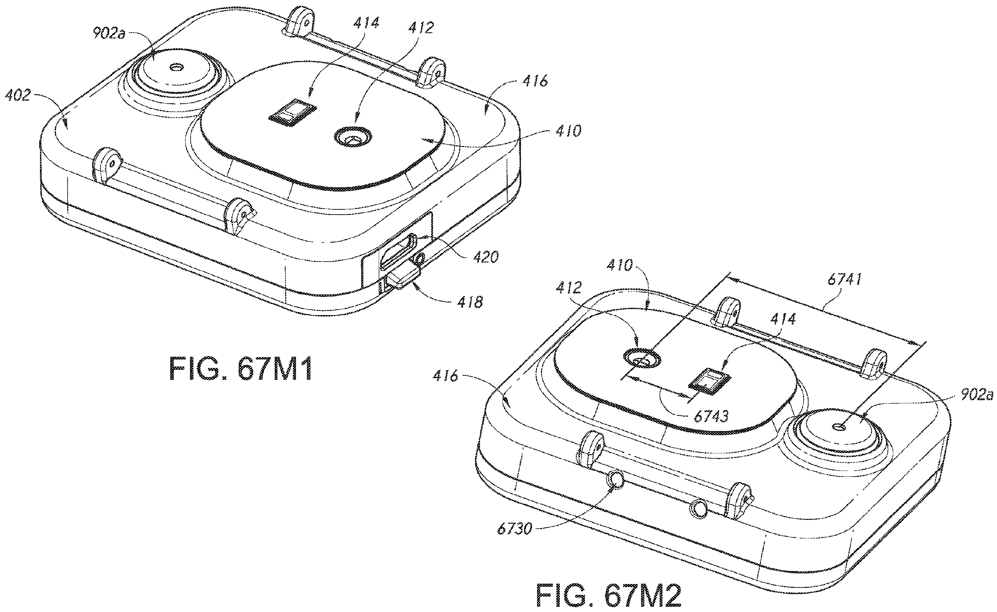

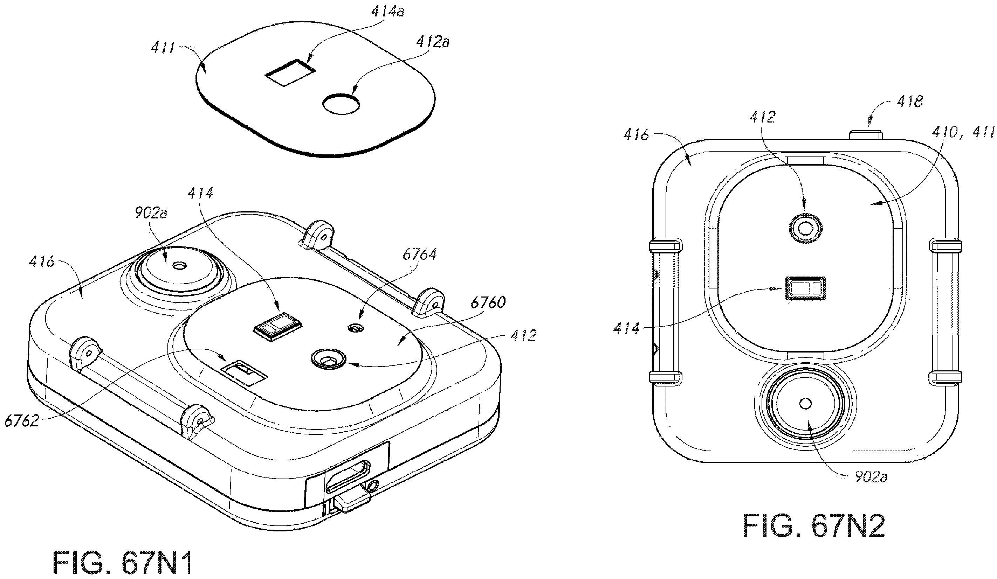

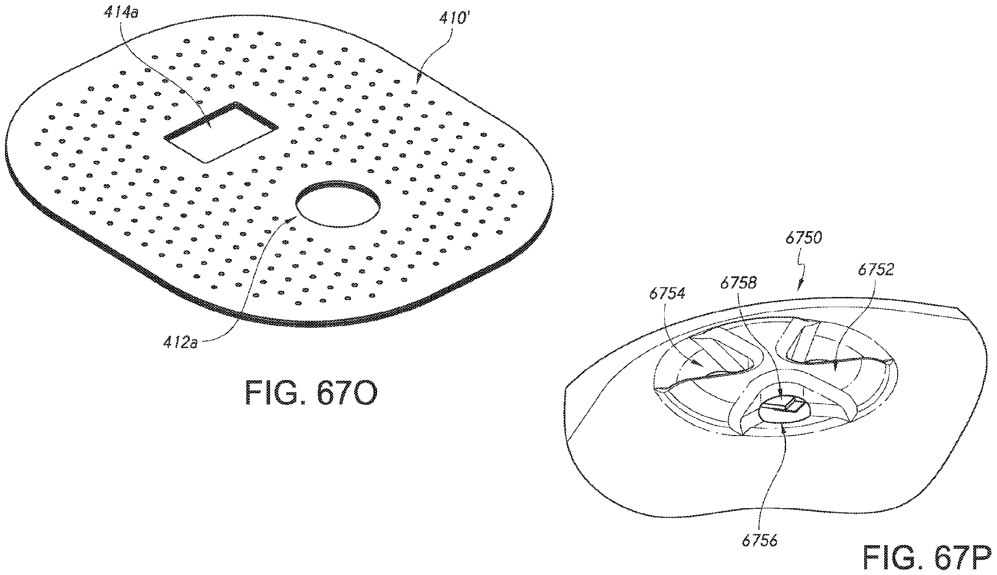

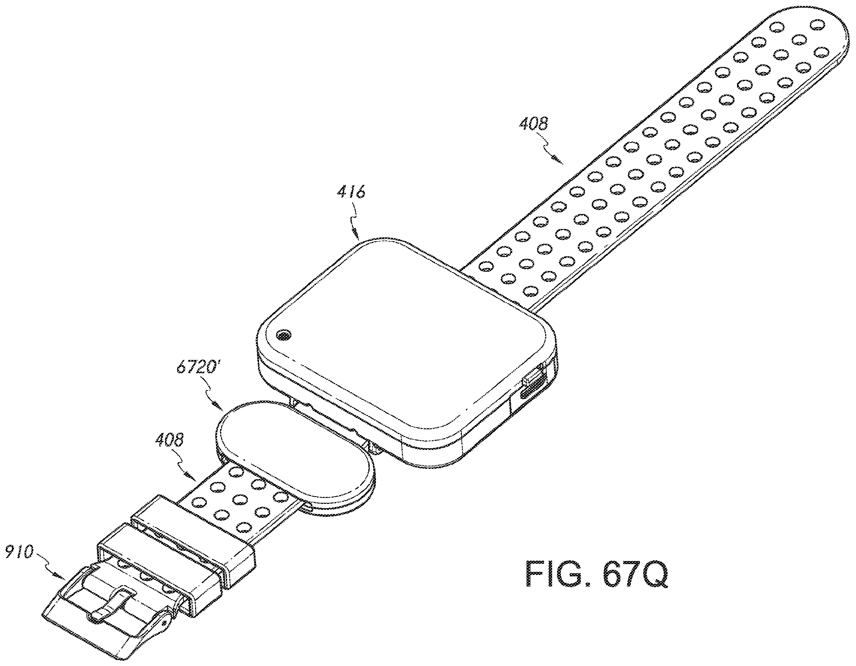

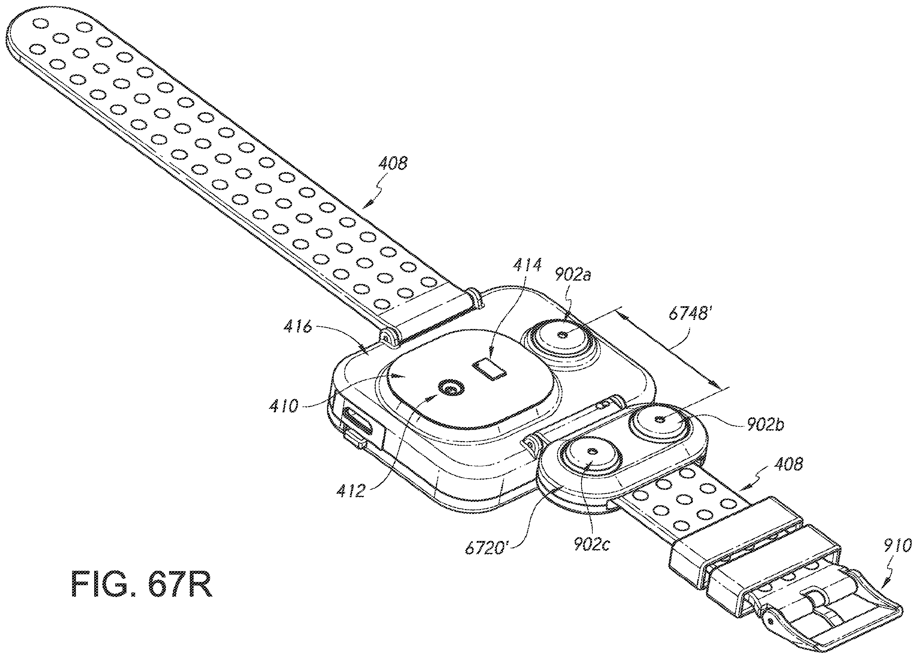

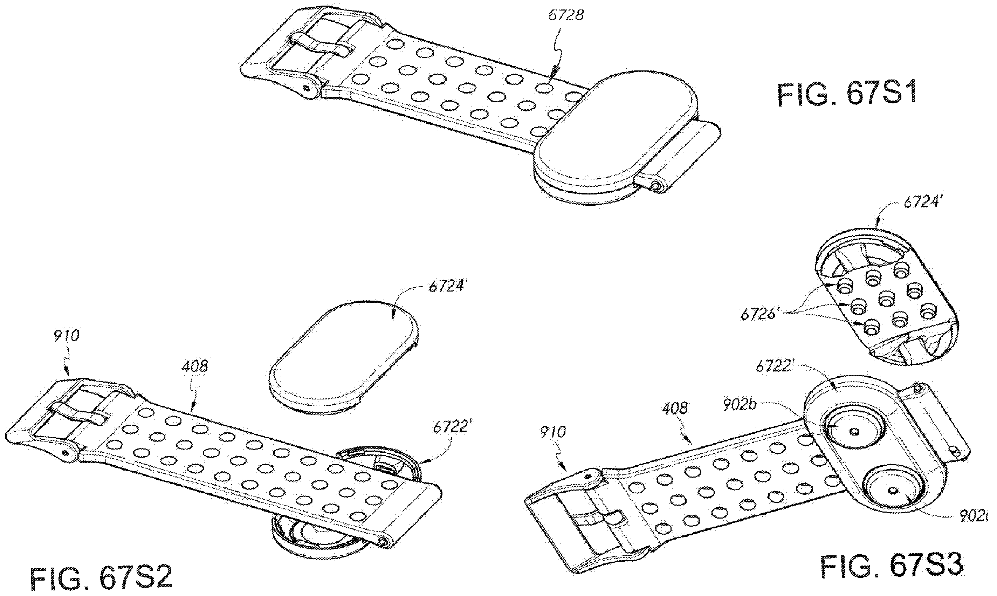

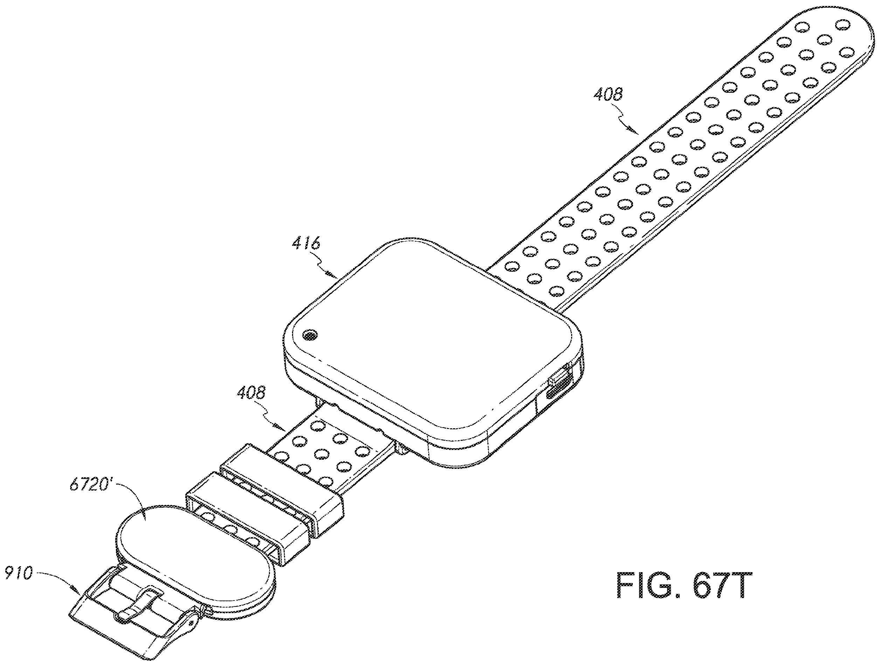

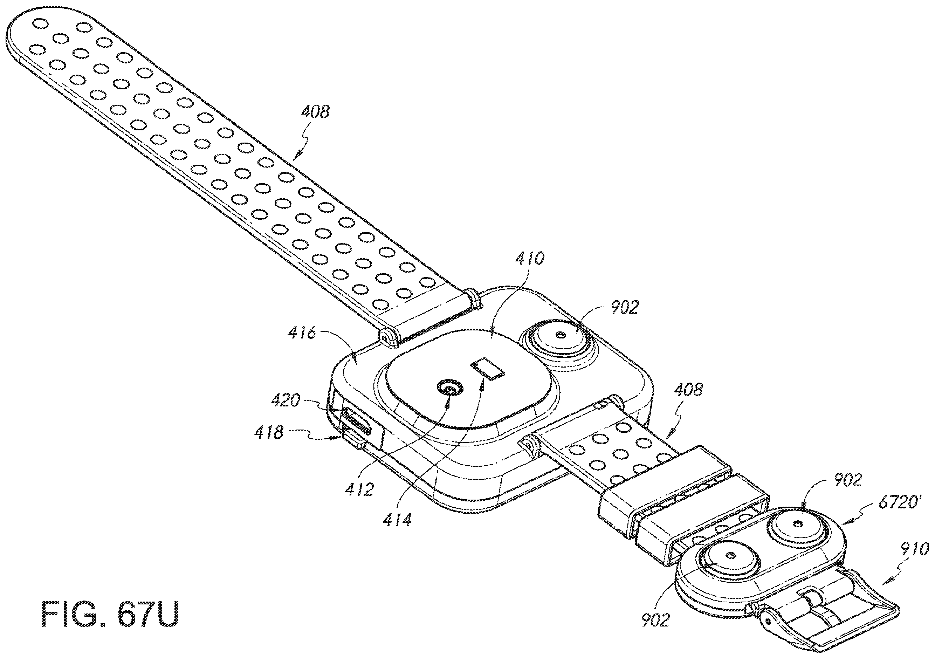

FIGS. 67C, 67D, 67E2, 67I, 67K1-67K3, 67M1, 67M2, 67N1, 67N2, 67R, 67S3, 67U, and 67V illustrate a skin-facing side of a device 400, including a second surface 402 of a body 416. Second surface 402 of a body 416 may be similar or identical to a second surface 402 of other devices described herein, for example second surface 402 may include a heat source 410, blood volume sensor 412, and skin temperature sensor 414. In some implementations, such as those illustrated in FIG. 67, second surface 402 includes one electrode 902a. A band 408 with a buckle 910 an electrode housing 6720 may carry additional electrodes 902, such as two electrodes 902b and 902c.

In some implementations, the device 400 includes a port 420 for electrically coupling the device to a power source, for example to charge a battery (such as battery 407) in the device 400. Additionally or alternatively, port 420 may electrically couple the wearable device to an external or remote computing device (e.g., laptop, desktop, server, workstation, etc.) to download data from the device or upload system parameters or install updates to the wearable device 400. Port 420 may also be used to connect auxiliary sensors, an input/output device (keyboard, joystick, buttons, switches, printer, camera, display), and/or memory unit. The wearable device may further include one or more user interface elements 418, for example one or more buttons and/or switches, that may be used for example to power on and off the device, to input user specific reactions, features, or characteristics, to customize an interface or functionality of the user device, to mark events, to initiate pairing or data transfer, to call for help, etc. User interface elements 418 may alternatively or additionally include output and/or feedback elements, such as a speaker, light, and/or haptic stimulator. In some implementations, user interface element 418 may be used, for example, to indicate power on, charging, low battery, pairing mode, heating phase, malfunction, health event, and/or other status of the device 400 and/or user. In some implementations, user feedback element 418 may be one or more LED behind a smoked, translucent, or transparent window. In some implementations, there is no display screen on the body 416.

Second surface 402 of body 416 may include a heat source 410, blood volume sensor 412, skin temperature sensor 414, and electrode 902a as discussed above. THe second surface 402 of body 416 may be arranged to contact the back (dorsal) side of the wrist when worn to locate the heat source 410, blood volume sensor 412, skin temperature sensor 414, and electrode 902 generally along a midline of the wrist. Blood volume sensor 412 and skin temperature sensor 414 may be placed within the heat source 410 spaced at a distance 6743 from each other. Distance 6743 may be about 10 mm to about 100 mm, for example 10 mm to about 20 mm, about 20 mm to about 30 mm, about 30 mm to about 40 mm, about 40 mm to about 50 mm, about 50 mm to about 60 mm, about 60 mm to about 70 mm, about 70 mm to about 80 mm, about 80 mm to about 90 mm, about 90 mm to about 100 mm, measured from a center point of the blood volume sensor 412 and a center point of the skin temperature sensor 414. Similarly, blood volume sensor 412 may be placed at a distance 6741 from the electrode 902a. Distance 6741 may be about 10 mm to about 200 mm, for example 10 mm to about 20 mm, about 20 mm to about 30 mm, about 30 mm to about 40 mm, about 40 mm to about 50 mm, about 50 mm to about 60 mm, about 60 mm to about 70 mm, about 70 mm to about 80 mm, about 80 mm to about 90 mm, about 90 mm to about 100 mm, about 100 mm to about 120 mm, about 120 mm to about 140 mm, about 140 mm to about 160 mm, about 160 mm to about 180 mm, about 180 mm to about 200 mm, measured from a center point of the blood volume sensor 412 and a center point of the electrode 902a.

The second surface 402 of the body 416 may include a raised platform 6760. Platform 6760 may include the heat source 410, blood volume sensor 412, and skin temperature sensor 414. In some implementations, the platform 6760 may include electrode 902a. Platform 6760 may improve contact between the skin and the heat source 410, blood volume sensor 412, and skin temperature sensor 414. In some implementations, the platform 6760 is sized to cover or substantially cover the back of the wrist when device 400 is worn. In some implementations, the platform 6760 is flexible and/or shaped, for example, curved, and may increase a contact area between the platform 6760 and the skin when the device 400 is worn. In some implementations, heat source 410 symmetrically surrounds the blood volume sensor 412 and/or symmetrically surrounds the skin temperature sensor 414. In some implementations, heat source 410 may surround the blood volume sensor 412 with an area approximately equal to the area of heat source 410 that surrounds the skin temperature sensor 414.

Heat source 410 may include a plate, as shown in FIG. 67N1. Heat source 410 may include a warming plate 411 for increased heat distribution and/or heat retention. The second surface 402 of body 416 may include an opening, such as opening 6762, to allow the heat source 410 and/or warming plate 411 to communicate with and/or access other components inside the body 416, for example the processor 409 and battery 407. The second surface 402 of the body 416 may include a thermistor 6764 or other temperature sensor for monitoring the temperature of the heat source 410 and/or warming plate 411. The thermistor 6764 may provide a heater temperature measurement, which may be used to control the heat source 410.

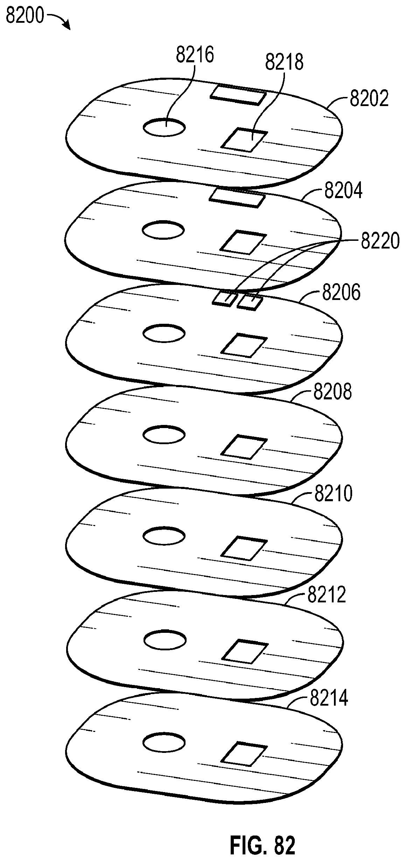

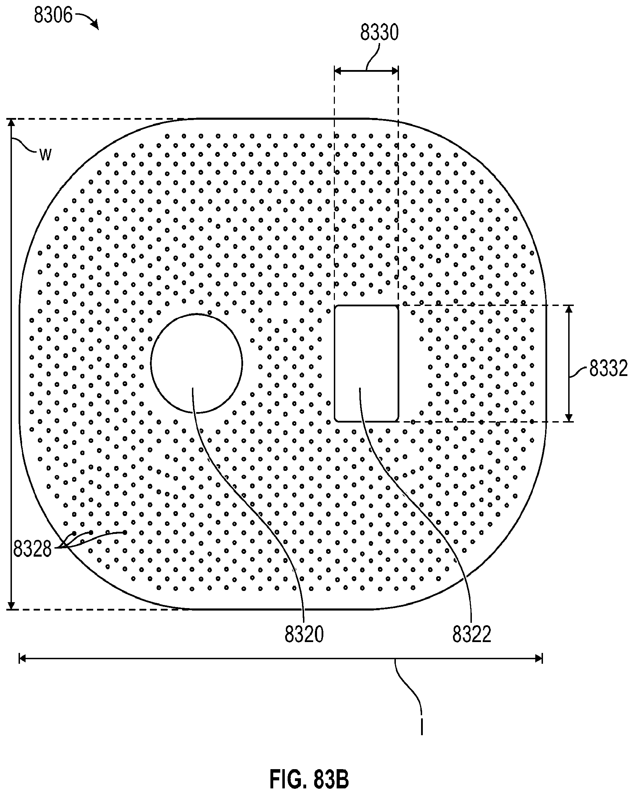

Warming plate 411 may include apertures for components surrounded and/or enclosed by the heat source 410, for example aperture 412a for blood volume sensor 412 and aperture 414a for skin temperature sensor 414. Aperture 412a and aperture 414a may allow improved contact between the skin and blood volume sensor 412 and skin temperature sensor 414. In some implementations, the blood volume sensor 412 includes two separate components, for example an emitter and a detector, and accordingly aperture 412a would include an aperture for each component. As discussed with respect to FIGS. 82 and 83B, heat source 410 may be a layered or laminate structure. As shown in FIG. 67O, a heat source 410' may include dimples and/or perforations for heat distribution and/or dissipation. Perforations may also improve adhesion. Alternative or additional features, such as ridges, channels, fins, and the like may also be included on a heat source 410 to improve uniform heating and cooling and/or direct heating and cooling. For example, surface features may be used to direct heat from the heat source 410, 410' toward or away from a wrist-facing side of the device 400. The surface features may alternatively or additionally be located on a warming plate 411. Heat source 410 may be positioned on a raised platform 6710 of the second surface 402 of the body 416. In some implementations, the heat source 410 is arranged farther from the hand than the electrode 902a when the device 400 is worn on the wrist.

In any of the implementations described herein, a wearable system or device, for example device 400, for detecting anomalous biologic events may include one or more electrodes 900, such as 902a, 902b, and 902c, positioned on the second surface 402 and/or a tensionable band 408 of the system 400. For example, as shown in FIGS. 67C, 67D, 67E2, 67I, 67K1-67K3, 67M1, 67M2, 67N1, 67N2, 67R, 67S3, 67U, and 67V, electrodes 902 can be positioned on the second surface 402 of the wearable system 400 and/or a skin-facing surface of the band 408. The electrodes 902 may be located away from the surface of the device 400. For example, electrode 902a may be placed on a raised platform 6712 of the second surface 402 of the body 416. Raised platform 6712 may be separate from raised platform 6710 as shown in FIGS. 67E2. As discussed above with regard to electrodermal sensors 424 and 426, electrodes 902 may be spaced at preselected distances. In some implementations electrodes 902b and 902c are spaced at a distance 6744. Distance 6744 may be about 5 mm to about 100 mm, for example 5 mm to about 10 mm, about 10 mm to about 20 mm, about 20 mm to about 30 mm, about 30 mm to about 40 mm, about 40 mm to about 50 mm, about 50 mm to about 60 mm, about 60 mm to about 70 mm, about 70 mm to about 80 mm, about 80 mm to about 90 mm, about 90 mm to about 100 mm, measured from a center point of each electrode 902. Further, electrodes 902b and 902c may be spaced apart from the heat source 410 and/or electrode 902a by distance 6748, which can be about 40 mm to about 300 mm, for example about 40 mm to about 50 mm, about 50 mm to about 60 mm, about 60 mm to about 80 mm, about 80 mm to about 100 mm, about 100 mm to about 120 mm, about 120 mm to about 150 mm, about 150 mm to about 175 mm, about 175 mm to about 200 mm, about 200 to about 225 mm, about 225 mm to about 250 mm, about 250 mm to about 275 mm, about 275 mm to about 300 mm, measured from a center point of the electrodes 902b, 902c to a center point of the heat source 410 and/or the electrode 902a. Still further, electrode 902a may be spaced apart from a heat source 410 by a distance 6746, which can be about 10 mm to about 20 mm, about 20 mm to about 30 mm, about 30 mm to about 40 mm, about 40 mm to about 50 mm, about 50 mm to about 60 mm, about 60 mm to about 70 mm, about 70 mm to about 80 mm, about 80 mm to about 90 mm, about 90 mm to about 100 mm, measured from a center point of the electrode 902a and a center point of the heat source 410. When the body 416 is secured to the wrist with the band 408, in some implementations, the electrode 902b and 902c are positioned on the band such that they contact the skin approximately along a midline of palm side of the wrist. The electrode 902a may be positioned approximately on a midline on the dorsal side of the wrist.

Band 408 of a device 400 may be similar or identical to band 408 of other devices described herein in some or all respects. For example, band 408 may be an adjustable or tensionable band 408 including a buckle 910 on a first side and a series of receiving notches on a second, opposing side. Band 408 may allow for similar or alternative number and arrangements of electrodes 902a-c. In some implementations, a Velcro^{®} or another hook-and-loop fastener (not shown), and/or a stretchable material (e.g., silicone, rubber, Lycra, Spandex, Elastane, neoprene, leather, fabric, etc.) can be employed along at least a portion of the band 408.

In some implementations, the band 408 may include connectors (not shown) for connecting electrodes (for example, electrodes 902b and 902c) to other elements housed in the body 416 including, but not limited to, a power supply (for example, battery 407 shown in FIG. 43) and a processor (for example, processor 409 shown in FIG. 43). The connectors can be electrical traces, for example wires, conductive ink, circuitry or another connector, optical connectors, for example fiber optic cable, or other suitable connector that may be on, or at least partially embedded in a band 408. In some implementations, these traces from the electrodes 902a-c of the band 408 to elements of the body 416 may be formed by cold molding or insert molding. In some implementations, connector wires may be threaded, woven, or sewn into the material of the band 408 and/or holes, channels, or other apertures in the band 408. In some implementations, the band 408 is at least partially made of a conductive material. In some implementations, connectors from a band 408 are connected to components (e.g., battery 407, processor 409) inside the body 416 through one or more holes 6730 in the body 416.

The band 408 may carry an electrode housing 6720 that may hold additional electrodes 902, such as two electrodes 902b and 902c. The electrode housing 6720 may be integral with or formed as a unitary structure with the buckle 910. As illustrated in FIGS. 67J1-J5, an electrode housing 6720 may be openable and/or removable from the band 408. The electrode housing 6720 may include a lower portion 6722 and upper portion 6724 for housing one or more electrodes 902, for example electrodes 902b and 902c. The lower portion 6722 and/or upper portion 6724 may include features for securely seating and retaining electrodes 902 and/or an end of band 408 and/or a buckle 910 and/or upper portion 6724. These securing features can include recesses, notches, alignment pins, fasteners, and/or clips to hold and/or align the assembly components. As illustrated in FIGS. 67K1-67K3, the upper portion 6724 may include pins 6726 for retaining and aligning an end of band 408. In some implementations, one or more holes 6728 in band 408 hold one or more pins 6720. For example, 1, 2, 3, 4, 5, 6, 7, 8, 9, or 10 or more pins 6726 may be inserted through a corresponding number of holes 6728 to secure the electrode housing 6720 to the band 408. In some implementations, the electrode housing 6720 is secured to a particular location on the band 408, where the electrode housing 6720 cannot slide (for example, with the pins 6726 and holes 6728). In some implementations, the lower portion 6722 and upper portion 6724 securely mate together and cannot be opened. In some implementations, the lower portion 6722 and upper portion 6724 securely mate together and can be opened by manipulating the pieces, for example, by sliding or twisting the pieces against each other, and/or opening a latch, button, or other retention feature. In some implementations, the electrode housing 6720 is secured to the band 408 and can slide along the length of the band 508. For example, the lower portion 6722 and upper portion 6724 may secure together over and/or around the band 408 and provide a clamping force to the band 408 to secure the electrode housing 6720 in position while being slidable along the band 408.

In some implementations, such as the device 400 shown in FIGS. 67Q-67U, an electrode housing 6720' may hold additional electrodes 902, such as two electrodes 902b and 902c. Electrode housing 6720' may be identical to electrode housing 6720 in some or all respects. As shown in FIGS. 67Q and 67R, the electrode housing 6720' can be placed at any part of band 408, including near the body 416 as in FIG. 67R or near the buckle 910 as in FIGS. 67T and 67U. Electrode housing 6720' may also be placed on the opposing band 408 (without the buckle 910). This arrangement can allow the electrode housing 6720', and therefore electrodes 902 such as electrodes 902b and 902c, to be placed at a distance 6748' from the electrode 902a and/or heat source 410. Distance 6748' may be the same as distance 6748 discussed above, or may be selected to facilitate measurements and/or calculations such as EMG, sEMG, ECG, BioZ, and the like as discussed herein. As illustrated in FIGS. 67S 1- 67S3, the electrode housing 6720' may include a lower portion 6722' and an upper portion 6724,' each of which may be similar or identical to upper portion 6722 and lower portion 6724 respectively, in some or all respects. As shown in FIGS. 67S2-67S3, lower portion 6722' includes recesses for one or more electrodes 902 and a portion of a band 408. Upper portion 6724' may include pins 6726' for retaining and aligning a portion of band 408. For example, one or more pins 6726' may extend into or through one or more holes 6728 in band 408.

In some implementations, electrode housing 6720, 6720' may further include additional components. For example, electrode housing 6720, 6720' may include a battery, charging circuit, preamplifier, processor, wired and/or wireless transmitter (e.g., Bluetooth^{®} or other transmitter), blood volume sensor, skin temperature sensor, electrodermal sensor, ambient temperature sensor, and/or user interface including a light (such as one or more LED), display, speaker, buzzer, button, switch, touchscreen and/or the like. In some implementations, electrode housing 6720, 6720' may include connective elements for electrically connecting internal components to electrical connectors in the band 408, and thereby to the body 416. For example, pins 6726 may be conductive and may pierce and/or clamp against conductive elements in the band 408. In some implementations, electrode housing 6720, 6720' includes one or more electrodes 902, for example, 1, 2, 3, 4, 5, 6, 7, and 8 or more electrodes 902.

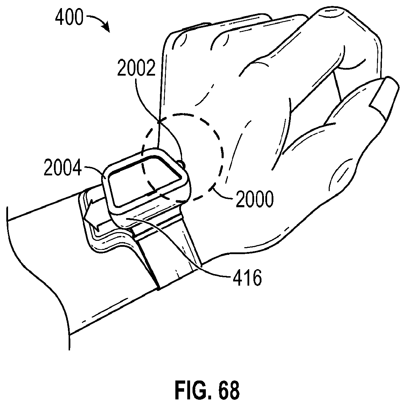

FIG. 68 shows a perspective view of one implementation of a device 400 being worn about a human wrist. In some implementations, when the device 400 features a larger body 416 such as that shown in FIG. 68, a wrist facing edge 2002 of the device 400 may collide with the back of the hand during certain motions of the wrist (circle 2000 highlighting impact) that sends the arm facing edge 2004 down into the arm. In addition to causing annoyance, discomfort, or pain to a wearer, such a motion can interfere with a consistency of measurement taken by the device 400, in some implementations. Many implementations described herein avoid this by implementing an asymmetric design that positions the body 416 of the device away from the range of motion of the wrist. Additionally, in implementations where a device 400 must be worn on both wrists, an asymmetric design can provide an easily recognizable visual clue as to which device 400 is to be worn on which wrist. Furthermore, an asymmetric design can allow for a more comfortable fit when worn about the same wrist and therefore adjacent to another wrist accessory, such as a watch or bracelet.

As used herein, an "asymmetric" body may comprise a plurality of sidewalls in which a first sidewall or arm facing sidewall, opposite a second sidewall or hand facing sidewall, is offset from a centerline of the band by about 0.5 mm to about 15 mm; about 1 mm to about 10 mm; about 5 mm to about 15 mm; about 0.1 mm to about 25 mm; etc.

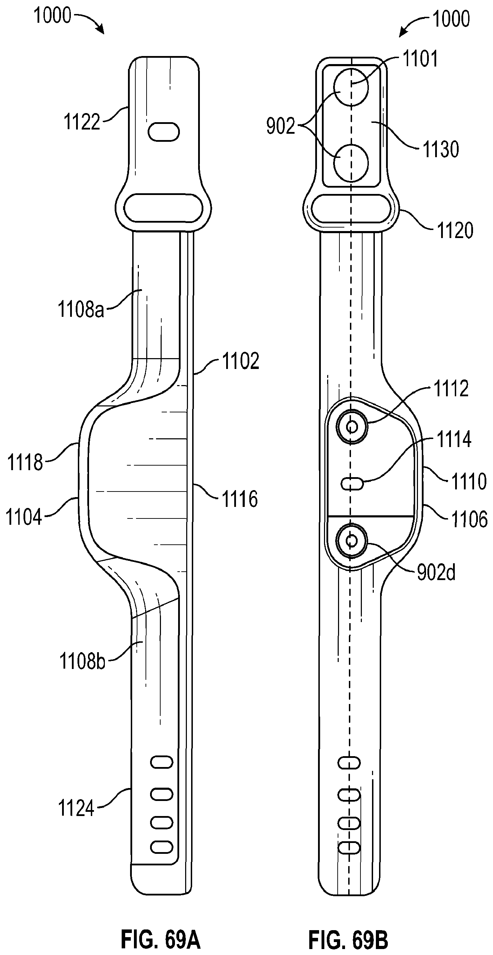

FIGS. 69A and 69B show a top and bottom view, respectively, of one implementation of a device 1000 for the detection of an anomalous biological event. In some implementations, the device 1000 comprises an asymmetric body 1106 and a band 1108. The asymmetric body 1106 can be shaped such that a wrist facing edge 1102 has a continuous, sleek contour with the band 1108 while an arm facing edge 1104 can take on a larger shape to compensate for displaced components within. As seen in the top view of the device 1000, as shown in FIG. 69A, the device can comprise an ambient temperature sensor 1116 and an indicator light 1118. In certain implementations, the indicator light 1118 can be positioned near the wrist facing edge 1102 while the ambient temperature sensor 1116 can be positioned near the arm facing edge 1104. The ambient temperature sensor 1116 may be exposed to the air via an aperture so that the ambient temperature sensor 1116 does not measure device temperature but rather environmental temperature. In some implementations, the indicator light 1118 can be one or more LED behind a smoked, translucent, or transparent window. As seen in the bottom view of the device 1000 as shown in FIG. 69B, the asymmetric body 1106 features a heat source 1110, an optical heart rate sensor 1114, an infrared body temperature sensor 1112, and one electrode 902d. In some implementations, the electrode 902d on the asymmetric body 1106 can be a circular electrode positioned in line with the infrared body temperature sensor 1114 and the centerline length 1101 of the band 1108, although other electrode shapes and positions can be employed in other implementations. One side of the band 1108 can comprise two circular electrodes 902b and 902c arranged along a centerline 1101 of the band 1108, although different shapes, numbers, and arrangements of electrodes 902b and 902c can be used in alternative implementations. In some implementations, the electrodes 902b and 902c of the band 1108 can be secured with a single metal plate 1130. In certain implementations, one side of the band 1108a can feature a loop 1120 and a flange 1122 while the other side 1108b can feature a series of holes or notches 1124 to engage with the flange 1122 when notched side 1124 passes through the loop 1120; although in other implementations, a variety of attaching mechanisms can be employed.

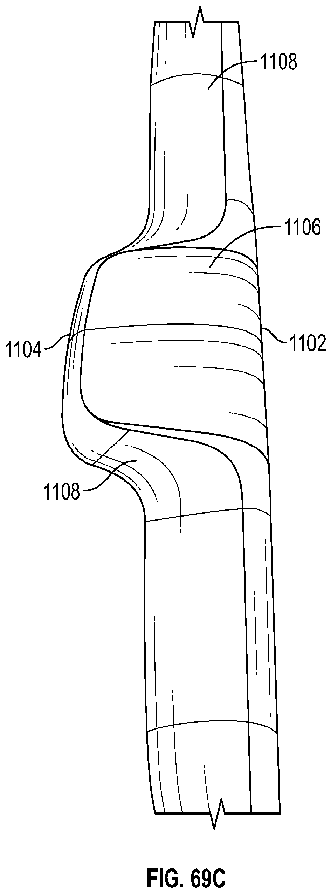

FIG. 69C depicts a perspective view of the asymmetric body 1106 in one implementation that illustrates its asymmetric design. In addition to its continuous contour with the band 1108, the wrist facing edge 1102 of the asymmetric body features a smooth profile curve in cross-section that exhibits no sharp edges towards a back of the hand and the wrist in many implementations. The arm facing edge 1108, being at least partially removed or apart from any range of motion from any body parts when worn, can have a bulkier shape with more abrupt edges in many implementations.

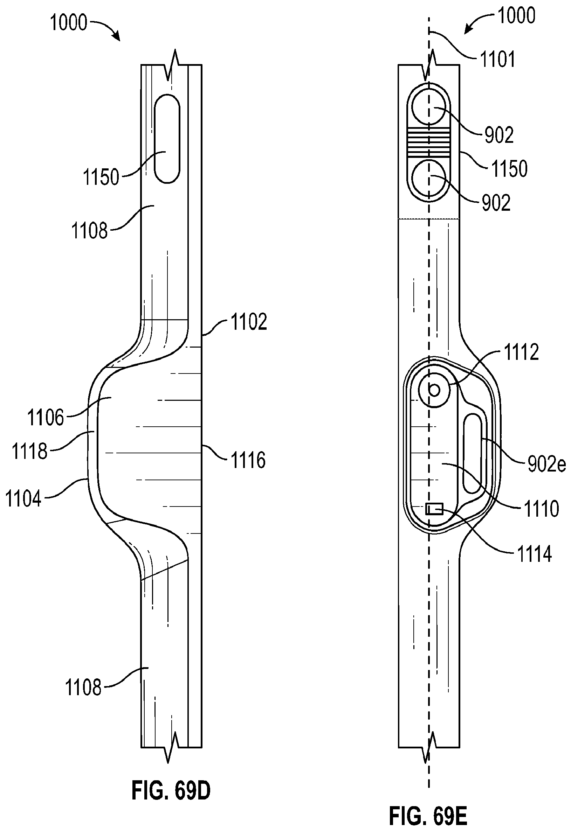

FIGS. 69D-69K show various views of another implementation of a device 1000 for detecting anomalous biological events. In some implementations, the device 1000 comprises an asymmetric body 1106 and a band 1108. The asymmetric body 1106 can be shaped such that a wrist facing edge 1102 has a continuous, sleek contour with the band 1108 while an arm facing edge 1104 can take on a larger shape to compensate for displaced components within. As seen in the top view of the device 1000, as shown in FIG. 69D, the device can comprise an ambient temperature sensor 1116 and an indicator light 1118. In certain implementations, the indicator light 1118 can be positioned near the wrist facing edge 1102 while the ambient temperature sensor 1116 can be positioned near the arm facing edge 1104. In some implementations, the indicator light 1118 can be one or more LED behind a smoked, translucent, or transparent window. As seen in the bottom view of the device 1000 as shown in FIG. 69E, the asymmetric body 1106 features a heat source 1110, a blood volume sensor 1114, an infrared body temperature sensor 1112, and one electrode 902. In some implementations, the electrode 902e on the asymmetric body 1106 can be an oval or elongate shape positioned nearer the arm facing edge 1104, thus offset from and adjacent to the heat source 1110, although different shapes, numbers, and arrangements of electrodes can be used in alternate implementations.

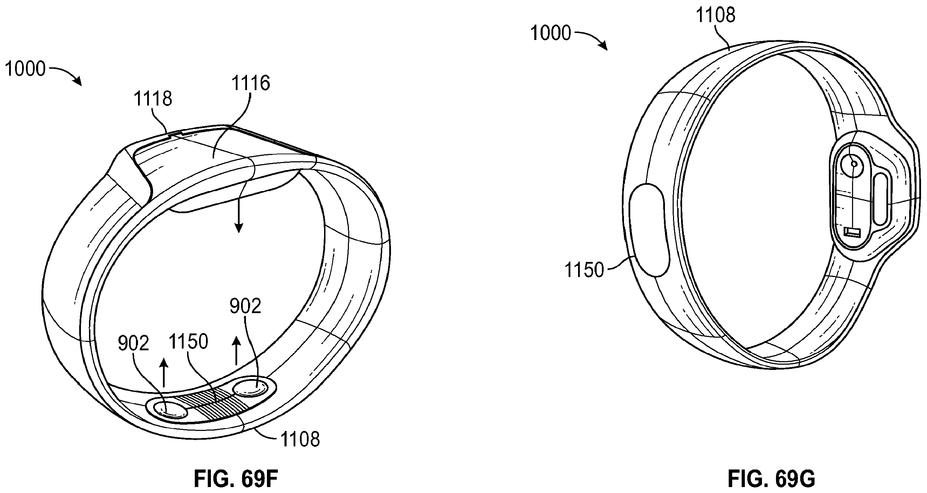

In some implementations, such as those depicted in FIGS. 69D-69K, the band 1108 can be a stretchable continuous band. In these implementations, no buckles or clasps are needed. Instead, the band 1108 loops around as one continuous element (as shown in FIGS. 69F and 69G) but comprises an elastic or stretchable material capable of conforming to a user's wrist. In many implementations, the band 1108 further comprises electrodes 902. In the implementation of FIGS. 69E-69G, the band 1108 features two electrodes 902b and 902c having a circular shape along a centerline 1101 of the band 1108 and a flexible area 1150 between them, although other shapes, numbers, and arrangements of electrodes can be used in alternative implementations. The flexible area 1150 is configured to conform to a wrist or arm of a user.

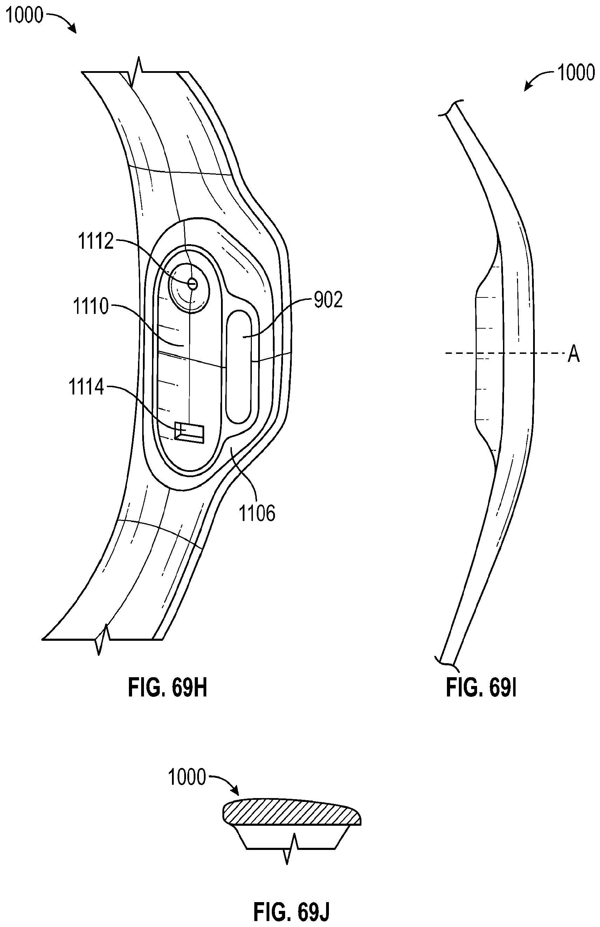

FIGS. 69H-69J depict various detailed views of the asymmetric body 1106 of one implementation of the device 1000. FIG. 69H shows a close up of the asymmetric body and the arrangement of a heat source 1110, an optical heart rate sensor 1114, an infrared body temperature sensor 1112, and one elongate electrode 902. FIG. 69I shows a side profile view depicting the relative proportions and curvature of the asymmetric body 1106 while FIG. 69J shows a cross section along line A in FIG. 69I.

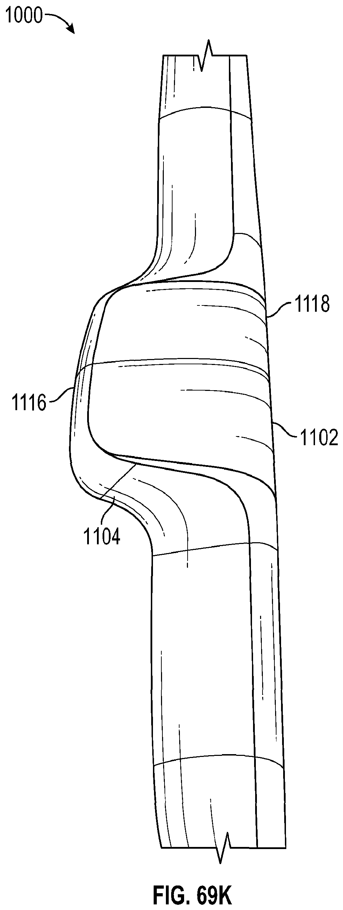

FIG. 69K shows a top, detailed view of an implementation of the device 1000. In this implementation, an indicator light 1118 is positioned behind a tinted window near the wrist facing edge 1102, and an ambient temperature sensor 1116 is positioned near the arm facing edge 1104.

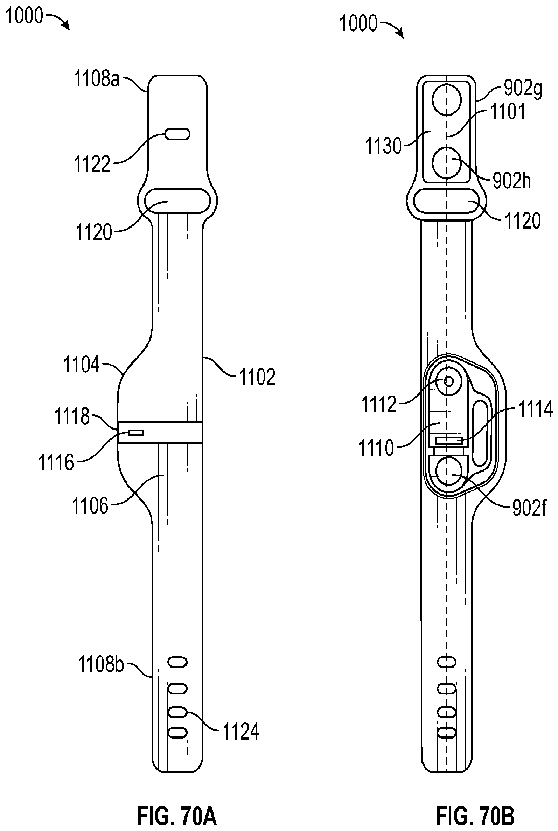

FIGS. 70A-D show various views of still another implementation of a device 1000 for the detection of an anomalous biological event. The device 1000 can comprise an asymmetric body 1106 and a band 1108. The asymmetric body 1106 can be shaped such that a wrist facing edge 1102 has a continuous, sleek contour with the band 1108 while an arm facing edge 1104 can take on a larger shape to compensate for displaced components within. As seen in the top view of the device 1000, as shown in FIG. 70A, the device can comprise an ambient temperature sensor 1116 and an indicator light 1118. In certain implementations, both the indicator light 1118 and the ambient temperature sensor 1116 can be positioned near the arm facing edge 1104. In some implementations, the indicator light 1118 can be one or more LED behind a smoked, translucent, or transparent window. As seen in the bottom view of the device 1000 as shown in FIG. 69B, the asymmetric body 1106 features a heat source 1110, an optical heart rate sensor 1114, an infrared body temperature sensor 1112, and one electrode 902. In some implementations, the electrode 902f can be a circular electrode in line with the infrared body temperature sensor 1112 and centerline length of the band 1108, although other electrode shapes and positions can be employed in other implementations. In some implementations, the temperature sensor 1112 may be placed in a center of the device 1000 to ensure that the sensor 1112 is unaffected by ambient temperature.

One side of the coupling member 1108 can comprise two circular electrodes 902g and 902h arranged along a centerline of the band, although different shapes, numbers, and arrangements of electrodes 902g and 902h can be used in alternate implementations. In some implementations, the electrodes 902g and 902h of the band 1108 can be secured with a single metal plate 1130. In certain implementations, one side of the band 1108a can feature a loop 1120 and a flange 1122 while the other side 1108b can feature a series of holes or notches 1124 to engage with the flange 1122 when notched side 1124 passes through the loop 1120; although, in other implementations, a variety of attaching mechanisms can be employed.

In operation, the infrared temperature sensor 1112 may be used to measure a temperature of the skin. For example, if the heat source 1110 remains off, the sensor 1112 may be used as a tissue/body temperature sensor. However, if the heat source 1110 is in use, then the sensor 1112 may function to measure the temperature of the tissue before, during, and after heating. In some implementations, the temperature sensor 1112 may detect whether the heating from heat source 1110 causes a local effect or a global effect (e.g., flushing of the tissue). In some implementations, the temperature sensor 1112 is on the same surface as the heat source 1110.

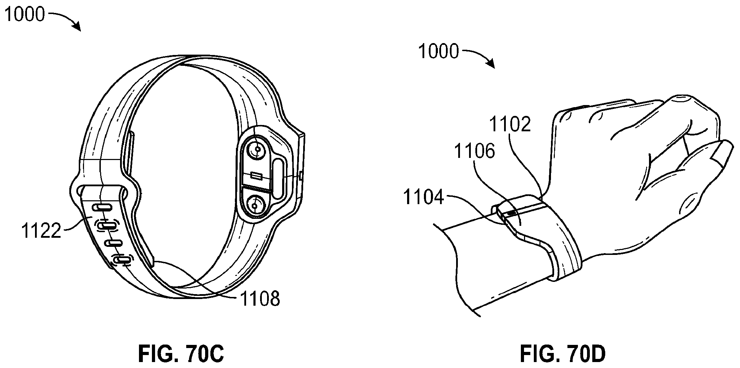

FIG. 70C depicts a detailed view of an implementation of the device 1000 with the band 1108 secured to form a loop. The electrodes (not shown) of the band 1108 are positioned in line with the length of the band 1108 in order to allow for a narrow fit of the band 1108 about a user's wrist. FIG. 70D depicts an implementation of the device 1000 being worn about a user's wrist. The asymmetric body 1106 of the device 1000 with its continuous wrist facing edge 1102 and its asymmetric arm facing edge 1104 avoid interfering with the motions of the user's wrist and hand.

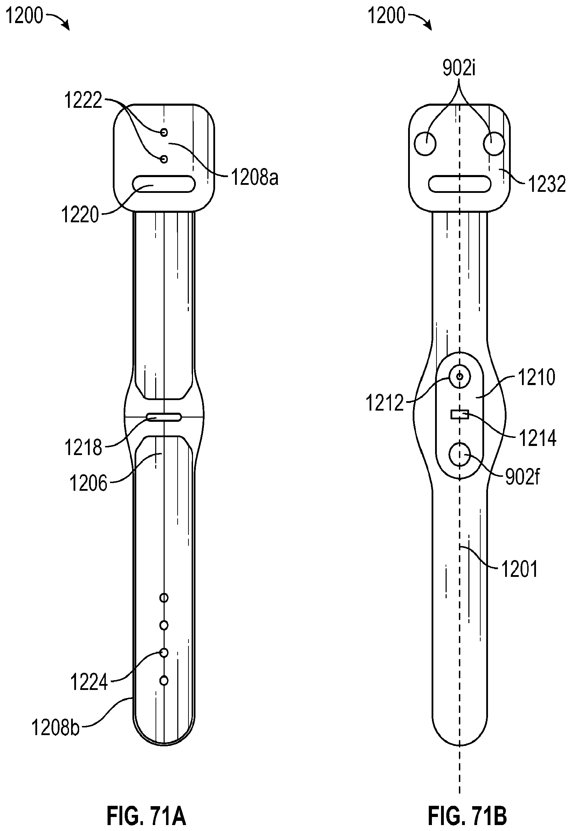

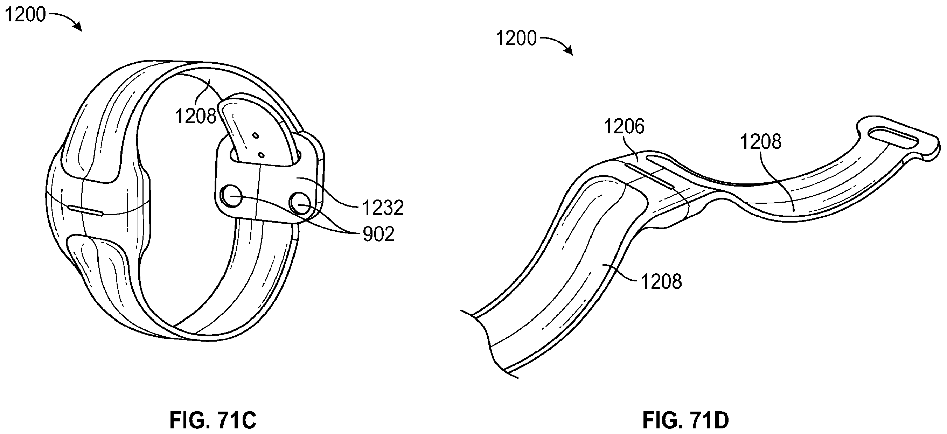

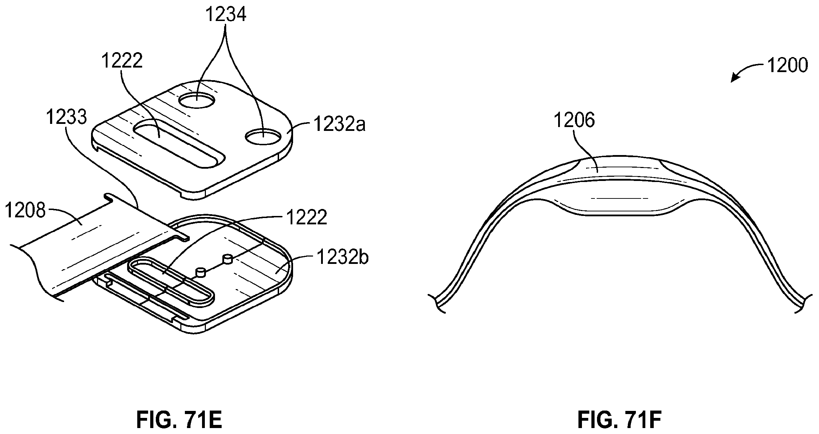

FIGS. 71A-F depict various views of another implementation of a device 1200 for detecting anomalous biological events. The device 1200 can comprise a symmetric body 1206 and a band 1208. The symmetric body 1206 can be shaped to have low-profile, continuous edges so as to avoid colliding with the back of a user's hand during certain motions of the wrist. As seen in the top view of FIG. 71A, the device 1200 can comprise an indicator light 1218. In other implementations, the device 1200 can further comprise an ambient temperature sensor (not shown). As seen in the bottom view of the device 1200 in FIG. 71B, the symmetric body 1206 (i.e., first sidewall opposite a second sidewall is not substantially offset) features a heat source 1210, an optical heart rate sensor 1214, an infrared body temperature sensor 1212, and one electrode 902. In some implementations, the electrode 902f can be a circular electrode in line with the infrared body temperature sensor 1214 and along a centerline of the band 1208; although other electrode shapes, numbers, and arrangements can be employed in other implementations. One side of the coupling member 1208 can comprise two electrodes 902i positioned side-by-side and perpendicular to the length of the coupling member 1208, although different numbers and arrangements of electrodes 902f and 902i can be used in alternate implementations. In some implementations, the electrodes can be encased in a rigid or semi-rigid housing 1232 so as to keep them separate, isolate signals, reduce noise, etc. In certain implementations, one side of the band 1208a can feature a loop 1220 and at least one flange 1222 while the other side 1208b can feature a series of holes or notches 1224 to engage with the at least one flange 1222 when notched side 1224 passes through the loop 1220; although in other implementations, a variety of attaching mechanisms can be employed. In some implementations, the loop 1222 can be integrated into the same housing 1232 as the electrodes 902. FIG. 71C depicts a detailed view of one implementation of the device 1200 having its band 1208 secured to form a ring, and FIG. 71D shows a detailed perspective view of the top of the device 1200 illustrating the smooth edges of its body 1206 and band 1208. FIG. 71E depicts an exploded view of the housing 1232, revealing its assembling according to one implementation. The housing 1232 can comprise a first plate 1232a and a second plate 1232b that secure around a support structure 1233 of the band 1208. The first plate 1232a defines openings 1234 for the electrodes (not shown), while both the first plate 1232a and second plate 1232b define an additional opening to form the loop 1222. In various implementations, traces (not shown) connect the electrodes (not shown) of the housing 1232 through or on the exterior of the band 1208. FIG. 71F depicts a cross-section of the body 1206 of the device 1200 through the centerline 1201 of FIG. 71B illustrating its sleek contours.

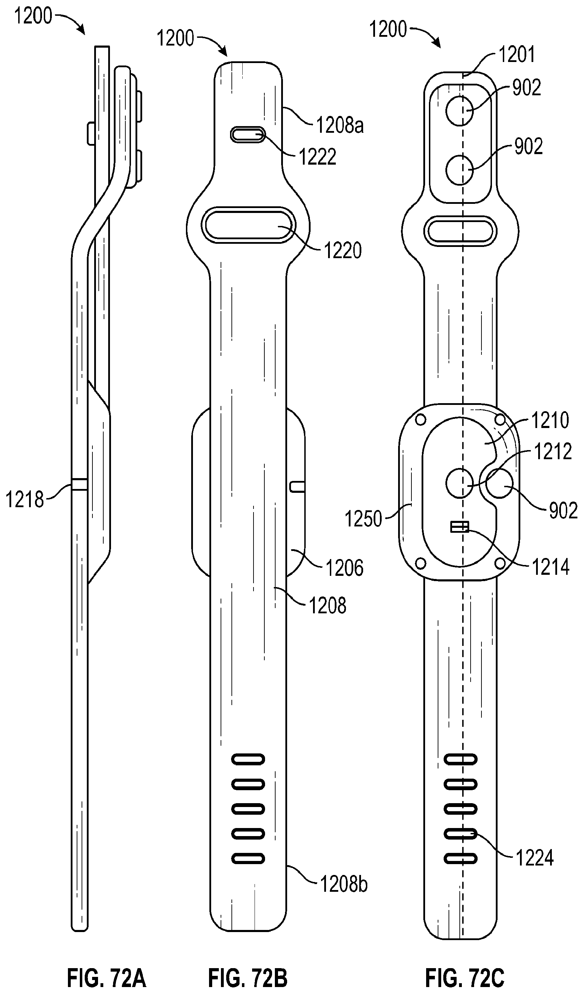

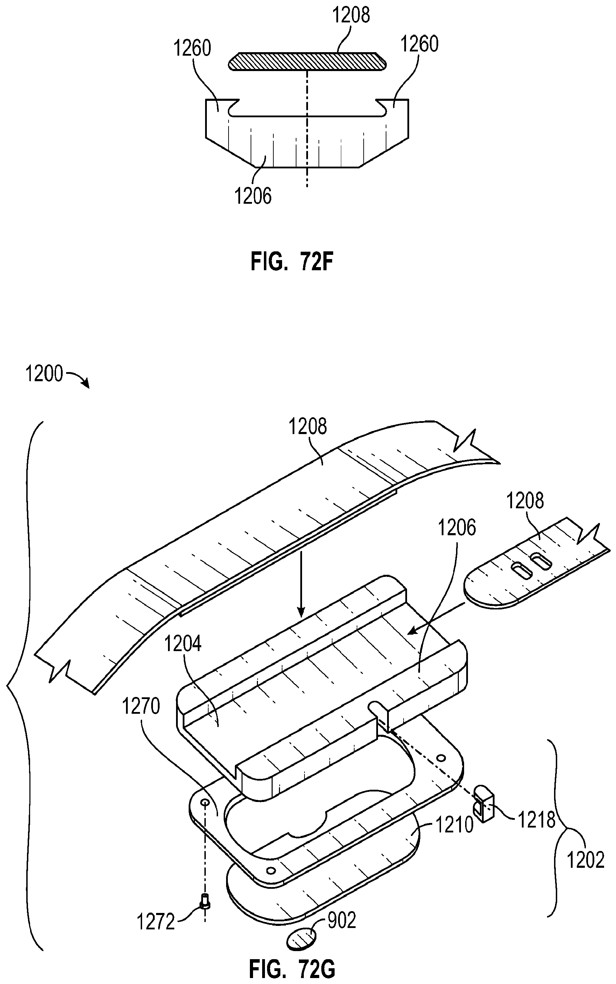

FIGS. 72A-G illustrate yet another implementation of a device 1200 for detecting anomalous biological events. The device 1200 can comprise a symmetric body 1206 and a band 1208. The symmetric body 1206 can be shaped to have low-profile, continuous edges so as to avoid colliding with the back of a user's hand during certain motions of the wrist. In some implementations, the band 1208 can be designed to attach to a first surface 1202 (e.g., as shown in FIG. 72F below) of the body 1206, while a second surface, opposite the first surface that also comprises one or more electrodes is in contact with a skin surface of the user. As seen in the side view of FIG. 72A and the top view of FIG. 72B, the device 1200 can comprise an indicator light 1218. In other implementations, the device 1200 can further comprise an ambient temperature sensor (not shown). As seen in the bottom view of the device 1200 in FIG. 72C, the symmetric body 1206 features a heat source 1210, an optical heart rate sensor 1214, an infrared body temperature sensor 1212, and one electrode 902. In some implementations, the electrode 902 can be a circular electrode offset from a centerline with the infrared body temperature sensor 1214, although other numbers, shapes, and arrangements of electrodes can be employed in other implementations. In order to assist users with the proper fitting of the device 1200, the device 1200 can comprise a hand indicator 1250 (e.g., text reading "left" or "right," text reading "L" or "R", etc.) that indicates on which wrist the device 1200 is to be worn. One side of the coupling member 1208 can comprise two circular electrodes 902 positioned in line and parallel with the length of the coupling member 1208, although different shapes, numbers, and arrangements of electrodes 902 can be used in alternate implementations. In certain implementations, one side of the band 1208a can feature a loop 1220 and at least one flange 1222 while the other side 1208b can feature a series of holes or notches 1224 to engage with the at least one flange 1222 when notched side 1224 passes through the loop 1220; although in other implementations, a variety of attaching mechanisms can be employed. In some implementations, the side of the band 1208a that features the loop 1220 can feature an S-shaped curve (see profile view of FIG. 72A) to facilitate the securing of the band 1208 and improve user comfort.

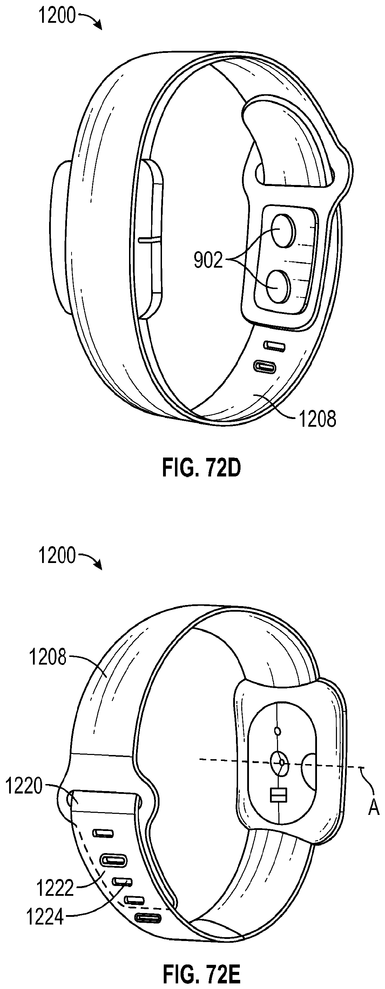

FIGS. 72D and 72E show perspective views of one implementation of the device 1200 when the band 1208 is secured to form a loop. In particular, FIG. 72D illustrates the consequences of a rigid, S-shaped bend perpendicular to a centerline of the band 1208 (e.g., see FIG. 72A), allowing for a more continuous contour of the band 1208 and, therefore, a sturdier positioning of its electrodes 902. FIG. 72E clearly shows the fitment of the flange 1222 through a notch 1224 as the band 1208b passes through the loop 1220.

FIG. 72F depicts an exploded cross-section of one implementation of body 1206 and band 1208 of the device 1200 along line A of FIG. 72E. In some implementations, the band 1208 can slot into a cavity formed in the body 1206 by two opposing edge lips 1260. In some implementations, the edges of the band 1208 can be shaped to undercut the analogous edge lips 1260 such that a top side of the band forms a continuous flat surface with a top side of the edge lips 1260.

FIG. 72G depicts an exploded view of one implementation of the body 1206 and band 1208 of the device 1200. A band 1208 can be slotted into and secured to a first surface 1204 of the body 1206 (e.g., by the mechanism as depicted in FIG. 72F). An indicator light 1218 can slot into the body 1206. A second surface 1204 of the body 1206 can comprise a surface housing 1270, the heat source 1210, and the electrode 902. In some implementations, the heat source 1210 also comprises an optical heart rate sensor (not shown) and an infrared body temperature sensor (not shown). In some implementations, the first surface 1204 and surface housing 1270 can be fastened together with screws 1272, but one of skill in the art will appreciate that various fasteners including, but not limited to, adhesives and snap-fit mechanisms, can be employed without deviating from the scope of the disclosure.

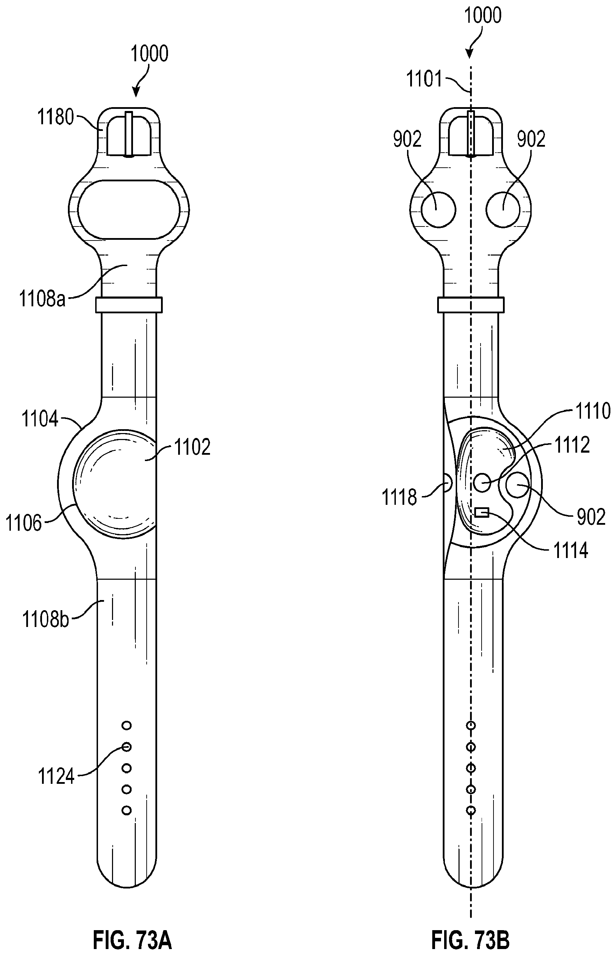

FIGS. 73A-73E illustrate another implementation of a device 1000 for the detection of an anomalous biological event. The device 1000 can comprise an asymmetric body 1106 and a band 1108. The asymmetric body 1106 can be shaped such that a wrist facing edge 1102 has a continuous, sleek contour with the band 1108 while an arm facing edge 1104 can take on a larger shape to compensate for displaced components within. In some implementations, the device can comprise one or both of an ambient temperature sensor (not shown) and an indicator light 1118. In certain implementations, the indicator light 1118 can be positioned near the wrist facing edge 1102. As seen in the bottom view of the device 1000 as shown in FIG. 73B, the asymmetric body 1106 features a heat source 1110, an optical heart rate sensor 1114, an infrared body temperature sensor 1112, and one electrode 902. In some implementations, the electrode 902 can be a circular electrode positioned offset from a centerline of the band 1108; although other shapes, numbers, and arrangements of electrodes 902 can be employed in other implementations. In some implementations, the heating source 1110 can have a kidney shape although alternate shapes can be employed in other implementations. One side of the coupling member 1108 can comprise two electrodes 902 arranged side-by-side and perpendicular to a centerline length of the band 1108; although different shapes, numbers, and arrangements of electrodes 902 can be used in alternate implementations. In some implementations, the electrodes 902 of the band 1108 can be secured with a single metal plate 1130. In certain implementations, one side of the band 1108a can feature a common loop buckle 1180 while the other side 1108b can feature a series of holes or notches 1124 to engage with the buckle 1180, although in other implementations, a variety of attaching mechanisms can be employed.

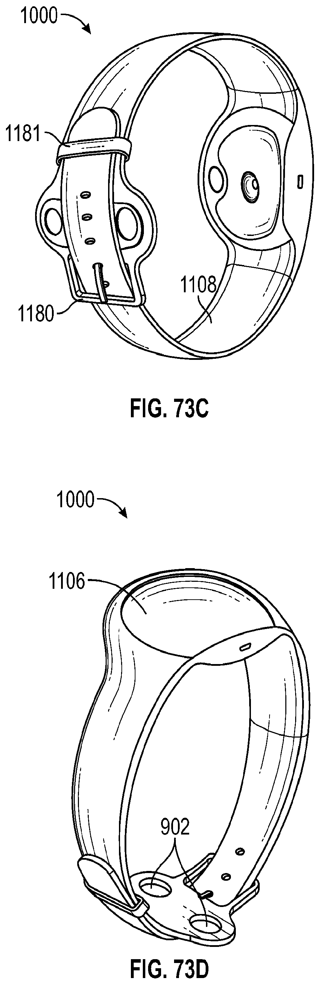

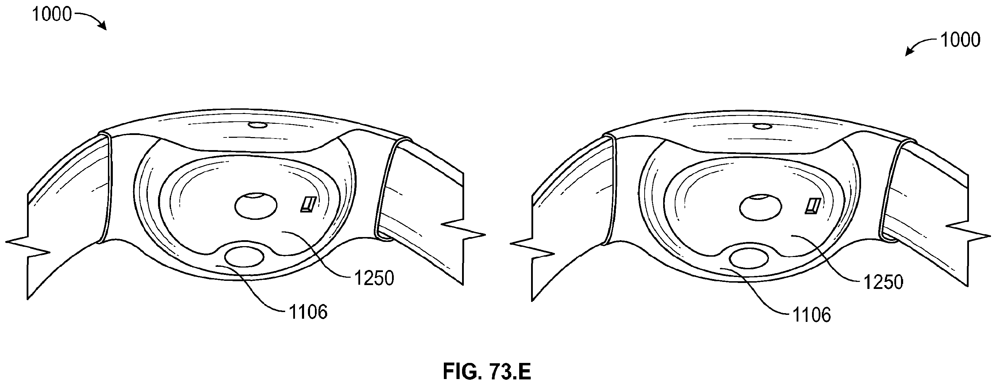

FIGS. 73C and 73D show perspective views of one implementation of the device 1000 when the band 1108 is secured to form a loop. In particular, FIG. 73C illustrates how the band 1108 passes through the buckle 1180 and is secured by a fixed loop 1181. FIG. 73D shows how this arrangement prevents the band 1108 from obstructing its own electrodes 902. For example, an active area of one or more electrodes may be facing and/or interacting with a skin surface of the user, while a backside or inactive area of one or more electrode may be facing a portion of the band, for example where a first end of the band couples to a second end of the band. FIG. 73E illustrates how the body 1106 of the device 1000 can further comprise a hand indicator 1250 (e.g., text reading "left" or "right," text reading "L" or "R", etc.) that indicates on which wrist the device 1000 is to be worn. For example, as shown in FIG. 73E, an asymmetry of a body of a device may indicate which side of the body it should be worn on; left side when an asymmetry of the body faces the arm, may also be described as the proximal side (as opposed to the hand), and right side when an asymmetry of the body faces the hand, may also be described as the distal side (as opposed to the arm).

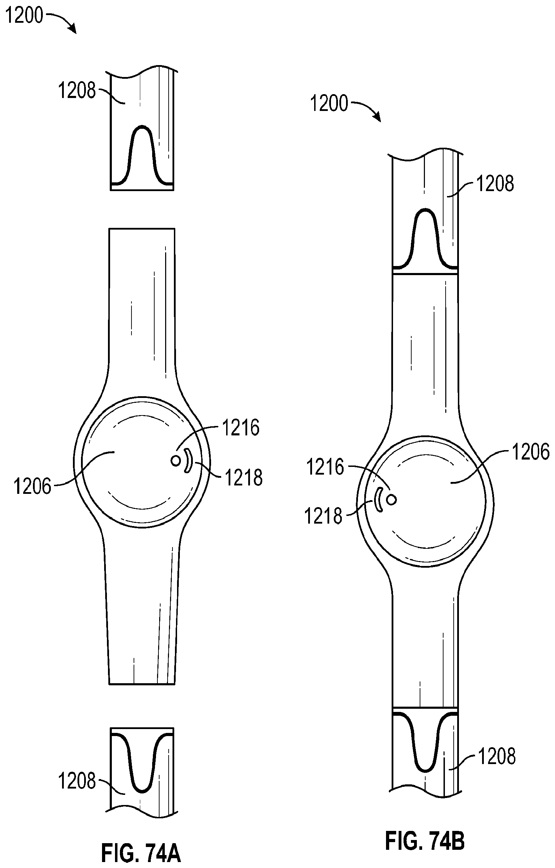

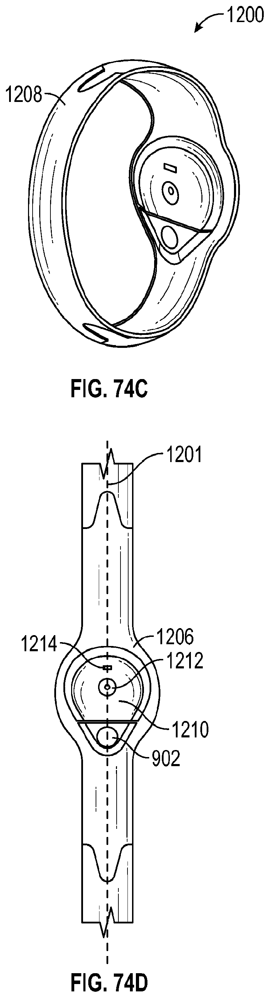

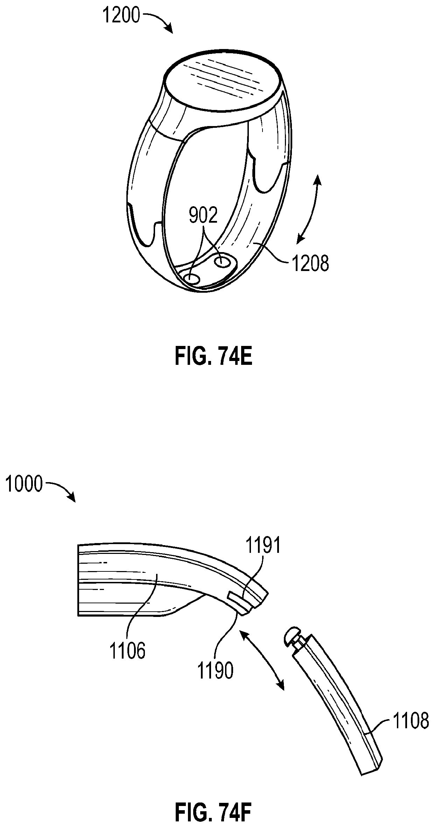

FIGS. 74A-74G depict still another implementation of a device 1200 for detecting anomalous biological events. The device 1200 can comprise a symmetric body 1206 and a band 1208. The symmetric body 1206 can be shaped to have low-profile, continuous edges so as to avoid colliding with the back of a user's hand during certain motions of the wrist. In some implementations, the band 1208 can be a single continuous band (see FIG. 74C and 74D) adapted to detach from the body 1206 of the device 1200, thus allowing for a plurality of bands 1208 having different lengths or sizes to fit to a singular body 1206 design. In many implementations, the band 1108 comprises a stretchable elastomeric material. As seen in the partial top views of FIG. 74A and FIG. 74B, the device 1200 can comprise an indicator light 1218 and an ambient temperature sensor 1216. As seen in the partial bottom view of the device 1200 in FIG. 74D, the symmetric body 1206 features a heat source 1210, an optical heart rate sensor 1214, an infrared body temperature sensor 1212, and one electrode 902. In some implementations, the electrode 902 can be a circular electrode in line with the infrared body temperature sensor 1214 and along a centerline 1201 (length or width) of the band 1208; although other electrode shapes, numbers, and arrangements can be employed in other implementations. FIGS. 74C and 74E depict perspective views of the device 1200 showing the band 1208 as a full continuous band. FIG. 74E depicts that the band 1208 can comprise two electrodes 902 along a centerline 1201 of the band 1208; although other shapes, numbers, and arrangements of electrodes 902 can be employed in alternate implementations.

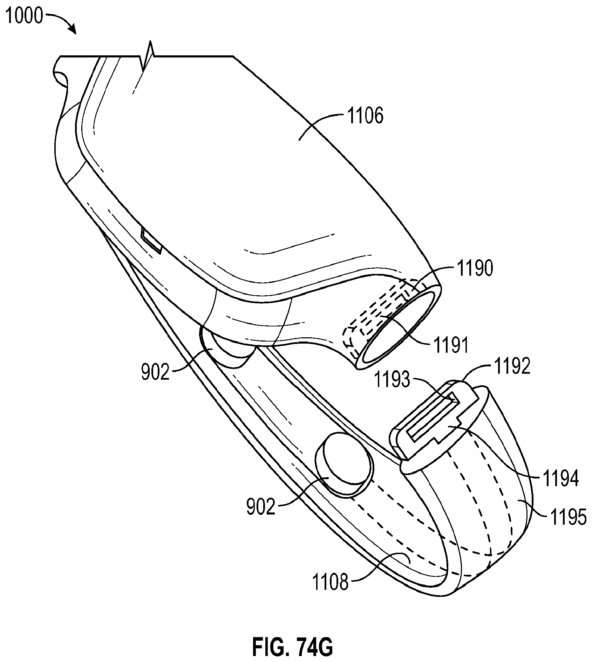

FIGS. 74F-74G depict a profile and perspective view, respectively, of another implementation of the device 1000 having a similar band 1108 to that of FIGS. 74A-74E. In this implementation, the body 1106 can feature at least one slot 1190 to receive a corresponding plug 1192 of the band 1108. In many implementations, the slot 1190 and plug 1192 are adapted such that when the plug 1192 is fully inserted into the slot 1190, mechanical features (for example, a tooth 1191 of the slot and a gap 1193 of the plug) engage to prevent an accidental or unwanted disconnection of the band 1108 from the body 1106. In additional implementations, at least one electrical connector 1194 can be included on the band 1108 in proximity to the plug 1192 such that when the plug 1192 is secured in the slot 1190, the electrical connector 1194 is in electrical communication with a corresponding connector (not shown) of the body 1106 that allows for the transmission of at least one of signal or data from the electrodes 902 of the band 1108 to the systems of the body (not shown). The electrodes 902 can be in electrical communication with the electrical connector 1194 via traces 1195 interior to the band 1108 and represented by the dotted lines in FIG. 74G in this implementation. Other numbers, arrangements, and positions of traces 1195 are possible in different implementations. In still other implementations, the arrangement of the slot 1190 and plug 1192 can be reversed (i.e., the body 1106 can feature a plug 1192 and the band 1108 can feature the slot 1190) without deviating from the scope of this disclosure.

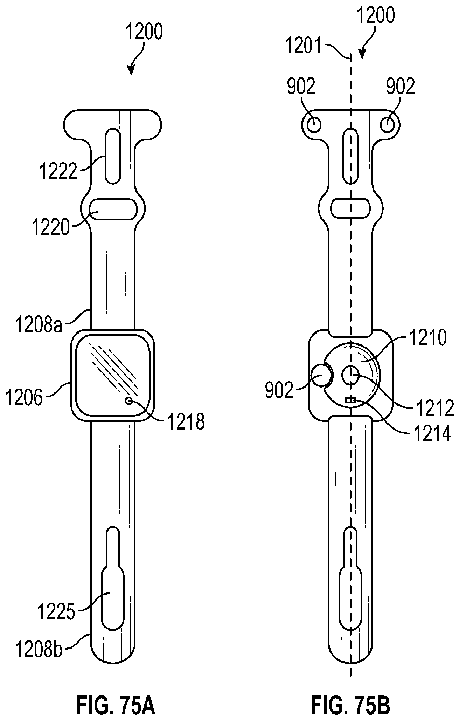

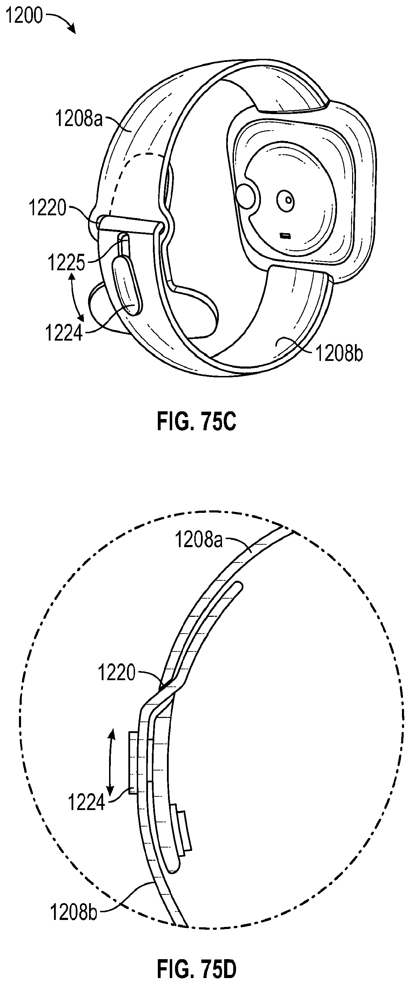

FIGS. 75A-75D illustrate yet another implementation of a device 1200 for detecting anomalous biological events. The device 1200 can comprise a symmetric body 1206 and a band 1208. The symmetric body 1206 can be shaped to have low-profile, continuous edges so as to avoid colliding with the back of a user's hand during certain motions of the wrist. As seen in the top view of FIG. 75A, the device 1200 can comprise an indicator light 1218. In some implementations, the indicator light 1218 can be positioned in a corner of a rectangular body 1206. In other implementations, the device 1200 can further comprise an ambient temperature sensor (not shown). As seen in the bottom view of the device 1200 in FIG. 75B, the symmetric body 1206 features a heat source 1210, an optical heart rate sensor 1214, an infrared body temperature sensor 1212, and one electrode 902. In some implementations, the electrode 902 can be a circular electrode offset from a centerline of the length of the band 1208, although other electrode shapes, numbers, and arrangements can be employed in other implementations. One side of the band 1208 can comprise two electrodes 902 positioned in line and perpendicular to the length of the band 1208, although different shapes, numbers, and arrangements of electrodes 902 can be used in alternate implementations. In some implementations, the side of the band 1208 that features the electrodes 902 can be flared or otherwise have a width that is larger than that of the rest of the band 1208 in order to, for example, afford a greater distance between adjacent electrodes to, for example, decrease interelectrode interference. In certain implementations, one side of the band 1208a can feature a loop 1220 and at least one flange 1222 while the other side 1208b can feature a slot 1225 having at least two portions of differing width that engages the flange 1222 when slotted side 1208b passes through the loop 1220; although in other implementations, a variety of attaching mechanisms can be employed. In some implementations, the side of the band 1208a that features the loop 1220 can feature a rigid, S-shaped bend perpendicular to a centerline of the band 1208 (see profile view of FIG. 75D) to facilitate the securing of the band1208 and positioning of electrodes 902 and to improve user comfort.

FIG. 75C depicts a perspective view of the device 1200 with the band 1208 secured to form a loop. In particular, it shows the flange 1222 fitted into the slot 1225, allowing for an adjustable length of the band 1208 when worn. FIG. 75D depicts a lengthwise cross-section of the band 1208 when secured to form a loop. One side of the band 1208b passes through the loop 1220 of the other side 1208a, and the flange 1222 fits into the slot 1225. In some implementations, slot 1225 enables flange 1222 to slide in slot 1225 to allow the user to fit the band over her hand and onto her wrist. An adjustment of the exact positioning of the flange 1222 within the slot 1225 can adjust the total length of the band 1208 in some implementations. In some implementations, band 1208 has a predetermined length/size; in other implementations, it is trimmable by the user to shorten the band 1208.

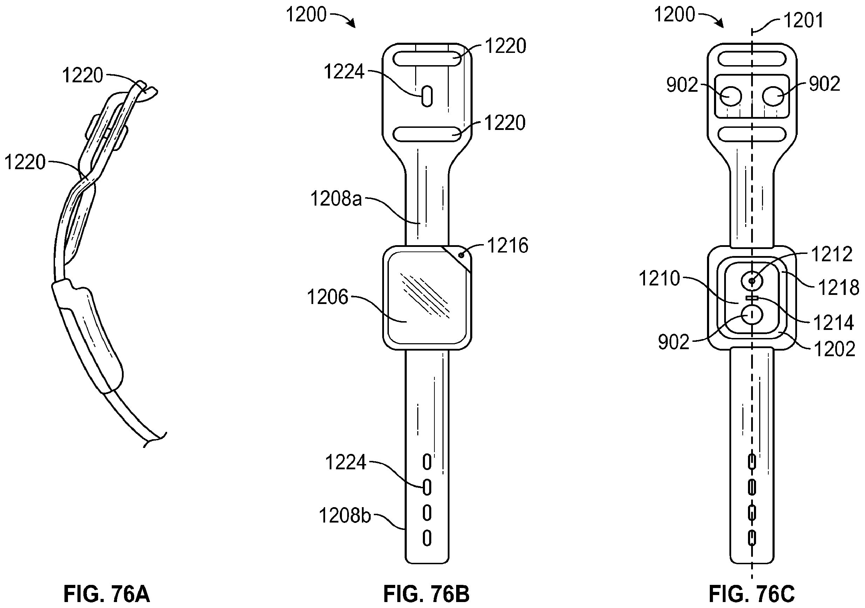

FIG. 76A-76H depict another implementation of a device for the detection of anomalous biological events. The device 1200 can comprise a symmetric body 1206 and a band 1208. The symmetric body 1206 can be shaped to have low-profile, continuous edges so as to avoid colliding with the back of a user's hand during certain motions of the wrist. As seen in the top view of FIG. 76B, the device 1200 can comprise an ambient temperature sensor 1216. In some implementations, the ambient temperature sensor 1216 can be positioned in a corner of a rectangular body 1206. As seen in the bottom view of the device 1200 in FIG. 75B, the symmetric body 1206 features a heat source 1210, an optical heart rate sensor 1214, an infrared body temperature sensor 1212, and one electrode 902. In some implementations, at least a portion of the second surface 1202 of the body 1206 can be at least partially translucent to allow the passage of light from an interior indicator light 1218 (e.g., an LED)

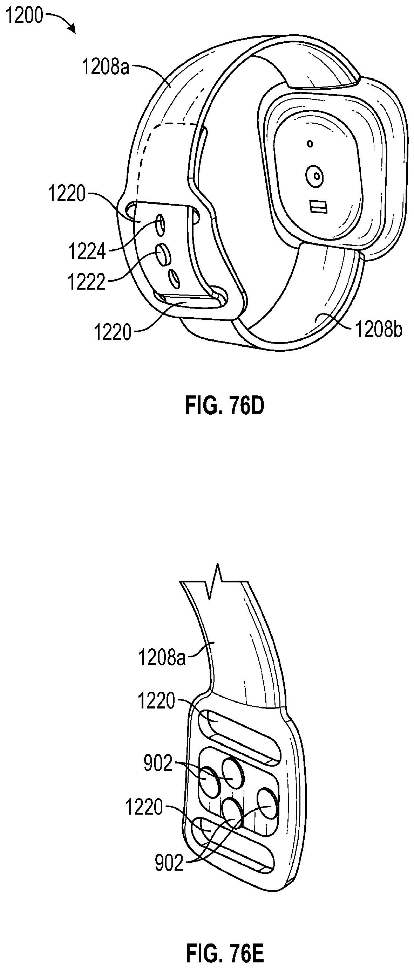

In some implementations, the electrode 902 can be a circular electrode in line with a centerline 1201 length of the band 1208 and surrounded by the heat source 1210; although other electrode shapes, numbers, and arrangements can be employed in other implementations. One side of the coupling member 1208 can comprise two electrodes 902 positioned in line and perpendicular to the length of the coupling member 1208, although different shapes, numbers, and arrangements of electrodes 902 can be used in alternate implementations. In certain implementations, one side of the band 1208a can feature two loops 1220 and at least one flange 1222 while the other side 1208b can feature one or more notches 1224 that engage the flange 1222 when slotted side 1208b passes through the loops 1220 (e.g., FIG. 76A and 76D), although in other implementations, a variety of attaching mechanisms can be employed. In some implementations, the side of the band 1208a that features the loop 1220 can feature an S-shaped curve (see cross-section view of FIG. 76A) to facilitate the securing of the band 1208 and positioning of electrodes 902 and to improve user comfort.

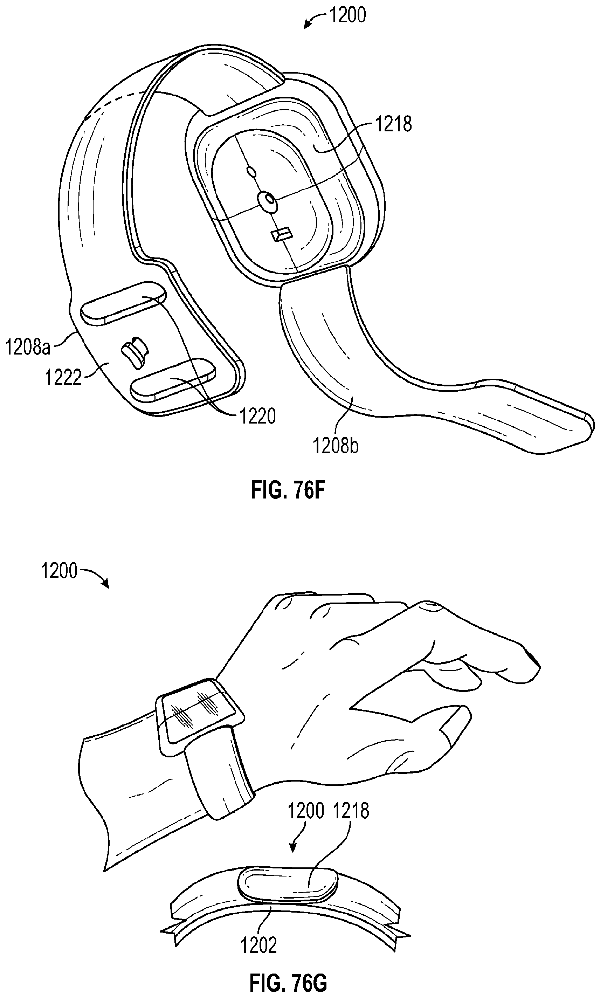

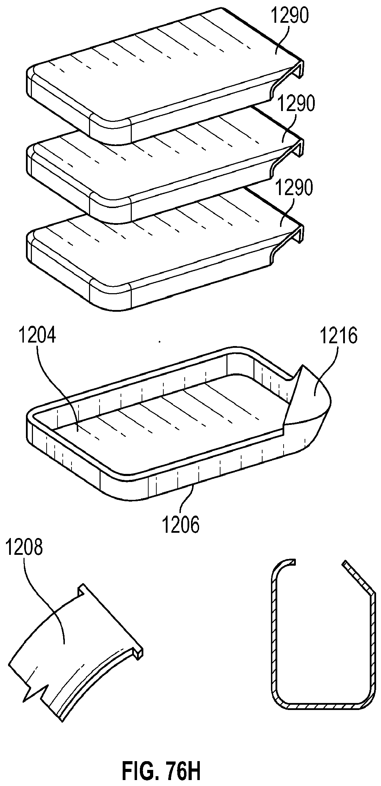

FIG. 76D illustrates a perspective view of one implementation of the device 1200 with the band 1208 secured to form a loop. In particular, FIG. 76D depicts the passage of the band 1208b through the two loops 1220 and the engagement of the flange 1222 with at least one notch 1224 for provide a secure fitment of the band as well as unobstructed positioning of the electrodes (not shown.) FIG. 76E provides a detail view of the end of the band 1208a that features the electrodes 902. In some implementations, a total of four electrodes 902 can be present, positioned between the two loops 1220. FIG. 76F shows a detailed perspective view of the device 1200 with the band 1208 not secured. In particular, the two loops 1220 and flange 1222 are visible in addition to the translucent portion of the second surface 1202 allowing for the transmission of light from an interior indicator light 1218. FIG. 76G depicts two views of the device 1200 being worn. When worn, the position of the interior indicator light 1218 provides subtle illumination around the back of the user's wrist. FIG. 76H depicts an exploded view of the device 1200. The first surface 1204 of the body 1206 can feature an interchangeable face plate 1290 that allows for user customization of style. In many implementations, the face plate features a cutout so that the ambient temperature sensor 1216 positioned in a corner of the body 1206 is not obstructed by the face plate 1290. The band 1208 can feature various materials or textures to modify its grip to a user's wrist as well as to accommodate user style and comfort.

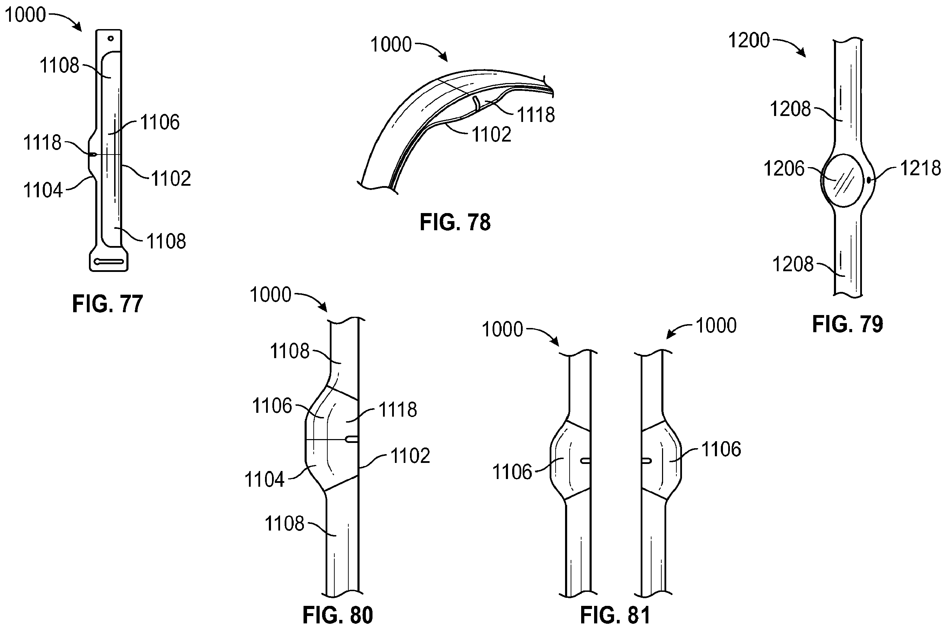

FIGS. 77-81 depict top and perspective views of numerous alternate implementations. FIG. 71 depicts a top view of a device 1000 featuring an asymmetric body 1106 and a band 1108. The asymmetric body 1106 can be shaped such that a wrist facing edge 1102 has a continuous, sleek contour with the band 1108 while an arm facing edge 1104 can take on a larger shape to compensate for displaced components within. In some implementations, the device can comprise one or both of an ambient temperature sensor (not shown) and an indicator light 1118. In certain implementations, the indicator light 1118 can be positioned near the arm facing edge 1104. FIG. 78 shows a detailed view of the implementation of FIG. 77 showing a relative position of the indicator light 1118 on the arm facing edge 1102. FIG. 79 illustrates another implementation of a device 1200 comprising a symmetric body 1206 and a band 1208. The device 1200 can comprise the device can comprise one or both of an ambient temperature sensor (not shown) and an indicator light 1218.

FIG. 80 illustrates an implementation of the device 1000 featuring an asymmetric body 1106 and a band 1108. The asymmetric body 1106 can be shaped such that a wrist facing edge 1102 has a continuous, sleek contour with the band 1108 while an arm facing edge 1104 can take on a larger shape to compensate for displaced components within. In some implementations, the device can comprise one or both of an ambient temperature sensor (not shown) and an indicator light 1118. In certain implementations, the indicator light 1118 can be positioned near the wrist facing edge 1102. FIG. 81 illustrates how an implementation for a device 1000 having an asymmetric body 1106 would be mirrored in order to accommodate the shape and motion of each wrist.

FIG. 82 shows an example diagram of a heating assembly 8200 for installation in the wearable devices described herein. For example, the heating assembly 8200 may be installed to function as heat source 410 in the wearable device 400 shown in FIGS. 67A-67W. The heating assembly 8200 may function to warm or heat over time. If the wearable device 400 is worn by a user (i.e., on a body), the heating assembly 8200 may warm the heat source 410 and directly or indirectly warm the tissue of the user.

As used herein, the term "heater assembly" may refer to a heating circuit, a heating element, a warming element, a warming circuit, a plurality of interconnected layers for heating and/or warming, a heat pad, and/or a heating pad.

The heating assembly 8200 may include any number of layers. The layers may be stacked and aligned to ensure each layer is substantially covered by a next adjacent layer. The combination of layers described herein may include a combination of circuit board materials and/or structures adapted to be thermal control components for heating and/or warming the heating elements (e.g., heat sources) described herein. In some implementations, each layer depicted in FIG. 82 may be composed of several layers or materials within a depicted layer. In some implementations, adhesive backing or adhesive layers may be provided on a first surface of a layer while active componentry or materials are provided on a second surface of the layer.

The heating assembly 8200 may be configured to warm to a target temperature or to an offset temperature and maintain the temperature to a surface of the assembly over a period of time. For example, the assembly 8200 may be configured to ensure temperature uniformity across the heating element and/or across a surface of the heating element. In some implementations, multiple zones (not shown) of the heating element may be configured. Each zone may be configured to maintain a same temperature or temperature offset or a different temperature or temperature offset. The zones may also be configured to ensure temperature uniformity across the zones over time. In some implementations, the zones may be configured to be evenly heated and evenly cooled over time.

As shown, the heating assembly 8200 includes an adhesive backing layer 8202 stacked above a thin film layer 8204. The thin film layer 8204 is stacked above a heater trace layer 8206 which is stacked above another thin film layer 8208. The thin film layer 8208 is stacked above an adhesive layer 8210, which is stacked above a heat spreader layer 8212. The heat spreader layer 8212 is stacked above another thin film layer 8214.

In operation, a person wearing a device 400 with installed assembly 8200 may place the device on the tissue with the layer 8214 nearest the tissue. In such a configuration, the trace layer 8206 may generate heat and the heat spreader layer 8212 may radiate the heat toward the tissue (e.g., skin surface site).

Each layer 8202-8214 may have a similar or identical shape, width, and length. The thickness of each layer 8202-8214 may correspond to the thicknesses described below with respect to layers 8302-8316 when a particular layer of assembly 8200 is composed of the same material as a layer of assembly 8300, for example. Each layer 8202-8214 may provide an opening 8216 for the skin temperature sensor 414 and an opening 8218 for the blood volume sensor 412. For example, each layer may provide an opening substantially sized to allow for sensor measurements to be captured from blood volume sensor 412 and skin temperature sensor 414. Other openings and/or shapes of openings are of course possible. Although seven layers are depicted in assembly 8200, any number of layers may be used to achieve a heater assembly configured to uniformly heat the heating elements (or surfaces of the heating elements) described herein.

The adhesive backing layer 8202 may be configured to adhere to a substrate (e.g., thin film layer 8204) on a first side and to an outer assembly package (not shown) of the wearable device 400, for example. The adhesive backing layer 8202 may be composed of solder, solder paste, and the like that may adhere the layer 8202 to the thin film layer 8204. The thin film layer 8204 may be a film or tape formed of Kapton^{®} or other thin material or polyimide. The layer 8204 may be formed with or without adhesive.

The heater trace layer 8206 may include a plurality of traces that may be interconnected with each other and with one or more other layers of the heating assembly 8200. In some implementations, the heater trace layer 8206 may include a single serpentine trace structure (e.g., wires, circuitry, and the like) formed to substantially cover the surface of layer 8206. The trace structure may be printed, etched, or otherwise formed on layer 8206. In some instances, the heater trace layer 8206 may be formed of a number of serpentine-like structures interconnected across the surface of layer 8206. In some instances, the heater trace layer 8206 may be formed of a combination of serpentine-like structures and arcs or curves near sensor openings. The traces (not shown) of heater trace layer 8206 may be spaced to ensure a particular uniform heating occurs over time across layer 8206, which translates to uniform heating across the heating element installed in wearable device 400, for example. In some implementations, the trace structure is tightly wound to maximize a total length of the traces 8304 for a fixed surface area of the heater trace layer 8206.

As shown in FIG. 82, the heater trace layer 8206 includes at least two solder pads 8220 mounted on a side of layer 8206 having an adhesive. The solder pads 8220 may be used to electrically connect the heater trace layer 8206 to any of the other layers shown in FIG. 82. Although not depicted, other connections and/or solder elements may also be present within the assembly 8200.

The thin film layer 8208 may be a pressure sensitive film or tape formed of Kapton^{®} or other thin material or polyimide. The layer 8208 may be formed with or without adhesive. The layer 8208 may function along with layer 8204 as an insulator to insulate the heater traces in layer 8206.

The adhesive layer 8210 may adhere layer 8208 to the heat spreader layer 8212. The heat spreader layer 8212 may be formed of a thin film composed of metal (e.g., copper (Cu)), copper-alloy, or any material that contains copper as a main component). The layer 8212 may be formed of a solid sheet of metal, grids of metal, traces of metal, and/or other metallic and/or interconnected heat storing structures placed across layer 8212. For example, the heat spreader layer 8212 may be a solid thin film of copper. In some instances, the heat spreader layer 8212 may be an interconnected mesh of traces.

The thin film layer 8214 may be a pressure sensitive film or tape formed of Kapton^{®} or other thin material. The layer 8214 may be formed with or without adhesive. The layer 8214 may function to insulate the heat spreader layer 8214.

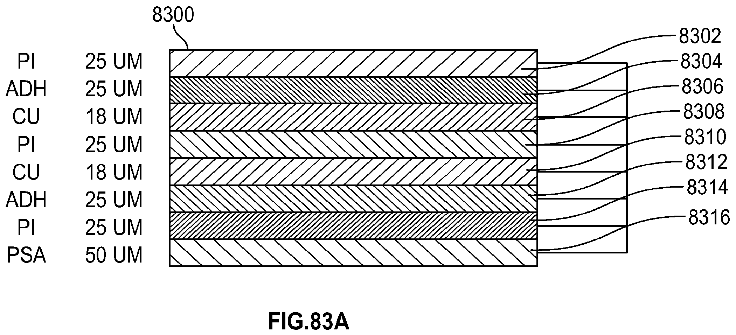

FIG. 83A illustrates a side view of an example of a heating assembly 8300 for installation in the wearable devices described herein. The heating assembly 8300 may be similar to heating assembly 8200. In some implementations, the measurement indications of particular layers of assembly 8300 may also be the same for assembly 8200.

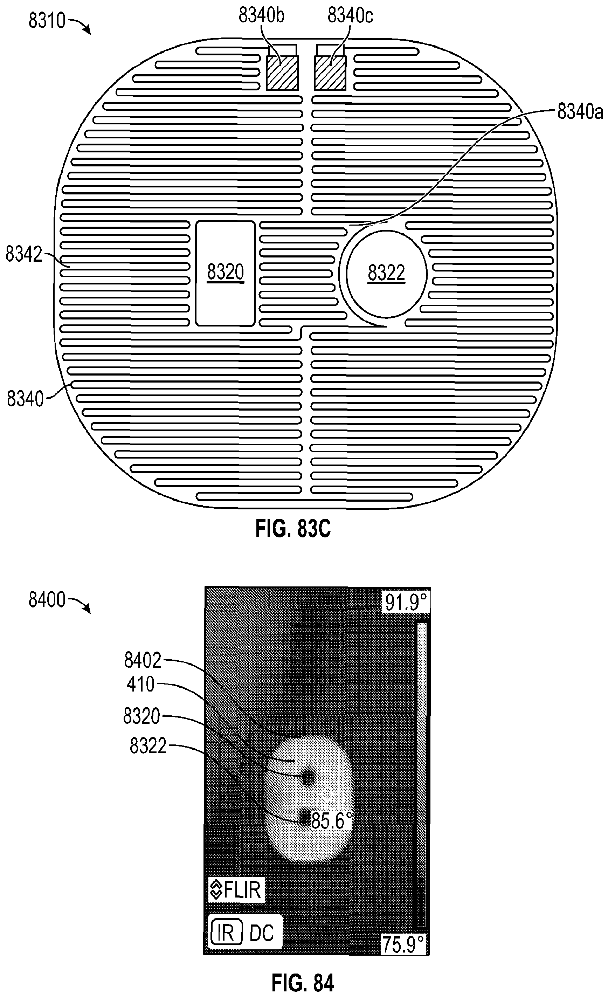

The heating assembly 8300 may include any number of layers. The layers may be stacked and aligned to ensure each layer is substantially covered by a next adjacent layer. The combination of layers described herein may include a combination of circuit board materials and/or structures adapted to be thermal control components for heating and/or warming the heating elements (e.g., heat sources) described herein. In some implementations, each layer depicted in FIG. 83 may be composed of several layers or materials within a depicted layer. In some implementations, adhesive backing or adhesive layers may be provided on a first surface of a layer while active componentry or materials are provided on a second surface of the layer. In some implementations, adhesive layers may be separate from active circuitry layers.

In operation, the heating assembly 8300 may be configured to heat to a target temperature or temperature offset (e.g., from a baseline temperature, until a vasodilation effect is achieved, etc.) and maintain the temperature to a surface of the assembly over a period of time. For example, the assembly 8300 may be configured to ensure temperature uniformity across the heating element and/or across a surface of the heating element. In some implementations, multiple zones (not shown) of the heating element may be configured to heat a surface of the heating element. Each zone may be configured to maintain a same temperature or a different temperature. The zones may also be configured to ensure temperature uniformity across the zones over time. In some implementations, the zones may be configured to be evenly heated and evenly cooled over time.

As shown, the heating assembly 8300 includes a physical insulator layer 8302 (e.g., thin film layer, FR4 layer, Kapton^{®} layer, and the like) stacked above an adhesive layer 8304, which is stacked above a heat spreader layer 8306. The heat spreader layer 8306 is stacked above another physical insulator layer 8308, which is stacked above a heater trace layer 8310. The heater trace layer 8310 is stacked above an adhesive layer 8312, which is stacked above another physical insulator layer 8314. The physical insulator layer 8314 is stacked above a pressure sensitive adhesive layer 8316.

In operation, a person wearing a device 400 with installed assembly 8300 may place the device on the tissue (e.g., to be in contact with a number of skin surface sites under the sensors and/or electrodes). In general, the layer 8302 may be nearest the tissue. In such a configuration, the trace layer 8310 may generate heat and the heat spreader layer 8306 may spread and radiate the heat toward the tissue.

In some implementations, the physical insulator layer 8302 has a film thickness of about 20 micrometers to about 28 micrometers. In some implementations, the physical insulator layer 8302 has a film thickness of about 24 micrometers to about 28 micrometers. In some implementations, the physical insulator layer 8302 has a film thickness of about 25 micrometers.

In some implementations, the adhesive layer 8304 has a film thickness of about 20 micrometers to about 28 micrometers. In some implementations, the adhesive layer 8304 has a film thickness of about 24 micrometers to about 28 micrometers. In some implementations, the adhesive layer 8304 has a film thickness of about 25 micrometers.

In some implementations, the heat spreader layer 8306 has a thickness of about 15 micrometers to about 20 micrometers. In some implementations, the heat spreader layer 8306 has a thickness of about 17 micrometers to about 19 micrometers. In some implementations, the heat spreader layer 8306 has a thickness of about 18 micrometers.

In some implementations, the physical insulator layer 8308 has a film thickness of about 20 micrometers to about 28 micrometers. In some implementations, the physical insulator layer 8308 has a film thickness of about 24 micrometers to about 28 micrometers. In some implementations, the physical insulator layer 8308 has a film thickness of about 25 micrometers.

In some implementations, the heater trace layer 8310 has a thickness of about 15 micrometers to about 20 micrometers. In some implementations, the heater trace layer 8310 has a thickness of about 17 micrometers to about 19 micrometers. In some implementations, the heater trace layer 8310 has a thickness of about 18 micrometers.

In some implementations, the adhesive layer 8312 has a film thickness of about 20 micrometers to about 28 micrometers. In some implementations, the adhesive layer 8312 has a film thickness of about 24 micrometers to about 28 micrometers. In some implementations, the adhesive layer 8312 has a film thickness of about 25 micrometers.

In some implementations, the physical insulator layer 8314 has a film thickness of about 20 micrometers to about 28 micrometers. In some implementations, the physical insulator layer 8314 has a film thickness of about 24 micrometers to about 28 micrometers. In some implementations, the physical insulator layer 8314 has a film thickness of about 25 micrometers.

In some implementations, the pressure sensitive adhesive layer 8316 has a thickness of about 40 micrometers to about 55 micrometers. In some implementations, the pressure sensitive adhesive layer 8316 has a thickness of about 45 micrometers to about 52 micrometers. In some implementations, the pressure sensitive adhesive layer 8316 has a thickness of about 50 micrometers.

Each layer 8302-8316 may have a similar or identical shape, width, and length. Each layer 8302-8316 may provide an aperture 8320 (FIG. 83B) for the skin temperature sensor 414 and an aperture 8322 (FIG. 83B) for the blood volume sensor 412. For example, each layer may provide an opening substantially sized to allow for sensor measurements to be captured from blood volume sensor 412 and skin temperature sensor 414. Other openings and/or shapes of openings are of course possible. Although eight layers are depicted in assembly 8200, any number of layers may be used to achieve a heater assembly configured to uniformly heat the heating elements (or surfaces of the heating elements) described herein.

The physical insulator layer 8302 may be composed of a thin film layer (e.g., polyimide), FR4, Kapton^{®}, or the like). Layer 8302 may be a pressure sensitive film or tape formed of Kapton^{®} or other thin material or polyimide. The layer 8302 may be formed with or without adhesive. The layer 8302 may function along with layer 8304 as an insulator to insulate the heater traces in layer 8306.

The adhesive layer 8304 may be configured to adhere layer 8302 to layer 8306. The adhesive layer 8304 may be composed of solder, solder paste, and the like that may be heated (e.g., via solder flow, soldering iron, and the like) to adhere adjacent layers together entirely or at particular junctions or features on the surfaces of one or more of the adjacent layers.

The heat spreader layer 8306 may be formed of a thin film composed of metal (e.g., copper (Cu)), copper-alloy, or any material that contains copper as a main component). The layer 8304 may be formed of a solid sheet of metal, grids of metal, traces of metal, and/or other metallic and/or interconnected heat storing structures placed across layer 8304. For example, the heat spreader layer 8306 may be a solid thin film of copper. In some instances, the heat spreader layer 8306 may be an interconnected mesh of traces.

The physical insulator layer 8308 may be composed of a thin film layer (e.g., polyimide, FR4, Kapton^{®}, or the like). Layer 8308 may be a pressure sensitive film or tape formed of Kapton^{®} or other thin material or polyimide. The layer 8308 may be formed with or without adhesive.

The heater trace layer 8310 may include a plurality of traces that may be interconnected with each other and with one or more other layers of the heating assembly 8300. The adhesive layer 8312 may be configured to adhere layer 8310 to layer 8314. The adhesive layer 8312 may be composed of solder, solder paste, and the like that may be heated (e.g., via solder flow, soldering iron, and the like) to adhere adjacent layers together entirely or at particular junctions or features on the surfaces of one or more of the adjacent layers.

The pressure sensitive adhesive layer 8316 may be a pressure sensitive film or tape formed of Kapton^{®} or other thin material or polyimide. The layer 8316 may be formed with or without adhesive. Although not depicted, other connections and/or solder elements may also be present within the assembly 8300.

FIG. 83B illustrates the heat spreader layer 8306 of the heating assembly 8300. The heat spreader layer 8306 is configured with a grid 8326 patterned with a plurality of substantially uniformly placed holes (e.g., holes 8328, etc.). The grid 8326 may be spread over the surface of the layer 8306. For example, the holes 8326 may account for about 2 percent to about 15 percent of the surface area of the layer 8306 in a uniformly spaced pattern with a portion relief from the perimeter of the layer 8306. For example, the relief of the grid 8326 around the perimeter may be uniformly spaced to cover about 0.5 to about 2 millimeters. The remainder of the surface area of layer 8306 (i.e., not including the holes 8328) may be composed of metal (e.g., copper).

The holes 8328 may be provided to release thermal energy from layer 8326 to another layer. The holes 8328 may assist in spreading heat on the surface of layer 8306 to further heat or transfer heat to or from other layers of heating assembly 8300. For example, the heat spreader layer 8306 may be a metal or partially metal surface that may be configured to disperse heat efficiently and uniformly across the surface of layer 8306 resulting in efficient and uniform heating of the heating elements and/or heat sources described herein.

The heat spreader layer 8306 may take any form and shape that may be installed within device 400. In this example, the layer 8306 is oblong in shape. However, in other implementations, the layer 8306 may be circular, rectangular, polygonal, square, or other shape configured to fit within device 400 and to maintain a predetermined thermal profile across a surface of assembly 8300.

In some implementations, the length (*l*) of layer 8306 is about 25 millimeters to about 35 millimeters with a radius at each of four corners of about 0.1 micrometers to about 0.4 micrometers. In some implementations, the length (*l*) of layer 8306 is about 28 millimeters to about 32 millimeters with a radius at each of four corners of about 0.1 micrometers to about 0.4 micrometers. In some implementations, the length (*l*) of layer 8306 is about 31.5 millimeters with a radius at each of four corners of about 0.1 micrometers to about 0.4 micrometers.

In some implementations, the width (w) of layer 8306 is about 20 millimeters to about 35 millimeters. In some implementations, the width (w) of layer 8306 is about 25 millimeters to about 32 millimeters. In some implementations, the width (w) of layer 8306 is about 27 millimeters.

An aperture 8320 is configured to receive blood volume sensor 412. Similarly, an aperture 8322 is configured to receive skin temperature sensor 414. The aperture 8320 is circular in shape with a radius of about 10.7 millimeters, in the depicted example. In some implementations, the aperture 8320 has a diameter of about 3.0 millimeters to about 5.5 millimeters. In some implementations, the aperture 8320 has a diameter of about 4.0 millimeters to about 5.5 millimeters. In some implementations, the aperture 8320 has a diameter of about 4.9 millimeters. Other shapes and/or dimensions are possible based on the size and shape of blood volume sensor 412.

The aperture 8322 is rectangular in shape with a radius in each of four corners of about 0.2 millimeters in the depicted example. In some implementations, the aperture 8322 has a width 8330 of about 3.0 millimeters to about 4.5 millimeters. In some implementations, the aperture 8322 has a width 8330 of about 3.5 millimeters to about 4.0 millimeters. In some implementations, the aperture 8322 has a width 8330 of about 3.7 millimeters.

In some implementations, the aperture 8322 has a length 8332 of about 4 millimeters to about 6.5 millimeters. In some implementations, the aperture 8322 has a length 8332 of about 5 millimeters to about 6.1 millimeters. In some implementations, the aperture 8322 has a length 8332 of about 6 millimeters. Other shapes and/or dimensions are possible based on the size and shape of skin temperature sensor 414.

In general, the sensors 8312 and 8314 may be configured for placement within particular devices 400 based on device requirements indicated for proper sensor measurements/recordings. In addition, the placement of sensors 8312 and 8314 within device 400 may be selected to ensure uniform heating occurs on surfaces between the sensors and around each sensor. Thus, the apertures 8320 and 8322 may be manufactured accordingly based on the selected sensor placement. In some implementations, the apertures 8320 and 8322 may be distanced apart at about 6 millimeters to about 8.5 millimeters between a center point of aperture 8320 and a center point of aperture 8322. In some implementations, the apertures 8320 and 8322 may be distanced apart at about 7 millimeters to about 8.1 millimeters between a center point of aperture 8320 and a center point of aperture 8322. In some implementations, the apertures 8320 and 8322 may be distanced apart at about 8.25 millimeters between a center point of aperture 8320 and a center point of aperture 8322.

FIG. 83C illustrates an example heater trace layer or pattern 8310 of the heating assembly 8300. The heater trace layer 8310 may include a plurality of traces that may be interconnected with each other and with one or more other layers of the heating assembly 8300. In some implementations, the heater trace layer 8310 may include a single serpentine trace structure (e.g., wires, circuitry, and the like) that is uninterrupted from trace portion 8340b and trace portion 8340c and is arranged to substantially cover the surface of layer 8310. The trace portions 8340b and 8340c may correspond to electrical terminals. The trace structure may be printed, etched, or otherwise formed on layer 8310. In some instances, the heater trace layer 8310 may be formed of a number of serpentine-like structures interconnected across the surface of layer 8310. In some instances, the heater trace layer 8310 may be formed of a combination of serpentine-like structures and arcs or curves near sensor openings, as shown by example arc portion 8340a. The trace layer 8310 may conform to the shape of apertures 832, which can correspond to openings for receiving sensors.

As shown in FIG. 83C, the heater trace layer 8206 includes at least two solder pads 8220 (shown here as trace portion 8340b and trace portion 8340c) connected in at least one location of trace 8340. The solder pads 8220 may be used to receive one or more connections or components to electrically connect the heater trace layer 8310 to any of the other layers shown in FIG. 83A, for example. Although not depicted, other connections and/or solder elements may also be present within the assembly 8300.

The traces 8340 (including 8340a) of heater trace layer 8310 may be configured with a particular width to ensure particular heating uniformity across layer 8310 and in turn across a surface of a heating element installed in the wearable device 400. In some implementations, the traces 8340 are separated by a first gap 8340d and a second gap 8340e as illustrated. The traces 8340 may be symmetrical across the first gap 8340d and the second gap 8340e. In some implementations, the first gap 8340d and the second gap 8340e splits the heater trace layer 8310 into two approximately symmetrical areas. The traces 8340 may cross an axis corresponding to the first gap 8340d and/or the second gap 8340e in the area between apertures 8320 and 8322. In some implementations, the traces 8340 may have a width of about 0.1 millimeters to about 0.2 millimeters. In some implementations, the traces 8340 may have a width of about 0.13 millimeters to about 0.15 millimeters. In some implementations, the traces 8340 (including 8340a) of heater trace layer 8310 may have a width of about 0.17 millimeters. Because solder trace portions 8340b and 8340c may use a larger area to connect layers, such portions may be larger than traces 8340 and/or 8340a. In some implementations, trace portions 8340b and 8340c may be about 1.5 millimeters in width by about 2.5 millimeters in length. In some implementations, trace portions 8340b and 8340c may be about 1.9 millimeters in width by about 2.2 millimeters in length. In some implementations, trace portions 8340b and 8340c may be about 2.0 millimeters in width by about 2.1 millimeters in length.

The density of traces 8340 (including 8340a, 8340b, and 8340c) placed on heater trace layer 8310 may be spaced to ensure a particular uniform heating across layer 8310, which may in turn, translate to providing uniform heating across a heating element installed in the wearable device 400. In some implementations, the spacing 8342 of traces 8340 depicted in layer 8310 may be about 0.1 millimeters to about 0.3 millimeters. In some implementations, the spacing 8342 of traces 8340 depicted in layer 8310 may be about 0.2 millimeters to about 0.29 millimeters. In some implementations, the spacing 8342 of traces 8340 depicted in layer 8310 may be about 0.237 millimeters.

In some implementations, the traces 8340 may cover a particular surface area of layer 8310. For example, the traces 8340 may be spread across the surface of layer 8310 to cover about 80 percent to about 90 percent of the surface area of layer 8310. In some implementations, the traces 8340 may provide a uniformly spaced serpentine pattern with a portion of relief from the perimeter of the layer 8310. For example, the relief may be uniformly spaced to cover about 0.5 millimeters to about 2 millimeters at the edges of the layer 8310. The remainder of the surface area of layer 8310 (i.e., not including the spaces between traces) may be composed of metal (e.g., copper).

Although not depicted in full for ease of discussion, the layers 8302, 8304, 8308, 8310, 8312, 8314, and 8316 may be substantially the same size and shape of the layer 8306.

FIG. 84 shows a diagram of an example thermal image 8400 captured during warming of a heating element. The thermal image 8400 indicates a temperature distribution of a heating source 410. For example, heating assembly 8200 or heating assembly 8300 may generate heat for a heating source 410, for example. An infrared image may be captured of the heating source 410 before, during, and/or after heating. In this example, the temperature distribution of heat source 410 varies from about 75.9 degrees Fahrenheit to about 91.9 degrees Fahrenheit.

In general, the image 8400 may be captured before, during, or after the heat source 410 is triggered to heat the heat source 410 to a target temperature or temperature offset based on a predefined schedule of sensor measurements. For example, the target temperature may be equal to a baseline skin temperature as measured by the skin temperature sensor 414 plus an offset, for example about 1 to about 20 degrees, about 1 to about 5 degrees, about 2 to about 10 degrees, about 2 to about 15 degrees, about 1 to about 10 degrees, about 5 to about 10 degrees, about 5 to about 15 degrees, about 8 to about 12 degrees, etc. In some implementations, the target temperature is equal to the baseline skin temperature as measured by the skin temperature sensor 414 plus about 5 to about 15 degrees. In some implementations, the target temperature is equal to the baseline skin temperature as measured by the skin temperature sensor plus about 7 to about 13 degrees. In some implementations, the target temperature is equal to the baseline skin temperature as measured by the skin temperature sensor plus about 10 degrees. If the target temperature is greater than a maximum temperature value, the system may pause or delay further heating until the baseline skin temperature drops below a minimum threshold. If the target temperature is less than a maximum temperature sensor, the system may proceed to activate the heat source 410 to heat the skin surface to the target temperature.

As shown in FIG. 84, the image 8400 depicts a thermal representation of heat source 410 with respect to aperture 8320, and aperture 8322. About 90 percent of the surface area of heat source 410 registers at about 91 degrees while the apertures 8320 and 8322 register from about 75 degrees to about 80 degrees. This illustrates the uniformity of the heating across the surface area of heat source 410. A relief edge 8402 of lower temperature is shown around the perimeter of heat source 410.

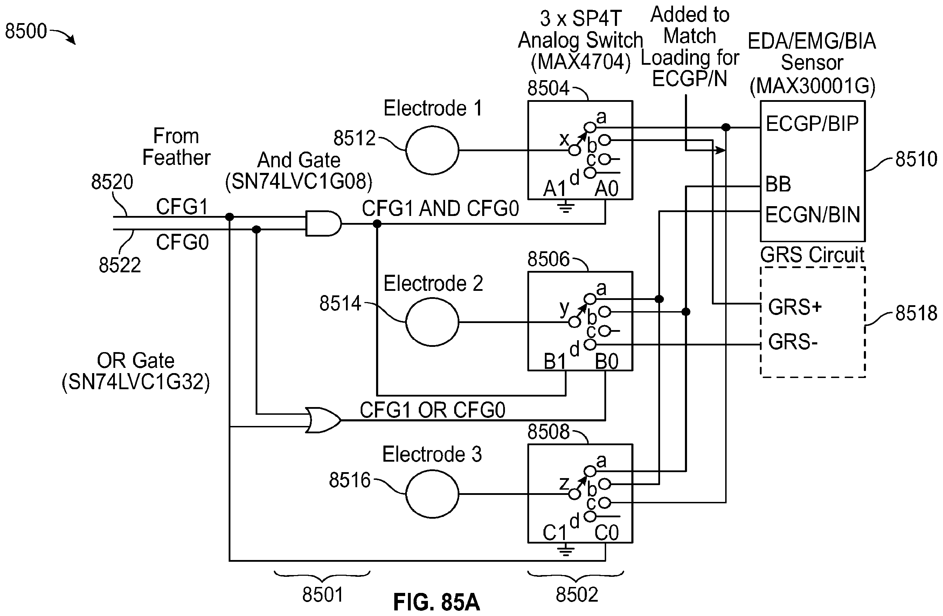

FIGS. 85A-D show an example switching circuit 8500 for use with the sensors, electrodes, and heating elements described herein. The switching circuit may function to ensure that particular sensors are operating in an on state while other sensors are operating in an off state to take advantage of fewer electrodes. That is, to ensure that multiple types of measurements can be performed with the same set of electrodes, the switching circuit 8500 can function to switch between sensor use on the fly to determine biomarkers and other measurements. This switching circuit may be employed to ensure signal accuracy of measuring devices while ensuring particular sensors are made available for intermittent or scheduled measurements (e.g., signal recordings). In some implementations, the switching circuit may be employed to switch on (i.e., connect to) particular sensors at particular time intervals (e.g., for a first measurement/recording at a first time) and switch on (i.e., connect to) different sensors at other time intervals (e.g., for a second measurement/recording at a second time).

The switching circuit 8500 includes at least gating logic 8501 and CMOS switching circuitry composed of a plurality of CMOS devices configured as a multiplexer 8502 (i.e., a MUX). The multiplexer 8502 is configured to select amongst a plurality of switches (e.g., relays), and within each switch, to select connection to at least one of a plurality of contacts a, b, c, and d. For example, a first switch 8504 includes connector (x) with possible contacts a, b, c, and d. Similarly, a second switch 8506 includes connector (y) with possible contacts a, b, c, and d. In addition, a third switch 8508 includes connector (z) with possible contacts a, b, c, and d. Selecting particular contacts a, b, c, or d determines which electrode 8512, 8514, 8516 connects to which sensor 8510, 8518.

The circuit 8500 may be installed in the wearable device 400, for example. The circuit 8500 is configured to signal switch between sensors 8510 and 8518 by triggering a connection from one or more of electrodes 8512, 8514, and 8516. For example purposes, the electrode 8512 may correspond to electrode 902a located nearest the heat source 410. Similarly, for example purposes, the electrode 8514 may correspond to electrode 902b depicted near the buckle 910 and above and parallel to electrode 902c, which in this example, corresponds to electrode 8516.

In some implementations, the circuit 8500 may switch between triggering electrodes 8512, 8514, and 8516 to connect or disconnect to particular traces that may cause sensors 8518 and sensor 8518 to perform measurements/recordings to be taken individually or together in a predefined time sequence. As shown, the switching logic employs multiplexers and CMOS componentry to switch between the use of electrodes 8512, 8514, and 8516 for taking one or more of EDA, EMG, and/or BioZ measurements/recordings.

In some implementations, the circuit 8500 may be used to switch between which sensor is used for particular biomarkers. In some implementations, the switching may be based on a predefined sequence of measurements. In some implementations, the switching may be based on timing of heat cycles. In some implementations, the switching may be based on programmable heat cycles, resting states, and/or heat up, cool down, and/or heat maintenance schedules and/or timing. In some implementations, the switching may be based on electrode availability.

Although three switches 8504, 8506, and 8508 are depicted in FIG. 85A, any number of switches are possible. For example, if additional electrodes and/or sensors are employed in device 400, additional switches may be added to switching circuitry 8500 to switch between any number of electrodes and/or sensors on device 400. The multiplexer 8502 interfaces to sensors 8510 (e.g., blood volume sensor 412 and or skin temperature sensor 414), electrode 8512, electrode 8514, electrode 8516 and galvanic skin response (GSR) sensor, shown here as GSR sensor 8518.

In operation, the switching circuit 8500 may receive a signal 8520 and a signal 8522 (CFG1 and/or CFG0, respectively) from a processor (e.g., processor 409). Depending on the signal information (i.e., logic 0, logic 1) received, the signals may trigger switching of switches 8504-8508 into various states. For each switch, the selected state may trigger operational use (or lack of operational use) of electrode 8512, electrode 8514, and/or electrode 8516 with pins on sensor 8510 and/or pins on GSR sensor 8518.

The operation of the switching circuit 8500 may be determined by a state of the device 400 and algorithms programmed into the processor 409, for example. In some implementations, the logic is programmed to trigger a particular sensor to use one or more of electrode 8512, electrode 8514, and/or electrode 8516. In operation, the signals 8520, 8522 pass through gating circuitry 8501 to begin selection of which contacts a, b, c, or d (of each switch 8504-8508) are to be connected to sensor 8510 and sensor 8518, as described in detail below.

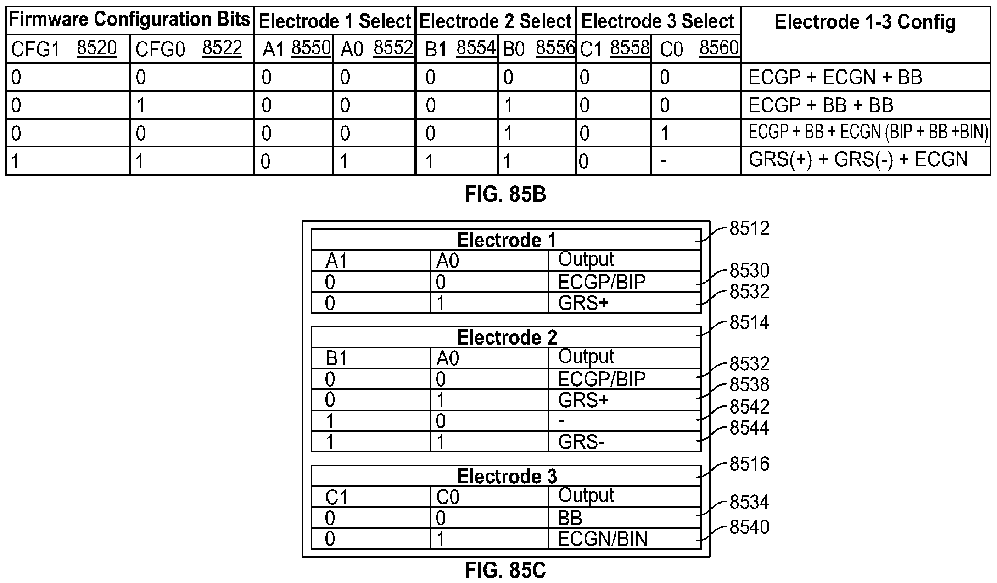

FIG. 85B illustrates example logic signaling used to operate the switching circuit 8500. In a first state of switching circuit 8500, signal 8520 is zero and signal 8522 is zero, which triggers connection of the first electrode to the ECGP pin (a positive ECG signal) of sensor 8510, the second electrode 8514 to the ECGN (a negative ECG signal) of the sensor 8510, and the third electrode 8516 to the BB pin (e.g., a body bias signal) of the sensor 8510. Such a configuration causes the first electrode 8512 to trigger an ECGP measurement (e.g., a positive biopotential recording) and trigger a BIP measurement (e.g., a positive bioimpedance recording), as shown by electrode 8512 in FIG. 85C at output 8530. In the first state of switching circuit 8500, the second electrode 8514 triggers an ECGN measurement (e.g., a positive biopotential recording) and triggers a BIN measurement (e.g., a negative bioimpedance recording), as shown by electrode 8514 in FIG. 85C at output 8532. In the first state of switching circuit 8500, the third electrode 8516 triggers the use of the BB pin to perform a BioZ and/or an EMG measurement/recording, as shown by electrode 8516 in FIG. 85C at output 8534.

In a second state of switching circuit 8500, signal 8520 is zero and signal 8522 is one, which triggers connection of the first electrode 8512 to a positive ECG pin of sensor 8510, the second electrode to a first BB pin of the sensor 8510, and the third electrode 8516 to a second BB pin of sensor 8510. Such a configuration causes the first electrode 8512 to trigger a positive GSR measurement (e.g., a galvanic skin response recording using the BB pin), as shown by electrode 8514 in FIG. 85C at output 8536. In the second state of switching circuit 8500, the second electrode 8514 triggers a BioZ and/or an ECG measurement using the first BB pin, as shown by electrode 8514 in FIG. 85C at output 8538. In the second state of switching circuit 8500, the third electrode 8516 triggers an ECGN measurement (e.g., a positive biopotential recording) and a BIN measurement (e.g., a negative bioimpedance recording), as shown by electrode 8516 in FIG. 85C at output 8540.

In a third state of switching circuit 8500, signal 8520 is one and signal 8522 is zero, which triggers connection of the first electrode 8512 to the positive ECG pin of the sensor 8510, the second electrode 8512 to a first BB pin of the sensor 8510, and the third electrode 8516 to a negative ECG signal (e.g., using the BIP signal, the BB signal, and the BIN signal) of the sensor 8510. However, the third state of switching circuit 8500 does not trigger measurements/recordings to be obtained, as indicated by output 8542.

In a fourth state of switching circuit 8500, signal 8520 is one and signal 8522 is one, which triggers connection of the first electrode 8512 to a positive GSR pin, the second electrode 8514 to the negative GSR pin, and the third electrode 8516 to a negative ECG pin of the sensor 8510. Such a configuration causes the second electrode to trigger performance of a negative GSR measurement/recording, as shown by electrode 8514 in FIG. 85C at output 8544.

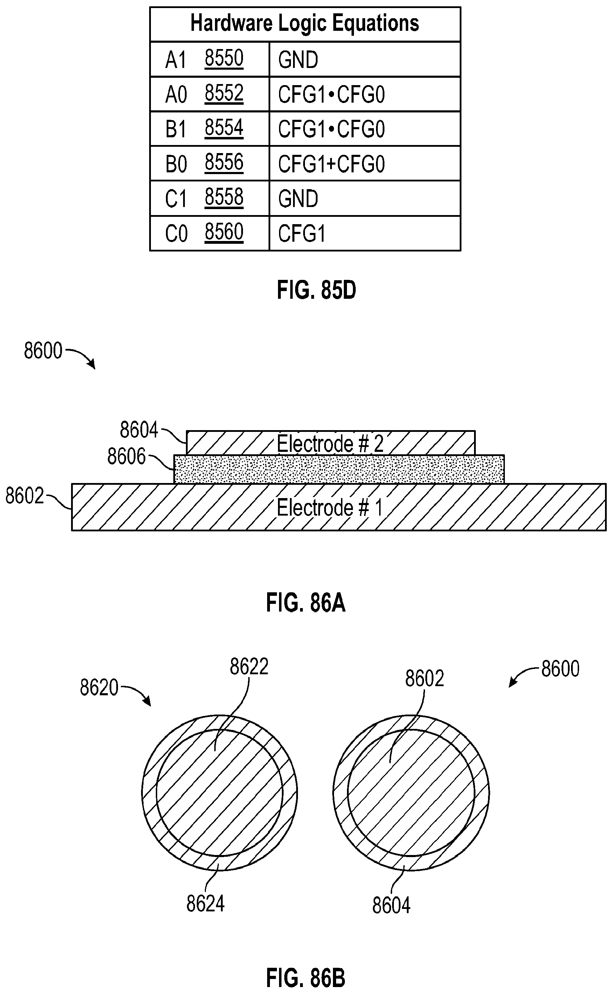

FIG. 85D illustrates the hardware logic equations used in each state of electrodes 8512-8516. As shown in FIGS. 85B and 85D, signal A1 8550 corresponds to a first signal of the first electrode 8512 and signal A0 8552 corresponds to a second signal of the first electrode 8512. Signal B1 8552 corresponds to a first signal of the second electrode 8514 and signal B0 8556 corresponds to a second signal of the second electrode 8514. Signal C1 8558 corresponds to a first signal of the third electrode 8516 and signal C0 8560 corresponds to a second signal of the third electrode 8516.

In some implementations, the switching circuit 8500 may be synchronized between two or more wearable devices 400 to perform synchronous measurements/recordings of any combination of biopotential (e.g., ECG, ECGN, ECGP), EDA (e.g., bioimpedance, BioZ, BIP, BIN, GSR), respiration rate, EMG, and the like. In some implementations, a combination of additional measurements/recordings may be performed using sensor 8510 and/or GSR sensor 8518, as described throughout this disclosure. For example, the switching circuit 8500 may be used to select which sensors are active with which electrodes (e.g., electrode 8512, electrode 8514, electrode 8516, or other electrode) to indicate a particular recording/measurement and the timing of such recordings/measurements. For example, the switching circuit 8500 may be configured to switch automatically in reference to heat cycles performed by heating elements described herein. That is if a heating cycle is underway, the switching circuit 8500 may be programmed to not interrupt the cycle, but instead wait until completion of the cycle to switch to another sensor and measurement task. In some implementations, a heat cycle may be configured with logic to trigger the switching circuit 8500 to perform particular measurements/recordings (e.g., ECG, EDA, BioZ, etc.) at particular times before, during, or after heating cycles.

Because the timing of a heat cycle is configurable and programmable with device 400, the circuit 8500 may ensure that the timing of performing other measurements/recordings is selected to ensure that the heat cycle does not interfere with the other measurements/recordings. For example, an EDA response changes as a result of thermal conditions. Accordingly, device 400 may be configured to decouple EDA measurements from measurements performed using heat source 410 as a stimulus. Decoupling measurements includes scheduling two or more measurements to be performed in different windows of time. The different windows of time may also be scheduled to ensure any cooling of components occurs before a particular measurement is obtained. In some implementations, the device 400 and circuit 8500 may be configured to perform measurements using different electrodes or sensors, or using a different configuration of electrodes and/or sensors to avoid obtaining altered results based on proximity to a heat source or based on proximity to an interfering signal (e.g., of an electrode or sensor).

In some implementations, the devices described herein may be used on coma patients. for example, one or more devices 400 may be used to monitor a coma patient over time. The one or more devices 400 may be used to determine a degree of activity of the comatose individual's vital information, BioZ, EDA, ECG, EKG, and the like. In some implementations, device 400 may be modified with one or more additional sensors and/or electrodes to record additional metrics. Additional metrics that may be obtained by device 100 with one or more additional sensors, processors, and/or electrodes may include EKG, EEG, movements, reflexes, stimulus response output, breathing patterns, metrics responsive to audio input, etc.

FIG. 86A depicts a side view of an example electrode assembly 8600. The electrode assembly 8600 shown here includes a first electrode 8602 and a second electrode 8604 with an insulation layer therebetween. In this example, the first electrode 8602 is larger than the second electrode 8604. In some implementations, the second electrode 8604 is instead larger than the first electrode 8602. In some implementations, the first electrode 8602 and the second electrode 8604 are substantially the same size. In general, each electrode 8602, 8604 may be at least large enough to counteract a contact resistance effect caused by the electrode being in contact with tissue.

In some implementations, the first electrode 8602 represents electrode 902b (e.g., electrode 8514) while the second electrode 8604 represents electrode 902c (e.g., electrode 8516). In some implementations, the first electrode 8602 represents electrode 902a (e.g., electrode 8512) while the second electrode 8604 represents another side of electrode 902a (not shown). In some implementations, the electrode assembly 8600 may replace any single electrode or pair of electrodes described herein. Electrodes may be single sided or dual sided.

FIG. 86B depicts a top-down view of the example electrode assembly 8600 and an additional electrode assembly 8620. The assembly 8600 depicts a full surface of the first electrode 8602 with the second electrode 8604 stacked below. The insulation layer (e.g., insulation layer 8606) may be stacked between the first electrode 8602 and the second electrode 8604.

The electrode assembly 8620 includes a third electrode 8622 stacked above a larger fourth electrode 8624. The third electrode 8622 may be stacked above an insulation layer (not shown) while the fourth electrode 8624 is stacked below the insulation layer (not shown).

In general, the assembly 8600 may replace any single electrode described herein. For example, the assembly 8600 and assembly 8620 may be installed in the band of device 400. In such a configuration, the assembly 8600 may replace electrode 902b and the assembly 8620 may replace electrode 902c. Such a replacement may allow additional electrodes to be used to retrieve additional measurements/recordings of tissue.

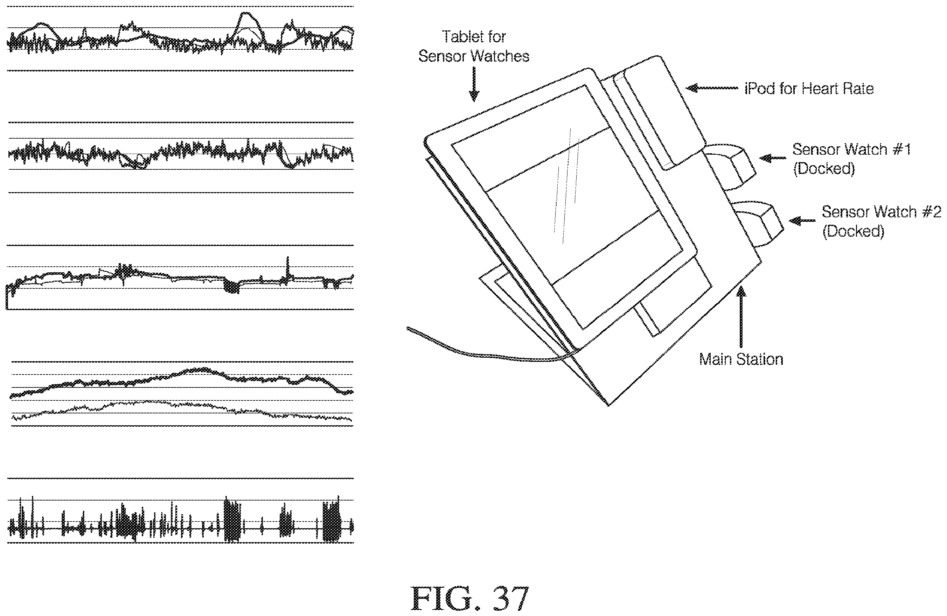

Referring to FIG. 37, a system for detecting stroke may include collect data from one or more sources, for example a contact-based source, a non-contact-based source, and a source that stimulates a response and then measures the response output. As shown in FIG. 37, the system may include a main station or docking station and/or measurement station for one or more measurement devices. For example, a heart rate monitor, devices for measuring asymmetrical responses or effects (e.g., watches worn on each wrist), etc. may be included in the system. The system may be portable such that may be positioned in a mobile stroke detection unit for rapid detection of stroke or positionable in homes of high-risk patients.

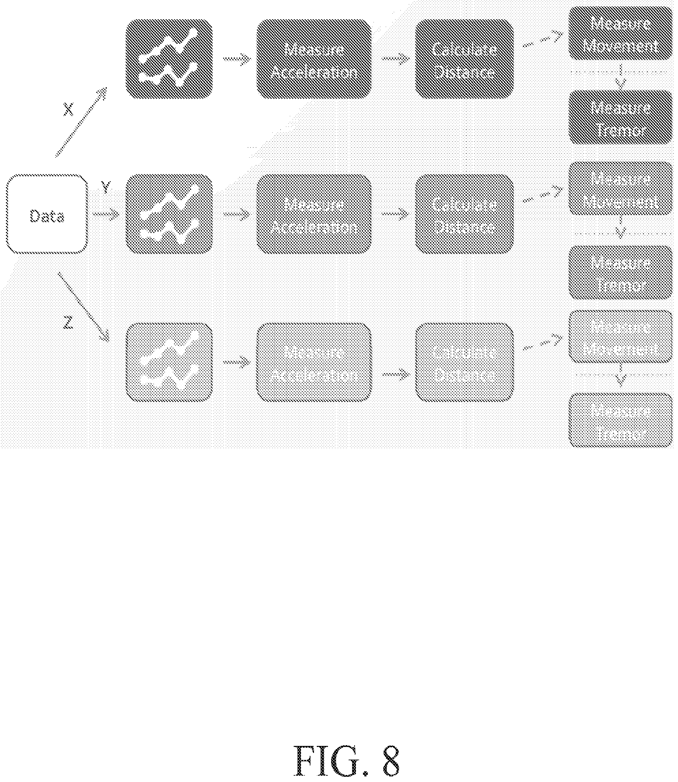

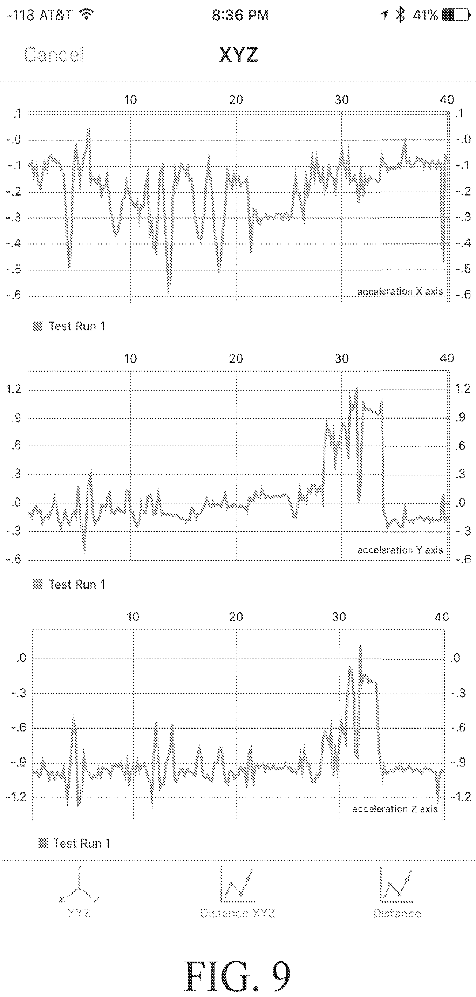

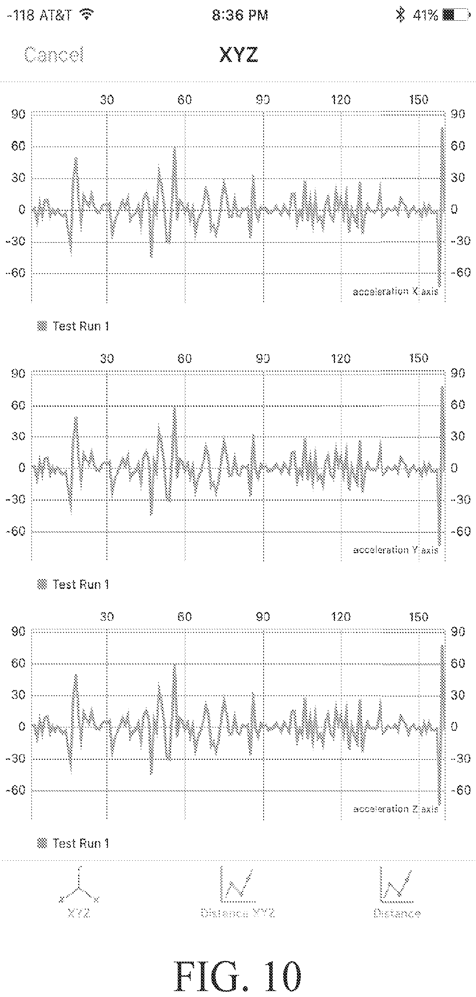

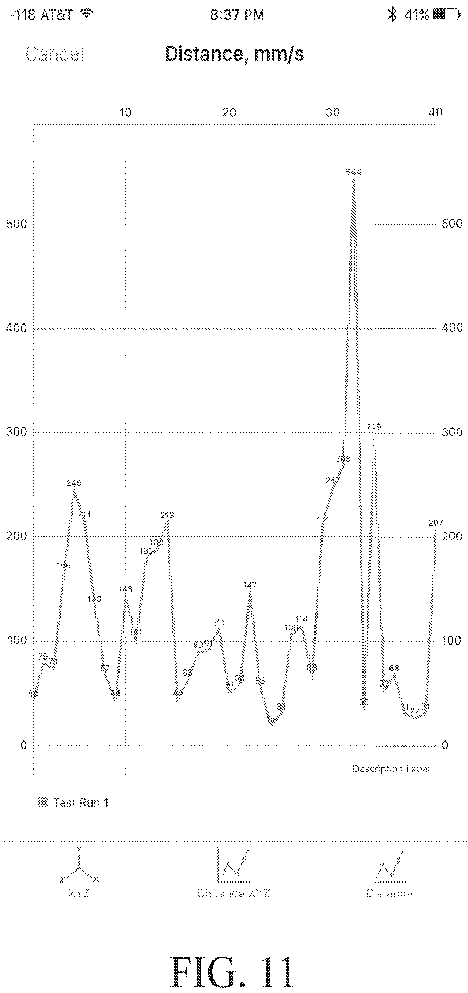

For example, as shown in FIG. 8, a method of detecting tremors (i.e., asymmetrical wrist movement) includes: measuring an acceleration in x, y, and/or z planes of two limbs (e.g., two arms or two legs) of an individual; measuring a distance in x, y, and/or z planes of the two limb of the individual; and calculating a movement of each limb, relative to the other limb, of the individual. In some implementations, symmetrical movement is indicative of healthy, non-stroke movement, and asymmetrical movement is indicative of a tremor or a stroke event. Exemplary acceleration data (XYZ) is shown in FIG. 9; distance data (XYZ) in FIG. 10; and distance (NMS; movement) data in FIG. 11. In some implementations, a specific pattern of time series movements is unique to an individual and classified as a tremor based on data collected over time. For example, tremor data may be collected for a number of hours, including wake cycles and sleep cycles. The statistical modeling of a tremor then becomes a signature for each patient. This signature also allows a baseline to be set for each patient. Again, this baseline behavior may be unique to an individual, and even to the 'awake' and 'sleep cycles' of the individual.

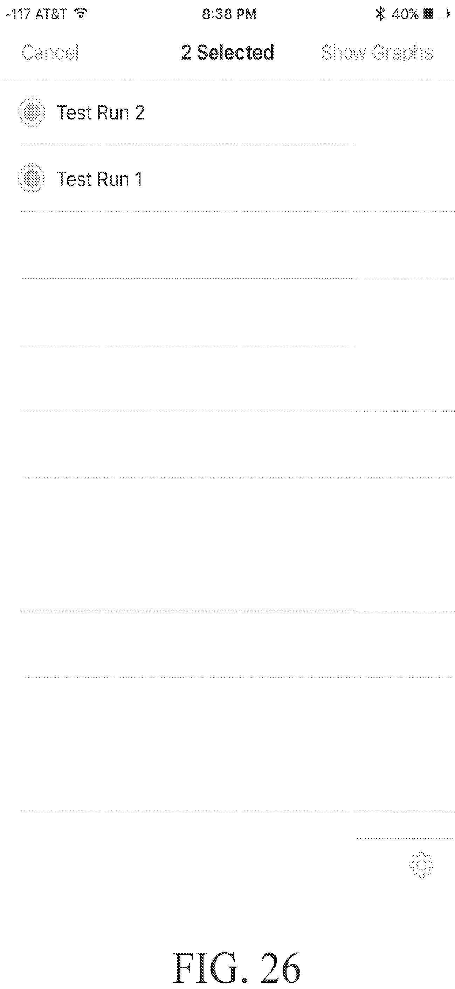

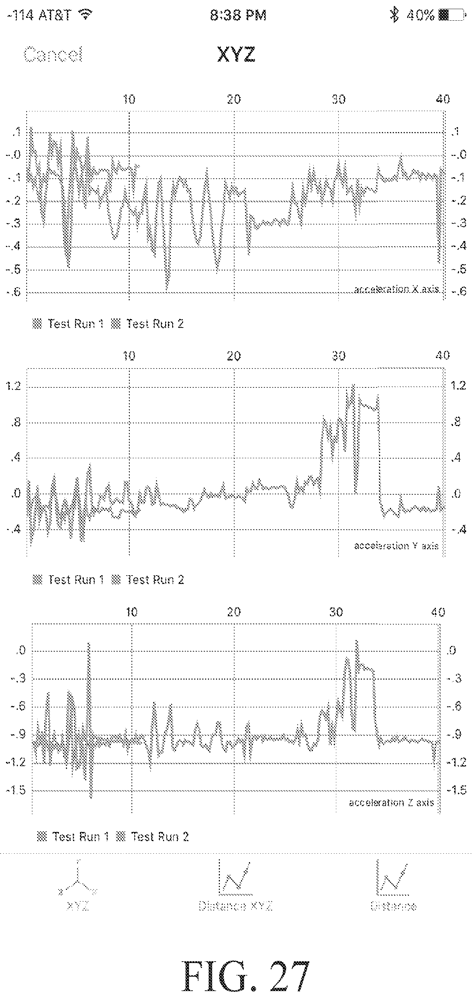

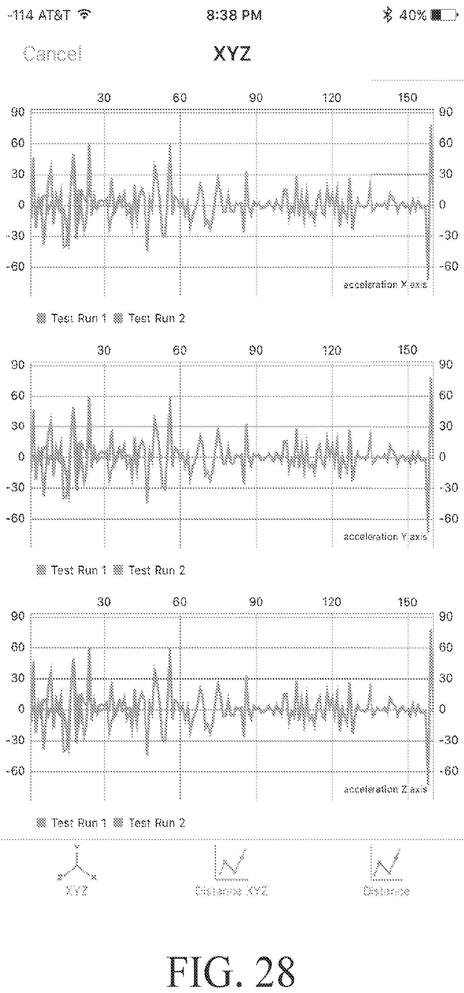

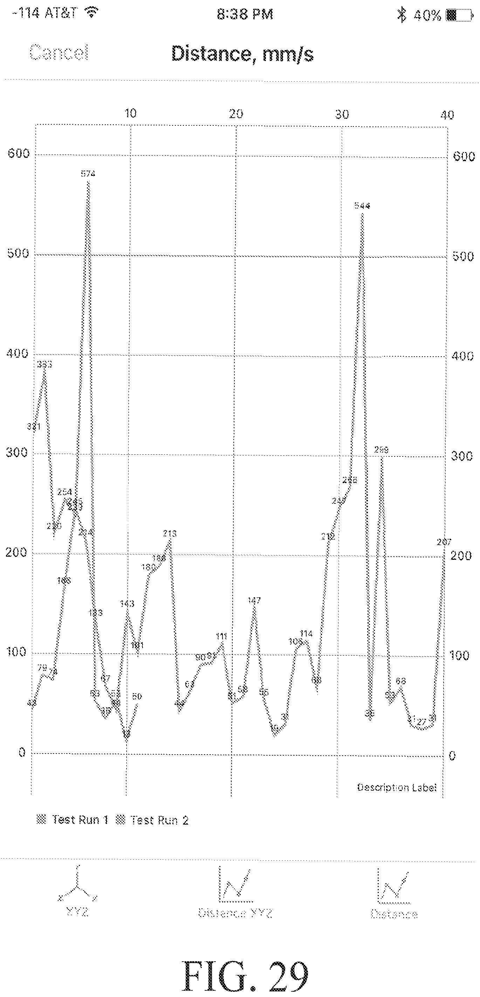

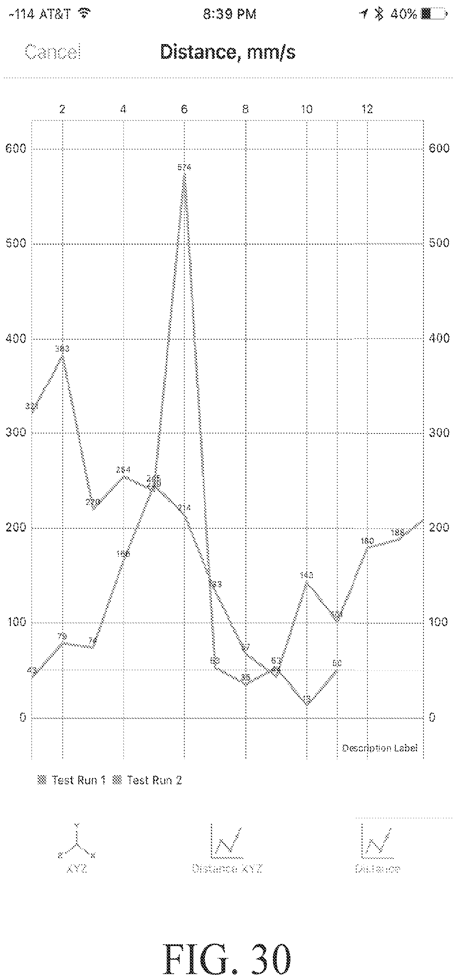

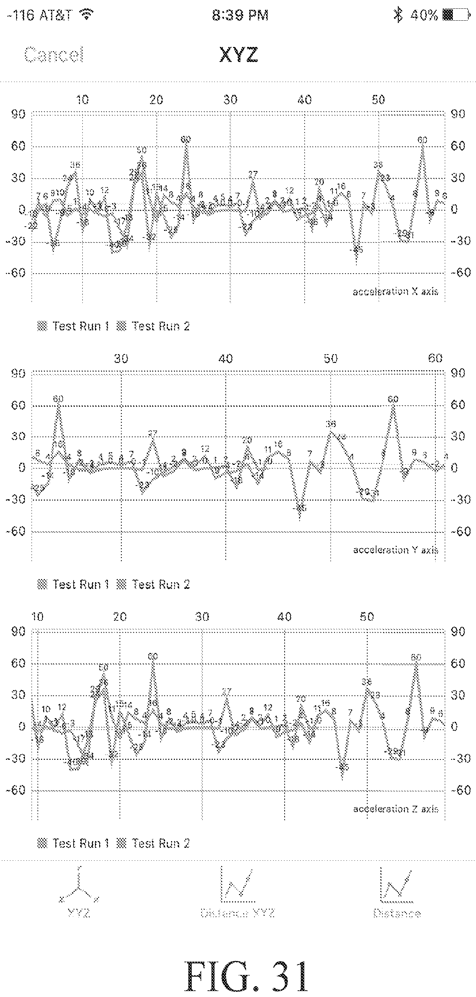

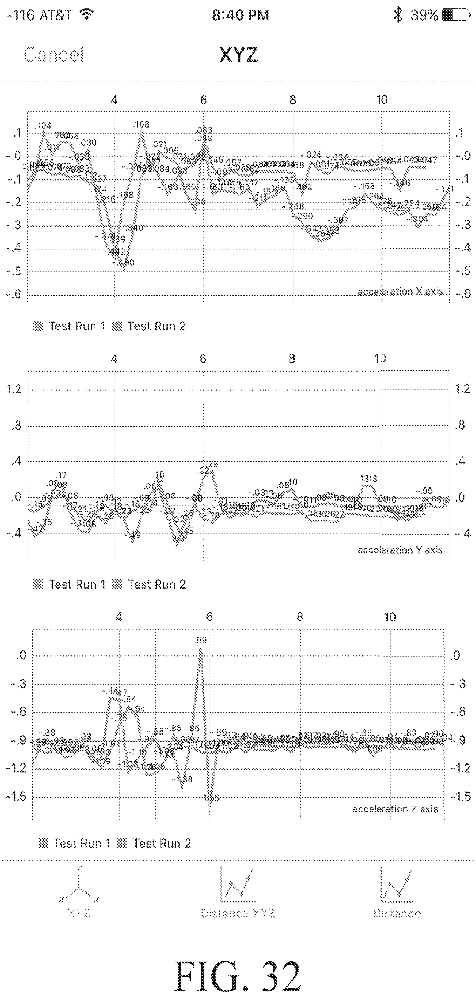

As shown in FIG. 26, an application downloaded and/or stored on a hardware component of a stroke detection system or a computing device collates and analyzes acceleration and distance data sensed by a sensor, for example an accelerometer or gyroscope. The comparison of two data sets (i.e., Test Run 1 and Test Run 2) derived from devices located on the two limbs (e.g., wrists) of the user is shown in FIG. 26. For example, an application on a computing device may be configured to compare two acceleration data sets (FIGS. 27, 32); two distance data sets (FIGS. 28, 31); and two movement data sets (FIGS. 29, 30) from devices positioned on two wrists of a user. As shown in FIGS. 29-30, an application on a computing device may further include a zoom feature, for example, for viewing a subset of the total data collected during a period of time (e.g., overnight, during a tremor instance, etc.).

In some implementations of a device for detecting tremors or asymmetrical motion, the device may include a feedback mechanism (e.g., visual, haptic, or audio) when a threshold has been reached or surpassed or various comparison criteria have been met, for example when a current movement pattern matches a previously identified tremor pattern for the individual. In some implementations, a mobile computing device communicatively coupled to a movement sensor or wearable device generates a vibration signal in the wearable device, sensor, and/or computing device if the comparison between the two signals exceeds a predefined threshold.

To determine which implementations would be best for stroke detection, several factors may be considered: alert 911 capability; passive monitoring; detection when patient is alone; and detection when patient is sleeping. Additional factors may include, but not be limited to: fully mobile; patient specific algorithm; active patient engagement after a passive alert; detection for the cognitively impaired patient; detection for prior stroke patient; detection of all strokes including posterior; diagnose type of stroke; passive monitor that wakes the patient up; and commence stroke treatment. For example, if a possible stroke event is detected, a wearable system may initiate a tactile, auditory, and/or visual alert to determine whether the user is conscious, unconscious, experiencing other stroke symptoms, etc. If the patient does not respond in a predetermined time window, a caregiver, emergency services, physician, etc. may be alerted to the stroke event. The wearable system can be linked to a clinician computing system. The alert can be transmitted directly to the clinician computing system that may prompt a telemedicine assessments. The clinician may work up an NIH Stroke Score assessment in response to the alert and/or data received from the wearable system. In some instances, the wearable system can by itself or in conjunction with a personal computing system enable self-assessment by walking the person and/or available witnesses through a FAST (Facial drooping, Arm weakness, Speech difficulties and Time) assessment.

In some instances, the wearable system can transmit a signal to the user's home automation system or to at least one electronically enabled door lock to unlock at least one door and / or disable the user's home alarm system in response to an alert for the stroke event. The wearable system can also initiate transmission of a floor plan access pathway leading from an access point of entry to the location of the patient, in the home or facility where the user has had indicium of a potential stroke. The location of the patient can be determined based on a local area network or differential GPS. In some implementations, a stroke detection device or system may trigger an audible alarm to alert a patient or caretaker, for example while sleeping, that a stroke event has occurred. The audible alarm can also enable emergency services to locate patient when they enter home. All of these measures can help to reduce the time it takes for the emergency services or caregivers to reach the patient.

The home automation system can also include smart displays and smart speakers. These smart displays and speakers can be used to convey information to emergency medical response personnel, such as the identification of which medications the patient should be taking and, if available, information about whether they are compliant with prescribed regimens. Information such as the identity of physicians, medical history, allergies, and the existence of medical care power of attorney or advance directives associated with the patient may also be conveyed.

Furthermore, when alerting emergency services or physicians, data including medical history may be transmitted directly to emergency services or physician computing systems, either directly from the wearable system or from a remote memory, initiated by a signal from the wearable system. In addition to alerts, the wearable system can also instruct a user to undertake or automatically activate certain stroke treatments. Stroke treatments can include inducing hypothermia to provide a neuro-protectant for the patient. The wearable system can trigger inhalation of cooling gases, activation of a cooling helmet, activation of an ultrasonic helmet to break up cloths, or ingestion or triggering administration of a drug patch or pill. The trigger can be instructions to the patient or medical responder, or automatic activation. In some instances, for Ischemic strokes, the wearable system can trigger mechanisms to increasing blood pressure and vasodilate blood vessels (through some of the mechanisms discussed above).

Treatments responsive to the detection of a potential stroke can be initiated by the patient if they are conscious and able, or by the medical response personnel via the home automation system. Patients in a particular high risk category may have previously been fitted with a wearable treatment device which can be activated automatically in response to a signal indicating the detection of a potential stroke, or activated by medical personnel following clinical examination which was initiated by an alert from the wearable system.

In some implementations, a stroke detection device or system may trigger an audible alarm to alert a patient or caretaker, for example while sleeping, that a stroke event has occurred. The audible alarm can also enable emergency services to locate patient when they enter home.

In any of the implementations described herein, a stroke detection device or system may record an onset of a stroke event and/or provide a "last known well" indicator to help inform treatment decisions.

In some implementations, a system for detecting stroke includes a data processing module. The data processing module may be configured to extract a pattern. The pattern may suggest any ischemic or hemorrhagic episode very early, possibly imminently prior to an actual stroke event. In some implementations, the pattern may be empirically determined, for example based on a population wide analysis, cohort analysis, and/or individual analysis of signals, which are analyzed for parameters and/or patterns indicative of stroke onset. In some implementations, signal processing may employ signal processing tools, for example filtering, extracting, digitizing, data de-convolution, machine learning, and/or other methods known in the art. Specifically, the signal processing may use higher order statistics to ascertain hidden patterns in data. Use of higher order statistics, known as cumulants, and their Fourier spectra, often termed poly spectra, not only reveal the amplitude information in the higher order (such as those carried by power spectra or auto correlation) but may also include phase information. Phase information can reveal salient features of the data, otherwise unattainable from simple harmonic analysis. Another important feature of the polyspectra is the fact that they are blind to Gaussian processes. As a result, they can automatically handle Gaussians processes and thus improve signal to noise ratio, allowing novel detection. In some implementations, a number of spectrums and their manipulations may be selected in order to identify hidden patterns in the sensed signals, for example BP(t), ECG(t) etc.

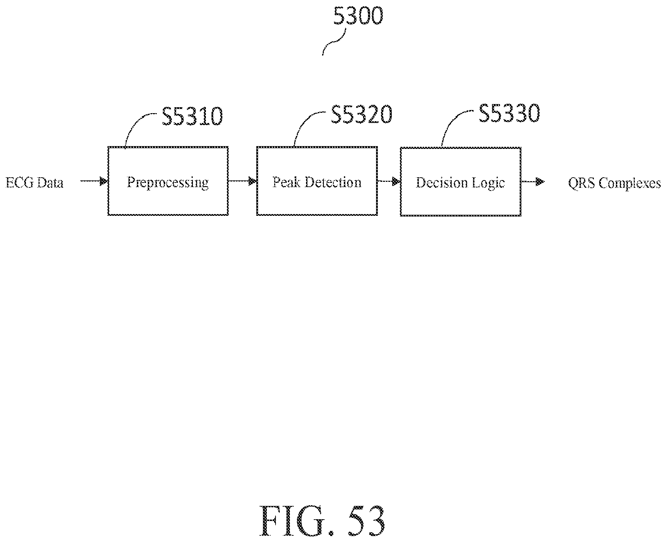

For example, as shown in FIGS. 53-55, a wearable system may collect electrocardiogram (ECG) data, pre-process the data, identify peaks in the data, and apply a decision logic to the data. FIG. 54 shows electrocardiogram data collected over time. FIG. 55 shows extracted R-R intervals from the electrocardiogram data (i.e., time between beats shown in milliseconds). The method 5300 shown in FIG. 53 may be used to calculate a heartbeat and/or a heart rate variability (i.e., specific changes in time between successive heart beats) of an individual. As shown in FIG. 53, ECG data is input into the method 5300, which detects QRS complexes (i.e., ventricular depolarization and the main spike in an ECG signal) in electrocardiographic signals. Preprocessing at block S5310 includes apply signal processing techniques for QRS feature extraction. For example, preprocessing may be applied to reduce the influence of muscle noise, powerline interference, baseline wander, and/or T-wave interference. Peak Detection at block S5320 includes QRS peak detection with adaptive threshold, for example. Each potential peak is compared to a baseline value. A baseline skin temperature is established by measuring unstimulated skin for a period of time. Once the baseline is determined, the stimulus (e.g., application of heat) can either reach a time limit or a temperature limit. The temperature limit can be absolute or relative to the baseline skin temperature. The baseline value is updated according to the amplitude of the detected peak. Decision Logic at block S5330 classifies the current peak as QRS, T-wave, or error beat, using the peak slope and/or peak-to-peak interval.

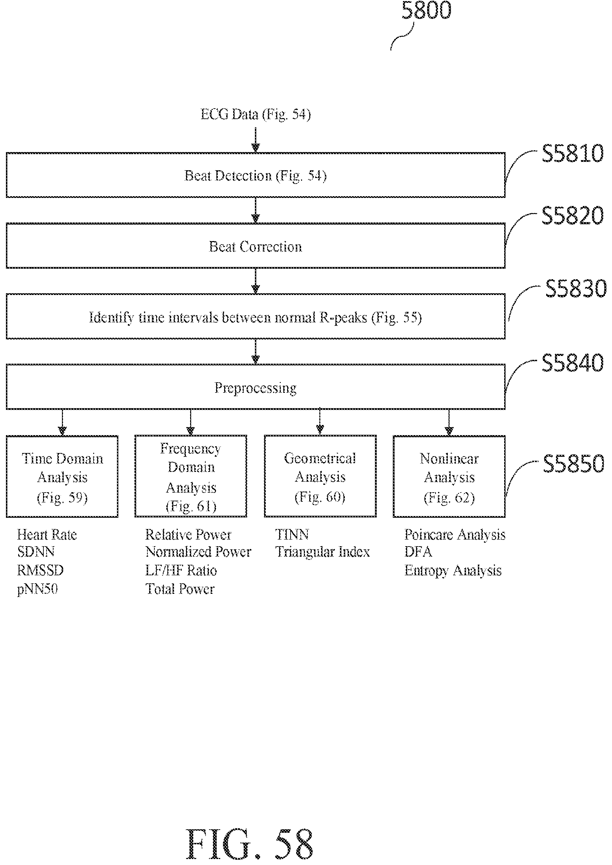

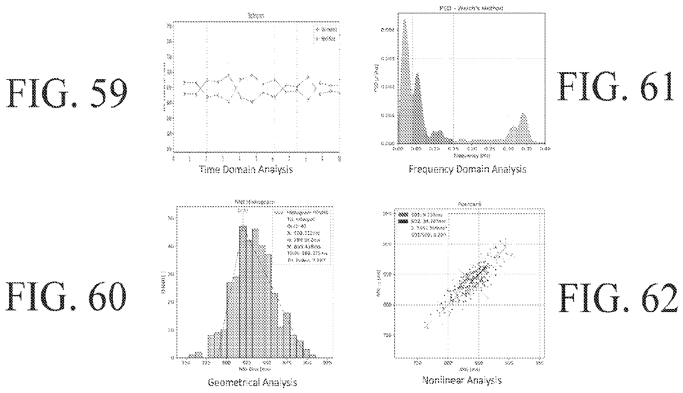

As shown in FIGS. 58-62, electrocardiogram data may be processed via several methods to extract various features, calculate one or more features (e.g., heart rate variability, heart rate, total power, etc.), etc. For example, a time domain analysis (FIG. 58), a geometrical analysis (FIG. 59), a frequency domain analysis (FIG. 60), and/or a nonlinear analysis (FIG. 61) analysis may be used.

As shown in FIG. 58, ECG data (e.g., FIG. 54) is fed into method 5800. The method includes: receiving ECG data of a user using an ECG; detecting beats in the ECG data (e.g., detect R-peaks in the ECG data) S5810; identifying and correcting irregular beats (e.g., missed, extra, and ectopic beats; uses neighboring beats to correct each beat) S5820; identifying intervals between normal R-peaks (i.e., NN Interval Time Series (NNIs) S5830; preprocessing the data (e.g., corrects outliers of NNIs) S5840; and performing one or more analyses S5850. For example, a time domain analysis, as shown in FIG. 59 may be used to calculate heart rate (e.g., 60 divided by the mean of NNIs); the standard deviation of NNIs (SDNN); the root mean square of successive differences (RMSSD); and the percentage of adjacent NNIs that differ from each other by more than 50 ms (pNN50). Further, for example, a frequency domain analysis, as shown in FIG. 61, may be used to calculate a relative power (e.g., relative power of each frequency band (VLF/Total, LF/Total, HF/Total)); a normalized power (e.g., normalized powers of the LF and HF frequency bands (LF/(LF+HF), HF/(LF+HF)); an LF/HF Ratio (e.g., LF power / HF power); and/or a total power (e.g., total power over all frequency bands). Further, for example, a geometrical analysis, as shown in FIG. 60, may be used to calculate a baseline width of the interpolated triangle (TINN); and/or the ratio between the total number of NNI and the maximum of the NNI histogram distribution (i.e., triangular index). Further, for example, as shown in FIG. 62, a nonlinear analysis may be used to perform a Poincare Analysis (i.e., analyze Poincare plot of NNIs - SD1, SD2, SD Ratio, Ellipse Area); a DFA (Detrended Fluctuation Analysis (i.e., short and long-term fluctuations of NNIs); and/or an Entropy Analysis (i.e., computes approximate entropy, sample entropy, and fuzzy entropy of NNIs).

In some implementations, the data processing module may use the continuously monitored or intermittently monitored physiological signals to differentiate changes from healthy "learned" or individualized baseline data. For example, the module may continuously learn the signals coming from an individual patient rather than using a statistical average taken from many patients. A custom reference signal may significantly improve minute changes in the physiological signals for an individual patient. In some implementations, the physiological parameters may be processed as a function of time that includes the shape of the curve changes, including hidden harmonics, changes in higher order derivatives, etc.

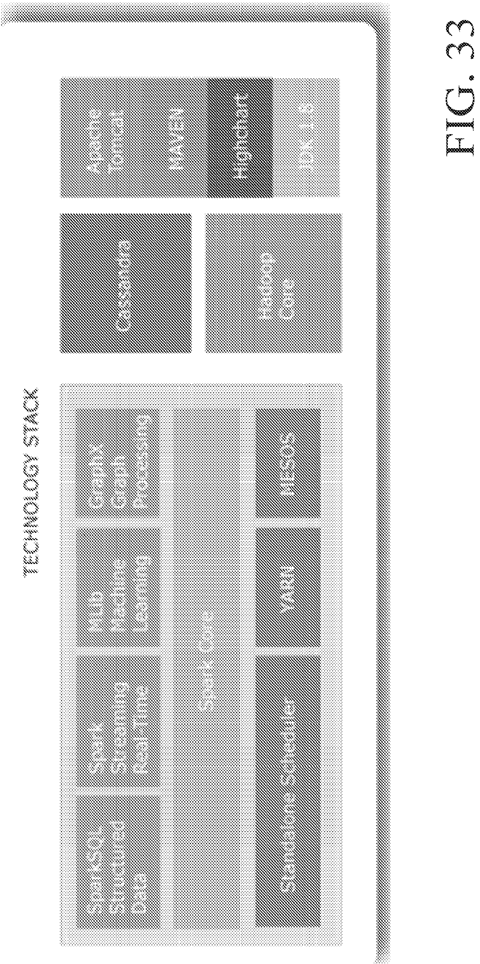

**FIG. 33** shows one implementation of various components of a data processing module. The core engine for one implementation of the data processing module may include one or more of the following parameters: fast processing, support for sophisticated analytics, real time stream processing, integration with both NoSQL and RDBMS, and integration with Hadoop.

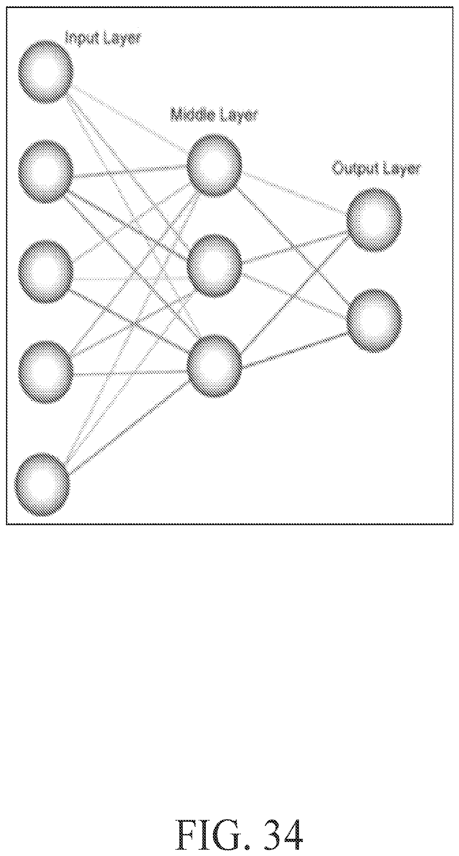

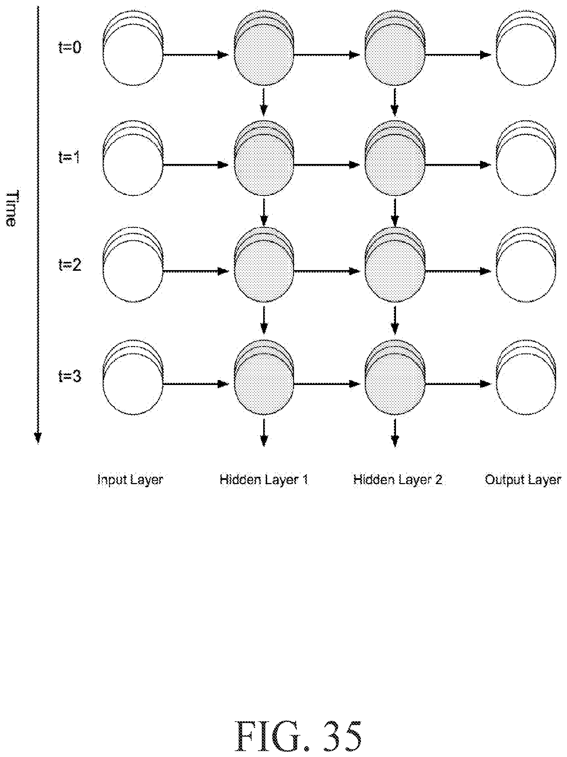

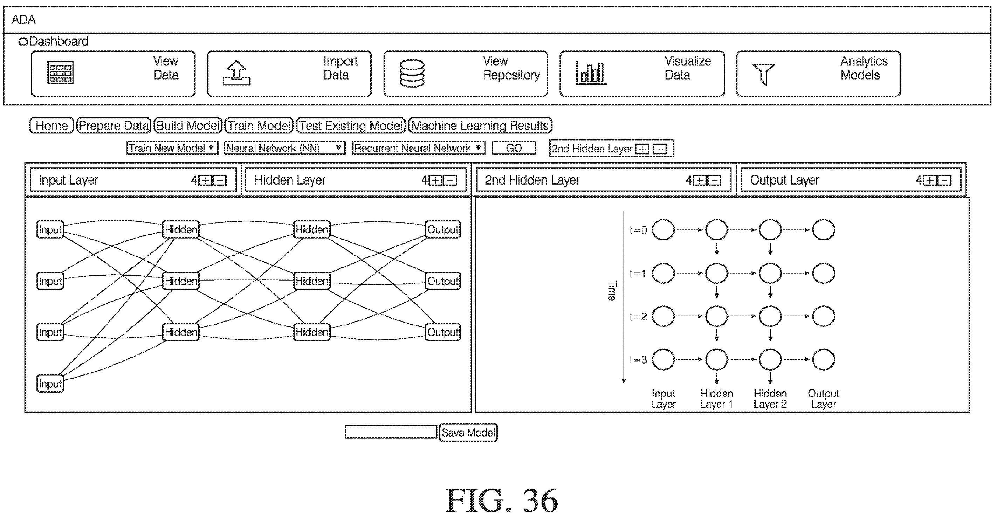

The data processing module may employ various machine learning methods to identify patterns, extract patterns, identify parameters indicative of stroke onset, etc. Machine learning can be broadly defined as the application of any computer-enabled algorithm that can be applied against a data set to find a pattern in the data. A machine-learning algorithm is used to determine the relationship between a system's inputs and outputs using a learning data set that is representative of all the behavior found in the system. This learning can be supervised or unsupervised. For example, a simple neural network called a Multilayer Perceptron (MLP), as shown in **FIG. 34****,** may be used to model various parameters or patterns of an individual, for example while sleeping. Each node is a neuron that uses a nonlinear activation function. Such a simple neural network may be used to distinguish data that are not linearly separable. In some implementations, as shown in **FIG. 35****,** a deep learning network may be used. A deep learning network may comprise a Leverage Recurrent Neural Networks (RNN) implementation, as shown in **FIG. 36****.** The system creates layers of interconnected networks, where each layer corresponds to a time slice. RNN are proven highly effective in handling time series data, assumes training inputs are time dependent, capable of accurately modeling / predicting changes through time, capable of generating an actual output value for a data point versus giving just a range, and each time slice is its own feed forward network - specified by a user.

In some implementations, a system for providing comprehensive stroke care comprises one or more of: educational resources tailored to the patient based on demographics, type of stroke, co-morbidities, medications, etc.; management tools to assist with the dramatic changes in lifestyle, such as reminders (e.g., medications, rehabilitation appointments, etc.), collaborative care resources (e.g., for spouse, doctor, physical therapist, caretaker, etc.), activity tracking with continuous data collection via a wearable, fitness tracking and guided meditation, stroke risk level assessment, etc.; community with others as part of the first national stroke survivor network where stroke survivors can give and receive support and encouragement connecting both patients and caregivers, "check in" with others in your group to make sure they are making progress towards their goals and are doing well mentally, share stories and relate to others, receive telemedicine/rehab resources with a speech therapist or mental health counselor; patient rehab and monitoring, or other enabling technologies; set recovery goals and track progress, cognitive evaluation tools, etc.; stroke Detection to alert caretakers via call/message, communication tools for patients with aphasia, etc.

### EXAMPLE 1

Various functional symptoms, quantitative markers, and blood/fluid products were scored for their ability to detect stroke. The scoring criteria were the following: should be grounded in scientific rationale, should be highly sensitive (>90%), should only have very few false positives (<10%), and stroke detection should be passive (automatic). Each of these parameters were scored from 0 to 5, except for passive detection which was scored on a scale of 0 (active detection) to 1 (passive detection). The score was then multiplied by a weight factor, shown in Table 1 below, and all the weighted factors summed to yield a total score.

As shown below in Tables 2 and 5, the functional symptoms with the highest total score were facial muscle weakness, unilateral weakness, limited visual field, gaze altered, and speech change. Of these functional symptoms, only facial muscle weakness, unilateral weakness, and speech change can be detected passively.

**Table 1. Analyzed Factors and Associated Weights**

| **Factor (score each on (0-5))** | **Weighting Factor** |
|---|---|
| Should be grounded in scientific rationale | 5 |
| Should be highly sensitive (>90%) | 5 |
| Should only have very few FPs (<10%) | 2 |
| Stroke detection should be passive (automatic) | 4 |

**Table 2. Analysis of Functional Symptoms of Stroke**

| **Functional Symptom** | **Time Period** | **Sample Implementation** | **Scientific rationale** | **Detection passive? (passive=1, active=0)** | **Sensitivity** | **Specificity** | **Total Score** |
|---|---|---|---|---|---|---|---|
| Speech Change | During, after | Amazon Alexa devices, Smart speakers, Smartwatch, external microphone coupled to 3^{rd} party device | 5 | 1 | 4 | 3 | 55 |
| Speech Comprehension | During, after | Amazon Alexa devices, Smart speakers, Smartwatch | 5 | 0 | 4 | 2 | 49 |
| Text Comprehension | During, after | Phone App, Tablet App | 3 | 0 | 4 | 2 | 39 |
| Consciousness | During | Camera, Wearable, Smartwatch | 4 | 1 | 3 | 2 | 43 |
| Coordination / Directions | During | Camera, Wearable, Smartwatch | 3 | 0 | 3 | 2 | 34 |
| Facial Muscle Weakness | During, after | Camera, Wearable | 5 | 1 | 5 | 4 | 62 |
| Arm Weakness | During | Camera, Wearable | 5 | 1 | 4 | 3 | 55 |
| Body Weakness - Grip | During | Camera, Wearable | 3 | 0 | 3 | 3 | 36 |
| Leg weakness | During | Camera, Wearable | 4 | 1 | 3 | 2 | 43 |
| Foot weakness | During | Camera, Wearable | 4 | 1 | 3 | 2 | 43 |
| Unilateral weakness | During | Camera, Wearable | 5 | 1 | 5 | 4 | 62 |
| Difficulty Walking | During, after | Camera, Wearable | 4 | 1 | 2 | 2 | 38 |
| Vertigo | During | Camera, Wearable | 4 | 0 | 4 | 2 | 44 |
| Sudden Vision Problems | During | Amazon Alexa devices, Phone app | 5 | 0 | 4 | 4 | 53 |
| Limited Visual Field | During | Amazon Alexa devices, Phone app | 5 | 0 | 5 | 3 | 56 |
| Gaze Altered | During | Camera, Phone app | 5 | 0 | 5 | 3 | 56 |
| Thunderclap Headache | Before, during, after | Amazon Alexa devices, Phone app | 5 | 0 | 4 | 3 | 51 |
| Nuchal rigidity (nape of neck) | | Amazon Alexa devices, Phone app | 5 | 0 | 3 | 3 | 46 |
| Respiration | Before, during | Wearable device, non-contact Doppler radar, Eulerian video processing techniques | 3 | 1 | 2 | 2 | 33 |
| Blood Pressure | Before, during | Wearable device (continuous use; periodic use) | 3 | 1 | 4 | 2 | 43 |

As shown in Tables 3 and 5, the quantitative markers with the highest total score were cerebral blood flow, EEG asymmetry, carotid artery stenosis, volumetric impedance spectroscopy, and limb asymmetry. Of these quantitative markers, all were considered to be detectable passively.

**Table 3. Analysis of Quantitative Symptoms of Stroke**

| **Marker** | **Time Period** | **Sample Implementation** | **Scientific rationale** | **Detection passive? (passive=1, active=0)** | **Sensitivity** | **Specificity** | **Total Score** |
|---|---|---|---|---|---|---|---|
| Volumetric impedance spectroscopy | During, after | Wearable, implant | 5 | 1 | 3 | 4 | 52 |
| EEG asymmetry | During, after | Wearable, implant | 5 | 1 | 4 | 4 | 57 |
| Brain perfusion | During, after | Wearable | 4 | 1 | 3 | 4 | 47 |
| Skin temperature | After | Wearable, IR imaging | 4 | 1 | 3 | 2 | 43 |
| Hyperhidrosis | After | Wearable | 3 | 1 | 4 | 2 | 43 |
| Limb asymmetry | During, after | Wearable, camera | 4 | 1 | 4 | 4 | 52 |
| Drift and pronation test | During, after | Camera | 4 | 0 | 4 | 4 | 48 |
| Cutaneous blood flow | After | Wearable, camera | 3 | 1 | 3 | 3 | 40 |
| Muscle tone | During, after | Wearable, camera | 3 | 1 | 4 | 3 | 45 |
| Heartrate variability | After | Wearable, implant, non-contact, Doppler radar | 3 | 1 | 3 | 3 | 40 |
| Facial surface EMG | During, after | Wearable, implant | 4 | 1 | 4 | 4 | 52 |
| Cerebral blood flow (CBF) | During, after | Wearable, implant | 5 | 1 | 5 | 5 | 64 |
| Carotid artery stenosis | During, after | Implant | 5 | 1 | 4 | 3 | 55 |
| Salivary cortisol | During, after | Wearable, implant | 3 | 1 | 2 | 2 | 33 |
| Neuron specific enolase (NSE) | During, after | Wearable, implant | 4 | 1 | 4 | 4 | 52 |
| Salivary NSE | During, after | Wearable, implant | 4 | 1 | 4 | 4 | 52 |

As shown in Tables 4 and 5, the products with the highest total score were Cornell University's products, SMARTChip, and ReST. Of these, none were considered to be passive detection.

**Table 4. Analysis of Products for Stroke Rapid Diagnosis**

| **Product** | **Time to Detect** | **Notes** | **Scientific rationale** | **Diagnosis passive?(passive=1, active=0)** | **Sensitivity** | **Specificity** | **Total Score** |
|---|---|---|---|---|---|---|---|
| CoaguCheck (Roche) | <1min | To shorten door-needle time; determine to start TPA faster | 2 | 0 | 2 | 2 | 24 |
| HemoChron (ITC) | ~few mins | To shorten door-needle time; determine to start TPA faster | 2 | 0 | 2 | 2 | 24 |
| iSTAT (Abbott) | <2mins | To shorten door-needle time; determine to start TPA faster | 2 | 0 | 2 | 2 | 24 |
| Cornell University | not known | Distinguish stroke from stroke mimics | 4 | 0 | 3 | 5 | 45 |
| ReST (Valtari Bio Inc) | <10mins | Initial stroke vs no stroke diagnosis | 3 | 0 | 3 | 3 | 36 |
| SMARTChip (Sarissa Biomedical) | ~few mins | Stroke vs no stroke using one drop of blood | 3 | 0 | 4 | 2 | 39 |

**Table 5. Results**

| **Functional** | **Total Score** | **Symptom** |
|---|---|---|
| #1 | 62 | Facial Muscle Weakness |
| #2 | 62 | Unilateral weakness |
| #3 | 56 | Limited Visual Field |
| #4 | 56 | Gaze Altered |
| #5 | 55 | Speech Change |
| | | |

| **Quantitative** | **Total Score** | **Symptom** |
|---|---|---|
| #1 | 64 | Cerebral blood flow (CBF) |
| #2 | 57 | EEG asymmetry |
| #3 | 55 | Carotid artery stenosis |
| #4 | 52 | Volumetric impedance spectroscopy |
| #5 | 52 | Limb asymmetry |
| | | |

| **Blood** | **Total Score** | **Organization** |
|---|---|---|
| #1 | 45 | Cornell University |
| #2 | 39 | SMARTChip (Sarissa Biomedical) |
| #3 | 36 | ReST (Valtari Bio Inc) |

Taken together, a multivariate system for stroke detection may include detecting one or more of: cerebral blood flow, EEG asymmetry, carotid artery stenosis, volumetric impedance spectroscopy, limb asymmetry, facial muscle weakness, unilateral weakness, and speech change. In some implementations, these various parameters may be measured at a variety of locations and/or times to determine stroke onset, occurrence, or after affects.

### EXAMPLE 2

Symmetrical and asymmetrical acceleration and distance were measured using an Apple^{®} Watch and displayed in a graphic representation (FIGS. 9-11, 27-32) in an application on a computing device. For this example, the implementation also measures the resolution of the Apple^{®} Watch accelerometer sensor and existing API capabilities.

For this example, the device was worn on a user's wrist. Any acceleration of the wrist was recoded and saved in the onboard database, including acceleration in x-, y- and z-axes. The computing device has a "sync" function that allows the data to be transferred to a computing device for analysis. Tables 6-8 show acceleration data, distance data, and calculated movement data (i.e., distance traveled), respectively, acquired using an Apple^{®} Watch worn on each wrist of a user. Data values were recorded at various time points, as shown in FIGS. 9-11, 27-32.

**Table 6. Acceleration (XYZ) of a Wrist**

| | **30** | **60** | **90** | **120** | **150** |
|---|---|---|---|---|---|
| **Acceleration X axis** | ~10 | ~0 | ~0 | ~15 | ~0 |
| **Acceleration Y axis** | ~10 | ~0 | ~0 | ~15 | ~0 |
| **Acceleration Z axis** | ~10 | ~0 | ~0 | ~15 | ~0 |

**Table 7. Distance Measurement of a Wrist**

| | **30** | **60** | **90** | **120** | **150** |
|---|---|---|---|---|---|
| **Acceleration X axis** | ~10 | ~0 | ~0 | ~15 | ~0 |
| **Acceleration Y axis** | ~10 | ~0 | ~0 | ~15 | ~0 |
| **Acceleration Z axis** | ~10 | ~0 | ~0 | ~15 | ~0 |

**Table 8. Movement Calculation of a Wrist**

| | **10** | **20** | **30** | **40** |
|---|---|---|---|---|
| **Description Label** | 143 | 51 | 247 | 207 |

Taken together, a system for stroke detection may include detecting one or more of: acceleration in x-, y- and/or z-axes; and /or distance in x-, y- and/or z-axes; and, in some implementations, calculating a distance traveled (i.e., movement) to determine asymmetrical limb movement, gait, etc. possibly indicative of a stroke event.

The systems and methods of the preferred implementation and variations thereof can be embodied and/or implemented at least in part as a machine configured to receive a computer-readable medium storing computer-readable instruction. The instructions are preferably executed by computer-executable components preferably integrated with the system and one or more portions of the hardware processor on the device for detecting stroke and/or computing device. The computer-readable medium can be stored on any suitable computer-readable media such as RAMs, ROMs, flash memory, EEPROMs, optical devices (e.g., CD or DVD), hard drives, floppy drives, or any suitable device. The computer-executable component is preferably a general or application-specific hardware processor, but any suitable dedicated hardware or hardware/firmware combination can alternatively or additionally execute the instructions.

As used in the description and claims, the singular form "a", "an" and "the" include both singular and plural references unless the context clearly dictates otherwise. For example, the term "signal" may include, and is contemplated to include, a plurality of signals. At times, the claims and disclosure may include terms such as "a plurality," "one or more," or "at least one;" however, the absence of such terms is not intended to mean, and should not be interpreted to mean, that a plurality is not conceived.

The term "about" or "approximately," when used before a numerical designation or range (e.g., to define a length or pressure), indicates approximations which may vary by ( + ) or ( - ) 5%, 1% or 0.1%. All numerical ranges provided herein are inclusive of the stated start and end numbers. The term "substantially" indicates mostly (i.e., greater than 50%) or essentially all of a device, substance, or composition.

The term "horizontal" as used herein is defined as a plane parallel to the conventional plane or surface of a heating element (e.g., heat source 410), regardless of its orientation. The term "vertical" refers to a direction perpendicular to the horizontal as just defined. Terms, such as "on", "above", "below", "bottom", "top", "side" (as in "sidewall"), "higher", "lower", "over", and "under", are defined with respect to the horizontal plane.

As used herein, the term "comprising" or "comprises" is intended to mean that the devices, systems, and methods include the recited elements, and may additionally include any other elements. "Consisting essentially of" shall mean that the devices, systems, and methods include the recited elements and exclude other elements of essential significance to the combination for the stated purpose. Thus, a system or method consisting essentially of the elements as defined herein would not exclude other materials, features, or steps that do not materially affect the basic and novel characteristic(s) of the claimed disclosure. "Consisting of" shall mean that the devices, systems, and methods include the recited elements and exclude anything more than a trivial or inconsequential element or step. Implementations defined by each of these transitional terms are within the scope of this disclosure.

The examples and illustrations included herein show, by way of illustration and not of limitation, specific implementations in which the subject matter may be practiced. Other implementations may be utilized and derived therefrom, such that structural and logical substitutions and changes may be made without departing from the scope of this disclosure. Such implementations of the inventive subject matter may be referred to herein individually or collectively by the term "invention" merely for convenience and without intending to voluntarily limit the scope of this application to any single invention or inventive concept, if more than one is in fact disclosed. Thus, although specific implementations have been illustrated and described herein, any arrangement calculated to achieve the same purpose may be substituted for the specific implementations shown. This disclosure is intended to cover any and all adaptations or variations of various implementations. Combinations of the above implementations, and other implementations not specifically described herein, will be apparent to those of skill in the art upon reviewing the above description.

## Claims

1. A system for detecting an anomalous biologic event in a person, the system comprising:
a wearable device (400) configured to monitor a plurality of skin surface sites of a person, the device comprising:
an electrode (902) configured to obtain bioelectrical data, the electrode being placed in contact with at least one site in the plurality of skin surface sites;
an electronic stimulus source (410) having a surface area to provide stimulus;
a sensor (412, 414) configured to sense physiological data, the sensor (412, 414) being placed adjacent to at least one of the plurality of skin surface sites; and
at least one processor communicatively coupled to the wearable device (400) and configured to:
cause the stimulus source (410) to generate a stimulus;
cause excitation of the electrode to trigger monitoring of respective contacted skin surface sites;
cause operation of the sensor (412, 414);
receive bioelectrical data from respective contacted skin surface sites;
receive physiological data from the sensor (412, 414);
continuously compute a difference in the received bioelectrical data over a predefined time period;
compute a difference in the received physiological data at intervals of the predefined time period; and
generate, based on the computation, an assessment including a likelihood of occurrence of the anomalous biologic event;
wherein the electrode (902) comprises a plurality of electrodes (902a, 902b, 902c); and **characterized in that**:
the wearable device (400) is configured to be worn on a wrist of the person;
the stimulus source is a heat stimulus source (410) configured to uniformly heat the surface area and emit heat toward a top region of the wrist; and
the at least one processor suppresses a portion of the plurality of electrodes (902a, 902b, 902c) from exciting until the stimulus source (410) ceases generating stimulus.

2. The system of claim 1, wherein the sensor (412, 414) is enclosed within the surface area of the stimulus source (410).

3. The system of any one of claims 1 to 2, wherein the at least one processor is further configured to:
detect a lack of stimulus from the stimulus source (410);
cause excitation of the electrode or a second electrode based on the detected lack of stimulus;
obtain additional bioelectrical data; and
responsive to determining that the additional bioelectrical data indicates an additional likelihood of the occurrence of the anomalous biologic event, updating the assessment.

4. The system of claim 1, wherein the plurality of electrodes (902a, 902b, 902c) includes:
a first electrode configured to contact a first skin surface site at a top region of the wrist and to obtain a skin hydration signal;
a second electrode configured to contact a second skin surface site at a bottom region of the wrist to obtain a first skin conductance signal; and
a third electrode configured to contact a third skin surface site at the bottom region of the wrist adjacent to the second electrode, the third electrode configured to obtain a second skin conductance signal.

5. The system of claim 1, wherein the plurality of electrodes (902a, 902b, 902c) includes:
a first electrode configured to contact a first skin surface site and to obtain an electrodermal activity signal;
a second electrode configured to contact a second skin surface site to obtain a first skin conductance signal; and
a third electrode configured to contact a third skin surface site adjacent to the second electrode, the third electrode configured to obtain a second skin conductance signal.

6. The system of claim 1, wherein the plurality of electrodes (902a, 902b, 902c) includes:
a first electrode configured to contact a first skin surface site at a top region of the wrist to obtain a reference electromyography (EMG) signal;
a second electrode configured to contact a second skin surface site at a bottom region of the wrist to obtain a first EMG signal from a first muscle; and
a third electrode configured to contact a third skin surface site at the bottom region of the wrist adjacent to the second electrode, the third electrode configured to obtain a second EMG signal from a second muscle.

7. The system of claim 1, wherein the wearable device (400) comprises a main portion, a first band portion, and a second band portion, and the plurality of electrodes (902a, 902b, 902c) comprise:
a first electrode arranged on a first side of the main portion;
a second electrode arranged on a first side of the first band portion; and
a third electrode arranged on the first side of the first band portion and parallel to the second electrode.

8. The system of any one of claims 1 to 7, wherein:
the plurality of electrodes (902a, 902b, 902c) is associated with an electrodermal sensor configured for the excitation and collection of the bioelectrical data from at least one of the plurality of skin surface sites, and
the plurality of sensors comprises at least a blood volume sensor (412) and an infrared temperature sensor.

9. The system of any one of claims 1 to 8, wherein the bioelectrical data is:
obtained simultaneously from the plurality of electrodes (902a, 902b, 902c); and
analyzed in combination to generate the assessment.

10. The system of any one of claims 1 to 9, further comprising an ambient temperature sensor (6750) placed on a top surface opposite the side of the electronic stimulus source (410), wherein the ambient temperature sensor (6750) is located at an edge or a corner of the top surface.

11. The system of any one of claims 1 to 10, wherein the electronic stimulus source (410) comprises a plurality of thin film layers (8204, 8208, 8214), and wherein at least one layer of the plurality of layers (8204, 8208, 8214) is a heater trace layer (8206) having a serpentine-shaped trace extending within a plane of the at least one layer to substantially cover a surface of the at least one layer.

## Patentansprüche

1. Ein System zum Erkennen eines anomalen biologischen Ereignisses bei einer Person, wobei das System umfasst:
eine tragbare Vorrichtung (400), die dazu konfiguriert ist, eine Mehrzahl von Hautoberflächenstellen einer Person zu überwachen, wobei die Vorrichtung umfasst:
eine Elektrode (902), die dazu konfiguriert ist, bioelektrische Daten zu erhalten, wobei die Elektrode in Kontakt mit mindestens einer Stelle der Mehrzahl von Hautoberflächenstellen angeordnet ist;
eine elektronische Stimulationsquelle (410) mit einer Oberfläche zum Bereitstellen eines Stimulus;
einen Sensor (412, 414), der dazu konfiguriert ist, physiologische Daten zu erfassen, wobei der Sensor (412, 414) angrenzend an mindestens eine der Mehrzahl von Hautoberflächenstellen angeordnet ist; und
mindestens einen Prozessor, der kommunikativ mit der tragbaren Vorrichtung (400) gekoppelt und dazu konfiguriert ist:
zu bewirken, dass die Stimulationsquelle (410) einen Stimulus erzeugt;
eine Anregung der Elektrode zu bewirken, um eine Überwachung jeweiliger kontaktierter Hautoberflächenstellen auszulösen;
einen Betrieb des Sensors (412, 414) zu bewirken;
bioelektrische Daten von jeweiligen kontaktierten Hautoberflächenstellen zu empfangen;
physiologische Daten von dem Sensor (412, 414) zu empfangen;
kontinuierlich eine Differenz in den empfangenen bioelektrischen Daten über einen vorgegebenen Zeitraum zu berechnen;
eine Differenz in den empfangenen physiologischen Daten in Intervallen des vorgegebenen Zeitraums zu berechnen; und
basierend auf der Berechnung eine Beurteilung zu erzeugen, die eine Wahrscheinlichkeit des Auftretens des anomalen biologischen Ereignisses aufweist;
wobei die Elektrode (902) eine Mehrzahl von Elektroden (902a, 902b, 902c) umfasst; und **dadurch gekennzeichnet, dass**:
die tragbare Vorrichtung (400) dazu konfiguriert ist, am Handgelenk der Person getragen zu werden;
die Stimulationsquelle eine Wärmestimulationsquelle (410) ist, die dazu konfiguriert ist, die Oberfläche gleichmäßig zu erwärmen und Wärme in Richtung eines oberen Bereichs des Handgelenks abzugeben; und
der mindestens eine Prozessor einen Teil der Mehrzahl von Elektroden (902a, 902b, 902c) daran hindert, angeregt zu werden, bis die Stimulationsquelle (410) die Erzeugung des Stimulus beendet.

2. Das System nach Anspruch 1, wobei der Sensor (412, 414) innerhalb der Oberfläche der Stimulationsquelle (410) eingeschlossen ist.

3. Das System nach einem der Ansprüche 1 bis 2, wobei der mindestens eine Prozessor ferner dazu konfiguriert ist:
ein Ausbleiben eines Stimulus von der Stimulationsquelle (410) zu erkennen;
basierend auf dem erkannten Ausbleiben eines Stimulus eine Anregung der Elektrode oder einer zweiten Elektrode zu bewirken;
zusätzliche bioelektrische Daten zu erhalten; und
als Reaktion auf die Feststellung, dass die zusätzlichen bioelektrischen Daten eine zusätzliche Wahrscheinlichkeit des Auftretens des anomalen biologischen Ereignisses anzeigen, die Beurteilung zu aktualisieren.

4. Das System nach Anspruch 1, wobei die Mehrzahl von Elektroden (902a, 902b, 902c) aufweist:
eine erste Elektrode, die dazu konfiguriert ist, eine erste Hautoberflächenstelle an einem oberen Bereich des Handgelenks zu kontaktieren und ein Hauthydratationssignal zu erhalten;
eine zweite Elektrode, die dazu konfiguriert ist, eine zweite Hautoberflächenstelle an einem unteren Bereich des Handgelenks zu kontaktieren, um ein erstes Hautleitfähigkeitssignal zu erhalten; und
eine dritte Elektrode, die dazu konfiguriert ist, eine dritte Hautoberflächenstelle an dem unteren Bereich des Handgelenks angrenzend an die zweite Elektrode zu kontaktieren, wobei die dritte Elektrode dazu konfiguriert ist, ein zweites Hautleitfähigkeitssignal zu erhalten.

5. Das System nach Anspruch 1, wobei die Mehrzahl von Elektroden (902a, 902b, 902c) aufweist:
eine erste Elektrode, die dazu konfiguriert ist, eine erste Hautoberflächenstelle zu kontaktieren und ein elektrodermales Aktivitätssignal zu erhalten;
eine zweite Elektrode, die dazu konfiguriert ist, eine zweite Hautoberflächenstelle zu kontaktieren, um ein erstes Hautleitfähigkeitssignal zu erhalten; und
eine dritte Elektrode, die dazu konfiguriert ist, eine dritte Hautoberflächenstelle angrenzend an die zweite Elektrode zu kontaktieren, wobei die dritte Elektrode dazu konfiguriert ist, ein zweites Hautleitfähigkeitssignal zu erhalten.

6. Das System nach Anspruch 1, wobei die Mehrzahl von Elektroden (902a, 902b, 902c) aufweist:
eine erste Elektrode, die dazu konfiguriert ist, eine erste Hautoberflächenstelle an einem oberen Bereich des Handgelenks zu kontaktieren, um ein Referenz-Elektromyographiesignal (EMG-Signal) zu erhalten;
eine zweite Elektrode, die dazu konfiguriert ist, eine zweite Hautoberflächenstelle an einem unteren Bereich des Handgelenks zu kontaktieren, um ein erstes EMG-Signal von einem ersten Muskel zu erhalten; und
eine dritte Elektrode, die dazu konfiguriert ist, eine dritte Hautoberflächenstelle an dem unteren Bereich des Handgelenks angrenzend an die zweite Elektrode zu kontaktieren, wobei die dritte Elektrode dazu konfiguriert ist, ein zweites EMG-Signal von einem zweiten Muskel zu erhalten.

7. Das System nach Anspruch 1, wobei die tragbare Vorrichtung (400) einen Hauptabschnitt, einen ersten Bandabschnitt, und einen zweiten Bandabschnitt umfasst, und die Mehrzahl von Elektroden (902a, 902b, 902c) umfassen:
eine erste Elektrode, die auf einer ersten Seite des Hauptabschnitts angeordnet ist;
eine zweite Elektrode, die auf einer ersten Seite des ersten Bandabschnitts angeordnet ist; und
eine dritte Elektrode, die auf der ersten Seite des ersten Bandabschnitts und parallel zu der zweiten Elektrode angeordnet ist.

8. Das System nach einem der Ansprüche 1 bis 7, wobei:
die Mehrzahl von Elektroden (902a, 902b, 902c) einem elektrodermalen Sensor zugeordnet ist, der zur Anregung und Erfassung der bioelektrischen Daten von mindestens einer der Mehrzahl von Hautoberflächenstellen konfiguriert ist, und
die Mehrzahl von Sensoren mindestens einen Blutvolumensensor (412) und einen Infrarot-Temperatursensor umfasst.

9. Das System nach einem der Ansprüche 1 bis 8, wobei die bioelektrischen Daten:
gleichzeitig von der Mehrzahl von Elektroden (902a, 902b, 902c) erhalten werden; und
in Kombination analysiert werden, um die Beurteilung zu erzeugen.

10. Das System nach einem der Ansprüche 1 bis 9, ferner umfassend einen Umgebungstemperatursensor (6750), der auf einer oberen Oberfläche gegenüber der Seite der elektronischen Stimulationsquelle (410) angeordnet ist, wobei der Umgebungstemperatursensor (6750) an einer Kante oder einer Ecke der oberen Oberfläche angeordnet ist.

11. Das System nach einem der Ansprüche 1 bis 10, wobei die elektronische Stimulationsquelle (410) eine Mehrzahl von Dünnschichtlagen (8204, 8208, 8214) umfasst und wobei mindestens eine Lage der Mehrzahl von Lagen (8204, 8208, 8214) eine Heizleiterbahnlage (8206) mit einer mäanderförmigen Leiterbahn ist, die sich innerhalb einer Ebene der mindestens einen Lage erstreckt, um eine Oberfläche der mindestens einen Lage im Wesentlichen zu bedecken.

## Revendications

1. Un système de détection d'un événement biologique anormal chez une personne, le système comprenant :
un dispositif portable (400) configuré pour surveiller une pluralité de sites de surface cutanée d'une personne, le dispositif comprenant :
une électrode (902) configurée pour obtenir des données bioélectriques, l'électrode étant placée en contact avec au moins un site dans la pluralité de sites de surface cutanée ;
une source électronique de stimulation (410) ayant une surface destinée à fournir une stimulation ;
un capteur (412, 414) configuré pour détecter des données physiologiques, le capteur (412, 414) étant placé adjacent à au moins un parmi la pluralité de sites de surface cutanée ; et
au moins un processeur couplé communicativement au dispositif portable (400) et configuré pour :
amener la source de stimulation (410) à générer une stimulation ;
provoquer l'excitation de l'électrode afin de déclencher la surveillance des sites de surface cutanée respectifs en contact avec celle-ci ;
provoquer le fonctionnement du capteur (412, 414) ;
recevoir des données bioélectriques provenant des sites de surface cutanée respectifs en contact avec celle-ci ;
recevoir des données physiologiques provenant du capteur (412, 414) ;
calculer en continu une différence dans les données bioélectriques reçues sur une période de temps prédéfinie ;
calculer une différence dans les données physiologiques reçues à des intervalles de la période de temps prédéfinie ; et
générer, sur la base des calculs, une évaluation incluant une probabilité de survenue de l'événement biologique anormal ;
dans lequel l'électrode (902) comprend une pluralité d'électrodes (902a, 902b, 902c) ; et **caractérisé en ce que** :
le dispositif portable (400) est configuré pour être porté au poignet de la personne ;
la source de stimulation est une source de stimulation thermique (410) configurée pour chauffer uniformément la surface et émettre de la chaleur vers une région supérieure du poignet ; et
ledit au moins un processeur empêche une partie de la pluralité d'électrodes (902a, 902b, 902c) de l'excitation jusqu'à ce que la source de stimulation (410) cesse de générer la stimulation.

2. Le système selon la revendication 1, dans lequel le capteur (412, 414) est enfermé à l'intérieur de la surface de la source de stimulation (410).

3. Le système selon l'une quelconque des revendications 1 à 2, dans lequel ledit au moins un processeur est en outre configuré pour :
détecter une absence de stimulation provenant de la source de stimulation (410) ;
provoquer l'excitation de l'électrode ou d'une seconde électrode sur la base de l'absence de stimulation détectée ;
obtenir des données bioélectriques supplémentaires ; et
en réponse à la détermination que les données bioélectriques supplémentaires indiquent une probabilité supplémentaire de survenue de l'événement biologique anormal, mettre à jour l'évaluation.

4. Le système selon la revendication 1, dans lequel la pluralité d'électrodes (902a, 902b, 902c) inclut :
une première électrode configurée pour entrer en contact avec un premier site de surface cutanée au niveau d'une région supérieure du poignet et pour obtenir un signal d'hydratation cutanée ;
une seconde électrode configurée pour entrer en contact avec un second site de surface cutanée au niveau d'une région inférieure du poignet afin d'obtenir un premier signal de conductance cutanée ; et
une troisième électrode configurée pour entrer en contact avec un troisième site de surface cutanée au niveau de la région inférieure du poignet adjacent à la seconde électrode, la troisième électrode étant configurée pour obtenir un second signal de conductance cutanée.

5. Le système selon la revendication 1, dans lequel la pluralité d'électrodes (902a, 902b, 902c) inclut :
une première électrode configurée pour entrer en contact avec un premier site de surface cutanée et pour obtenir un signal d'activité électrodermale ;
une seconde électrode configurée pour entrer en contact avec un second site de surface cutanée afin d'obtenir un premier signal de conductance cutanée ; et
une troisième électrode configurée pour entrer en contact avec un troisième site de surface cutanée adjacent à la seconde électrode, la troisième électrode étant configurée pour obtenir un second signal de conductance cutanée.

6. Le système selon la revendication 1, dans lequel la pluralité d'électrodes (902a, 902b, 902c) inclut :
une première électrode configurée pour entrer en contact avec un premier site de surface cutanée au niveau d'une région supérieure du poignet afin d'obtenir un signal d'électromyographie (EMG) de référence ;
une seconde électrode configurée pour entrer en contact avec un second site de surface cutanée au niveau d'une région inférieure du poignet afin d'obtenir un premier signal EMG provenant d'un premier muscle ; et
une troisième électrode configurée pour entrer en contact avec un troisième site de surface cutanée au niveau de la région inférieure du poignet adjacent à la seconde électrode, la troisième électrode étant configurée pour obtenir un second signal EMG provenant d'un second muscle.

7. Le système selon la revendication 1, dans lequel le dispositif portable (400) comprend une partie principale, une première partie de bracelet et une seconde partie de bracelet, et la pluralité d'électrodes (902a, 902b, 902c) comprennent :
une première électrode disposée sur un premier côté de la partie principale ;
une seconde électrode disposée sur un premier côté de la première partie de bracelet ; et
une troisième électrode disposée sur le premier côté de la première partie de bracelet et parallèle à la seconde électrode.

8. Le système selon l'une quelconque des revendications 1 à 7, dans lequel :
la pluralité d'électrodes (902a, 902b, 902c) est associée à un capteur électrodermique configuré pour l'excitation et la collecte des données bioélectriques provenant d'au moins un site parmi la pluralité de sites de surface cutanée ; et
la pluralité de capteurs comprend au moins un capteur de volume sanguin (412) et un capteur de température infrarouge.

9. Le système selon l'une quelconque des revendications 1 à 8, dans lequel les données bioélectriques sont :
obtenues simultanément à partir de la pluralité d'électrodes (902a, 902b, 902c) ; et
analysées en combinaison pour générer l'évaluation.

10. Le système selon l'une quelconque des revendications 1 à 9, comprenant en outre un capteur de température ambiante (6750) placé sur une surface supérieure opposée au côté de la source électronique de stimulation (410), dans lequel le capteur de température ambiante (6750) est situé à un bord ou à un coin de la surface supérieure.

11. Le système selon l'une quelconque des revendications 1 à 10, dans lequel la source électronique de stimulation (410) comprend une pluralité de couches de films minces (8204, 8208, 8214), et dans lequel au moins une couche parmi la pluralité de couches (8204, 8208, 8214) est une couche de piste chauffante (8206) ayant une piste en forme de serpentin s'étendant dans un plan de ladite au moins une couche de manière à recouvrir sensiblement une surface de ladite au moins une couche.
